# EUROPEAN PATENT APPLICATION

(11) **EP 1 715 053 A1**
(43) Date of publication of application: **25.10.2006**
(21) Application number: 05103092.2
(22) Date of filing: 18.04.2005
(51) Int. Cl.: C12N 15/90, A01K 67/027, C12N 5/10

(54) **Gene trap cassettes for random and targeted conditional gene inactivation**

(71) Applicant: FrankGen Biotechnologie AG, 61476 Kronberg/Taunus (DE); GSF Forschungszentrum für Umwelt und Gesundheit GMBH, 85764 Neuherberg (DE); Max-Planck-Gesellschaft zur Förderung der Wissenschaften e.V., 80539 München (DE)
(72) Inventor: Schnütgen, Frank, 63755, Alzenau (DE); Melchner, Harald, von, 61476, Kronberg/Taunus (DE); Wurst, Wolfgang, 80937, München (DE); Ruiz, Patricia, 10437, Berlin (DE); De-Zolt, Silke, 63517, Rodenbach (DE); Floss, Thomas, 85406, Oberappersdorf (DE); Hansen, Jens, 85551 Kirchheim (DE)
(74) Representative: Helbing, Jörg

(57) **Abstract**

The present invention provides for a new type of gene trap cassettes, which can induce conditional mutations. The cassettes rely on directional site-specific recombination systems, which can repair and re-induce gene trap mutations when activated in succession. After the gene trap cassettes are inserted into the genome of the target organism, mutations can be activated at a particular time and place in somatic cells. In addition to their conditional features, the gene trap cassettes create multipurpose alleles amenable to a wide range of post-insertional modifications. Such gene trap cassettes can be used to mutationally inactivate all cellular genes. In addition the invention relates to a cell, preferably a mammalian cell which contains the above mentioned gene trap cassette. Moreover, the invention relates to the use of said cell for identification and/or isolation of genes and for the creation of transgenic organisms to study gene function at various developmental stages, including the adult, as well as for the creation of animal models of human disease useful for *in vivo* drug target validation. In conclusion, the present invention provides a process which enables a temporally and/or spatially restricted inactivation of all genes that constitute a living organism.

## Description

The present invention provides for a new type of gene trap cassettes, which can induce conditional mutations. The cassettes rely on directional site-specific recombination systems, which can repair and re-induce gene trap mutations when activated in succession. After the gene trap cassettes are inserted into the genome of the target organism, mutations can be activated at a particular time and place in somatic cells. In addition to their conditional features, the gene trap cassettes create multipurpose alleles amenable to a wide range of postinsertional modifications. Such gene trap cassettes can be used to mutationally inactivate all cellular genes. In addition, the invention relates to a cell, preferably a mammalian cell, which contains the above mentioned gene trap cassette. Moreover, the invention relates to the use of said cell for identification and/or isolation of genes and for the creation of transgenic organisms to study gene function at various developmental stages, including the adult, as well as for the creation of animal models of human disease useful for *in vivo* drug target validation. In conclusion, the present invention provides a process which enables a temporally and/or spatially restricted inactivation of all genes that constitute a living organism.

### Background of the Invention

With the complete sequencing of the human and mouse genomes, attention has shifted towards comprehensive functional annotation of mammalian genes (Austin, C.P. et al., Nat. Genet. 36, 921-4 (2004); Auwerx, J. et al., Nat. Genet. 36, 925-7 (2004)). Among the various approaches for addressing gene function, the most relevant for extrapolation to human genetic disease is mutagenesis in the mouse. Although several model organisms have been used in a variety of mutagenesis approaches, the mouse offers particular advantages because its genome structure and organization is closely related to the human genome. Most importantly, mouse embryonic stem (ES) cells, which grow indefinitely in tissue culture, allow the generation of mice with defined mutations in single genes for functional analysis and studies of human disease.

Several mutagenesis strategies have been deployed in mice, ranging from random chemical (ENU) mutagenesis coupled with phenotype driven screens (Cox., R.D. and Brown, S.D., Curr. Opin. Genet. Dev. 13, 278-83 (2003); Brown, S.D. and Balling, R. Curr. Opin. Genet. Dev. 11, 268-73 (2001)) to sequence-based approaches using ES cell technology, such as gene trapping and gene targeting (Floss, T. and Wurst, W., Methods Mol. Biol. 185, 347-79 (2002); Mansouri, A., Methods Mol. Biol. 175, 397-413 (2001)).

Gene trapping is a high-throughput approach that is used to introduce insertional mutations across the mouse genome. It is performed with gene trap vectors whose principal element is a gene trap cassette consisting of a promoterless reporter gene and/or selectable marker gene flanked by an upstream 3' splice site (splice acceptor; SA) and a downstream transcriptional termination sequence (polyadenylation sequence; polyA). When inserted into an intron of an expressed gene, the gene trap cassette is transcribed from the endogenous promoter in the form of a fusion transcript in which the exon(s) upstream of the insertion site is (are) spliced in frame to the reporter/selectable marker gene. Since transcription is terminated prematurely at the inserted polyadenylation site, the processed fusion transcript encodes a truncated and non-functional version of the cellular protein and the reporter/selectable marker (Stanford, W.L. et al., Nat. Rev. Genet. 2, 756-68 (2001)). Thus, gene traps simultaneously inactivate and report the expression of the trapped gene at the insertion site, and provide a DNA tag (gene trap sequence tag, GTST) for the rapid identification of the disrupted gene. As gene trap vectors insert randomly across the genome, a large number of mutations can be generated in ES cells within a limited number of experiments. Gene trap approaches have been used successfully in the past by both academic and private organizations to create libraries of ES cell lines harboring mutations in single genes (Wiles, M.V. et al., Nat. Genet. 24, 13-4 (2000); Hansen, J. et al., Proc. Natl. Acad. Sci. USA 100, 9918-22 (2003); Stryke, D. et al., Nucleic Acids Res. 31, 278-81 (2003); Zambrowicz, B. P. et al., Proc. Natl. Acad. Sci. USA 100, 14109-14 (2003)). Collectively, the existing resources cover about 66% of all protein coding genes within the mouse genome (Skarnes, W.C. et al., Nat. Genet. 36, 543-4 (2004)). However, the gene trap vectors which have been used to generate the currently available resources induce only null mutations and mouse mutants generated from these libraries can report only the earliest and non-redundant developmental function of the trapped gene. Therefore, for most of the mutant strains the significance of the trapped gene for human disease remains uncertain, as most human disorders result from late onset gene dysfunction. In addition, between 20% - 30% of the genes targeted in ES cells are required for development and cause embryonic lethal phenotypes when transferred to the germline, which precludes their functional analysis in the adult (Hansen, J. et al., Proc. Natl. Acad. Sci. USA 100, 9918-22 (2003); Mitchell, K.J. et al., Nat. Genet. 28, 241-9 (2001)).

To circumvent the limitations posed by germline mutations, conditional gene targeting strategies use site-specific recombination to spatially and temporally restrict the mutation to somatic cells (von Melchner, H. and Stewart, A.F., Handbook of Stem Cells, ed. Lanza, R., Vol. 1, pp 609-622 (2004)). The creation of conditional mouse mutants requires the generation of two mouse strains, i.e. the recombinase recognition strain and the recombinase expressing strain. The recombinase recognition strain is generated by homologous recombination in ES cells whereby the targeted exon(s) is (are) flanked by two recombinase recognition sequences (hereinafter "RRSs"), e.g. loxP or frt. Since the RRSs reside in introns they do not interfere with gene expression. The recombinase expressing strain contains a recombinase transgene (e.g. Cre, Flp) whose expression is either restricted to certain cells and tissues or is inducible by external agents. Crossing of the recombinase recognition strain with the recombinase expressing strain deletes the RRS-flanked exons from the doubly transgenic offspring in a prespecified temporally and/or spatially restricted manner. Thus, the method allows the temporal analysis of gene function in particular cells and tissues of otherwise widely expressed genes. Moreover, it enables the analysis of gene function in the adult organism by circumventing embryonic lethality, which is frequently the consequence of gene inactivation. For pharmaceutical research, aiming to validate the utility of genes and their products as targets for drug development, inducible mutations provide an excellent genetic tool. However, targeted mutagenesis in ES cells requires a detailed knowledge of gene structure and organization in order to physically isolate a gene in a targeting vector. Although the completed sequencing of the mouse genome greatly assists targeted mutagenesis, the generation of mutant mouse strains by this procedure is still time consumming labor intensive, expensive and relatively inefficient as it can handle only one target at a time.

To address this problem a conditional gene trapping strategy as described in WO 99/50426 has been developed. It utilizes a gene trap cassette capable of producing mutations that can be switched on and off in a spatio-temporal restricted manner. These gene trap cassettes comprise suitably arranged frt or Ioxp recombinase recognition sites, which - when exposed to Flp or Cre, respectively - lead to removal or inversion of the gene trap cassette and thereby to induction or repair of the mutation. However, recombination reactions mediated by conventional site specific recombinases, such as FLPe and Cre are normally reversible (described in the above documents) between identical recombinase recognition sites (e.g., two loxP or two frt sites). In a conditional gene trap setting, where the recombinase effects a "flipping" of the gene trap cassette as described in the above documents, this would mean that equal amounts of sense and antisense products would be generated, so that one half of the targeted alleles carrying the gene trap would be inactivated whereas the other half would still have a functional configuration. Therefore, it is important to shift the equilibrium of the recombinase reaction towards the gene trap inversion that causes gene inactivation.

The shifting of the equilibrium of the recombinase reaction was achieved with a gene trap system comprising two specific recombinase recognition sites capable of unidirectional inversion if exposed to the corresponding recombinase. Suitable recombination systems are for example the Cre/loxP recombination system with mutant loxP recognition sites (e.g., single mutant recognition sites lox66 and lox71; Albert et al., Plant J., 7, 649 - 659 (1995)), which - if subjected to Cre recombination - generates a double mutant- and a wildtype-loxP site, each on one side of the inverted DNA. Since the latter combination of loxp sites is less efficiently recognised by the Cre-recombinase, the inversion is predominantly unidirectional.

An only predominantly unidirectional inversion, however, has the disadvantage that a significant number of non-inverted cassettes will also be present. While this is acceptable for cultured cells, it cannot be tolerated in the living animal.

WO 02/088353 and Schnutgen, F. et al., Nat. Biotechnol. 21, 562-5 (2003) disclose a new strategy for directional site specific recombination termed "flip-excision" ("FIEx"). Two sets of incompatible site-specific recombination recognition sites are flanking the DNA fragment to be inverted, in the same order but in reverse orientation.

However, there is still a need for a site specific recombination strategy that is truly directional to enable successive gene trap cassette inversions to first repair and then re-induce a gene trap mutation.

### Summary of the Invention

It was found that the FlEx strategy, if applied on a gene trap cassette of the present invention, allows for true unidirectional inversions. In particular, the gene trap cassettes employ two directional site-specific recombination systems, which, when activated in succession, invert the gene trap cassette from its mutagenic orientation on the sense, coding strand to a non-mutagenic orientation on the anti-sense, non-coding strand and back to a mutagenic orientation on the sense, coding strand. These cassettes rely on directional site-specific recombination systems, and can induce conditional mutations in most genes expressed in mouse embryonic stem (ES) cells. They were used to assemble the largest library of ES cell lines with conditional mutations in single genes yet assembled, presently totaling 1,000 unique genes. Moreover, it could be shown that mutations induced by these vectors in ES cells can be both repaired and re-induced by site-specific recombination.

The present invention thus provides:
(1) a gene trap cassette capable of causing conditional mutations in genes, which comprises a functional DNA segment (FS) inserted in a mutagenic or non-mutagenic manner, in sense or antisense direction relative to the gene to be trapped, said FS being flanked by the recombinase recognition sequences (RRSs) of at least two independent directional site-specific recombination systems, wherein each system
   (i) comprises two pairs of heterotypic RRSs, said RRSs being oriented in opposite orientation and the RRSs of the two pairs being lined up in opposite order on both sides of the FS, and
   (ii) is capable of inverting FS by means of a recombinase mediated flip-excision mechanism;
(2) a preferred embodiment of the gene trap cassette defined in (1) above, which comprises two functional DNA segments,
   (a) a first DNA segment (disruption segment) having a FS being oriented in antisense orientation relative to the transcriptional orientation of the gene to be trapped and being flanked by the RRSs of said at least two independent directional site-specific recombination systems, and
   (b) a second segment (selection segment) being positioned in sense direction relative to the transcriptional orientation of the gene to be trapped and being flanked by two RRSs of a third site specific recombinase in the same orientation;
(3) a cell, a culture of cells or tissue, or a transgenic organism comprising the gene trap cassette as defined in (1) or (2) above;
(4) a process for preparing the cell, the culture of cells or tissue, or the transgenic organism of (3) above, which comprises introducing a gene trap cassette as defined in (1) or (2) above into a suitable cell;
(5) a process for the generation of conditional mutations in one or more genes of an organism comprising
   (i) introduction of a gene trap cassette as defined in (1) or (2) above into a suitable cell,
   (ii) selection of cells in which the construct is incorporated in a gene,
   (iii) identification and/or isolation of the gene in which the construct is incorporated,
   (iv) inversion or deletion of the selection segment in or from the trapped gene,
   (v) induction of a mutation in the trapped gene by inversion of the functional DNA segment;
(6) a transgenic mammal, preferably a transgenic non-human mammal obtainable by the method of (5) above;
(7) the use of the cell, the culture of cells or tissue, or the transgenic organism of (3) above for the identification and/or isolation of genes; and
(8) the use of the transgenic organism of (3) above or the transgenic mammal of (6) above
   (i) to study gene function at various developmental stages;
   (ii) as an animal model of human disease; or
   (iii) as an in vivo drug target validation model in drug development.

### Description of Figures

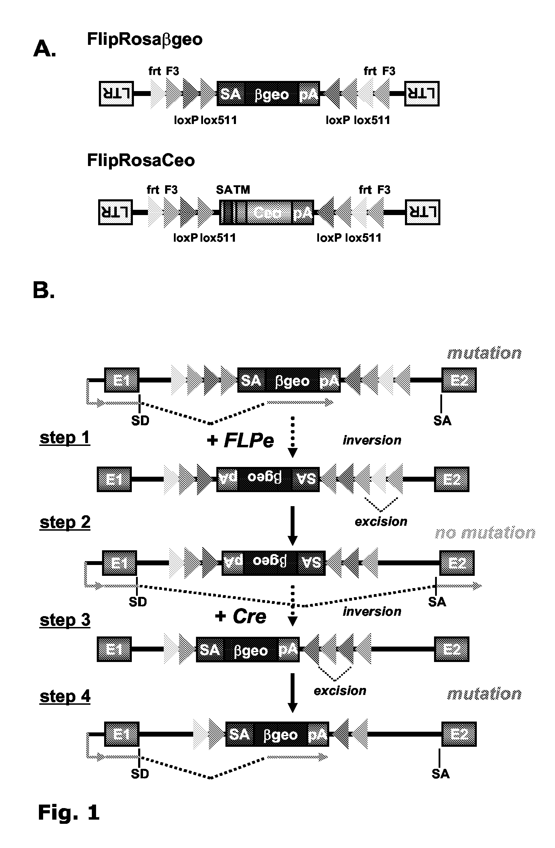

Figure 1 shows conditional gene trap vectors and the mechanism of gene inactivation.
   A: Schematic representation of the retroviral gene trap vectors. LTR, long terminal repeat; frt (yellow triangles) and F3 (green triangles), heterotypic target sequences for the FLPe recombinase; IoxP (red triangles) and lox511 (purple triangles), heterotypic target sequences for the Cre recombinase; SA, splice acceptor; βgeo, β-galactosidase/neomycinphosphotransferase fusion gene; pA, bovine growth hormone polyadenylation sequence; TM, human CD2 receptor transmembrane domain; Ceo, human CD2 cell surface receptor/neomycinphosphotransferase fusion gene.
   B: Conditional gene inactivation by a SApgeopA cassette. The SApgeopA cassette flanked by recombinase target sites (RTs) in a FlEx configuration is illustrated after integration into an intron of an expressed gene. Transcripts (shown as grey arrows) initiated at the endogenous promoter are spliced from the splice donor (SD) of an endogenous exon (here exon 1) to the splice acceptor (SA) of the SApgeopA cassette. Thereby the βgeo reporter gene is expressed and the endogenous transcript is captured and prematurely terminated at the cassette's polyadenylation sequence (pA) causing a mutation. In step 1, FLPe inverts the SApgeopA cassette onto the anti-sense, non-coding strand at either frt (shown) or F3 (not shown) RTs and positions frt and F3 sites between direct repeats of F3 and frt RTs, respectively. By simultaneously excising the heterotypic RTs (step 2), the cassette is locked against re-inversion as the remaining frt and F3 RTs cannot recombine. This reactivates normal splicing between the endogenous splice sites, thereby repairing the mutation. Cre mediated inversion in steps 3 and 4 repositions the SApgeopA cassette back onto the sense, coding strand and reinduces the mutation. Note that the recombination products of steps 1 and 3 are transient and transformed into the stable products of step 2 and 4, respectively (Schnutgen, F. et al., Nat. Biotechnol. 21, 562-5 (2003)).

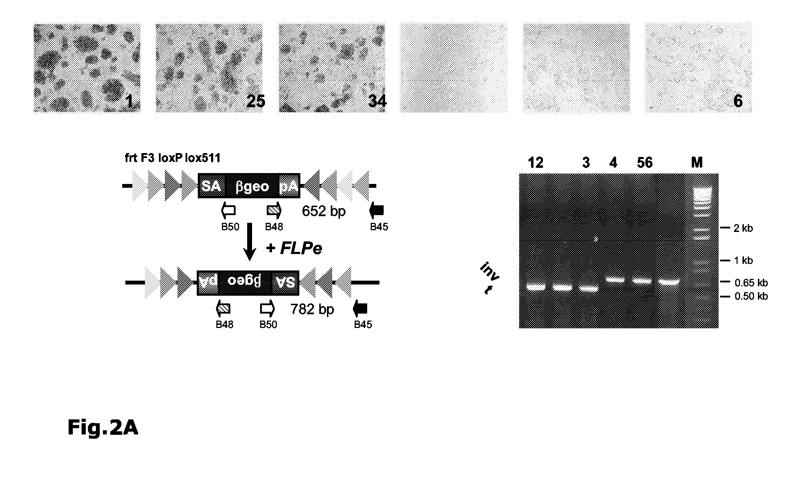

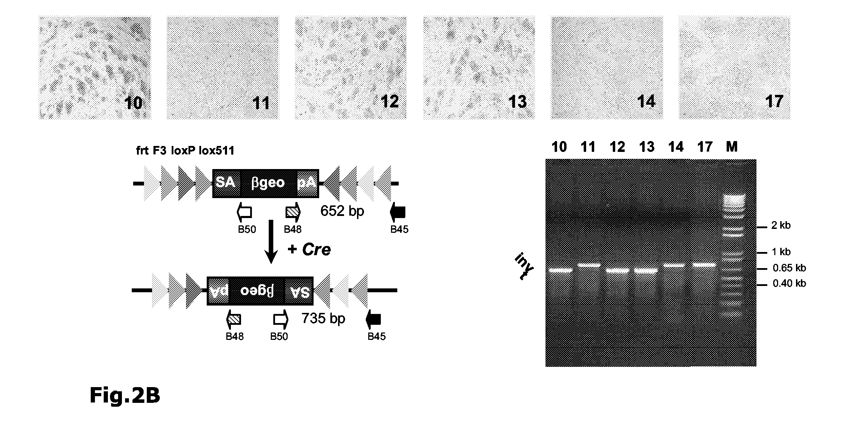

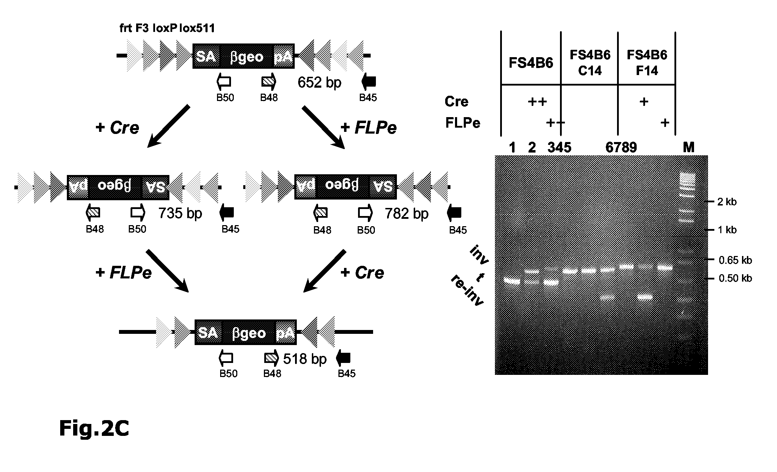

   Figure 2 shows site-specific recombinase induced inversions in FlipRosaβgeo trapped ES cell lines.
   A and B: ES cells were infected with FlipROSAβgeo virus and selected in G418. X-Gal positive sub-lines (blue) were electroporated with FLPe (A) or Cre (B) expression plasmids and stained with X-Gal after incubating for 10 days. DNA extracted from blue and white sub-lines was subjected to a multiplex PCR to identify inversions. Primer positions within FlipRosaβgeo are indicated by large arrows; allele specific amplification products are visualized on ethidium bromide stained gels to the right. Legend: t, trapped allele; inv, inverted allele; M, molecular weight marker (1 kb + ladder, Invitrogen); lanes 1-3, parental FlipRosaβgeo trapped ES cell line; lanes 4-6, FLPe (A) and Cre (B) inverted sub-line.
   C: sub-lines of the FS4B6 ES cell line harboring Cre or FLPe inverted gene trap insertions were electroporated with both FLPe and Cre expression plasmids. The amplification products obtained from the progeny lines by allele specific PCR are visualized on the ethidium bromide stained gel to the right. Legend: t, trapped allele; inv, inverted allele; re-inv, re-inverted allele; M, molecular weight marker (1 kb + ladder, Invitrogen); FS4B6 (lanes 1-3), parental FlipRosaβgeo trapped ES cell line; FS4B6C14 (lanes 4-6), Cre inverted sub-line; FS4B6F14 (lanes 7-9), FLPe inverted sub-line.

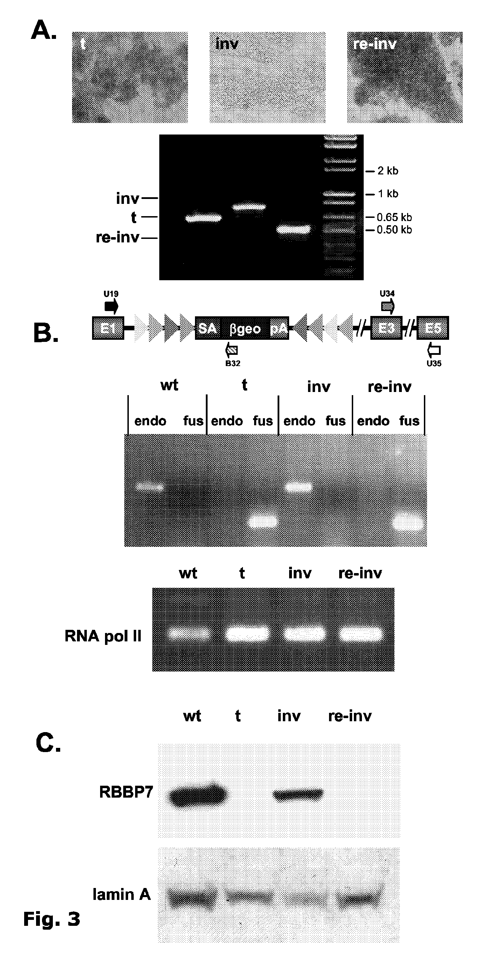

   Figure 3 shows conditional mutation induced by a FlipRosaβgeo gene trap insertion in the RBBP7 gene (ENSEMBL ID: ENSMUSG00000031353). The Q017B06 gene trap cell line (t) was transiently transfected with a FLPe expression plasmid and several sub-lines with inverted gene trap cassettes were identified by X-Gal staining and allele specific PCR (inv). Inverted sub-lines were then electroporated with a Cre expression plasmid and enriched for re-inversions by selecting in G418 (re-inv).
   A: X-Gal staining (top) and allele specific PCR amplification products (bottom) from the trapped RBBP7 locus in trapped (t), inverted (inv) and re-inverted (re-inv) Q017B06 cell lines. Primers used for the multiplex PCR reactions were identical to those shown in the diagrams of Figure 2.
   B: RT-PCR for the amplification of RBBP7 wild-type and trapped fusion transcripts expressed in Q017B06 cells before and after exposure to FLPe and Cre recombinases. The positions of the primers used are shown on top whereby U19 = 5'-GCT CTT GAC TAG CGA GAG AGA AG-3' (SEQ ID NO:12), B32 = 5'-CAA GGC GAT TAA GTT GGG TAA CG-3' (SEQ ID NO: 13), U34 = 5'-CCA GAA GGA AAG GAT TAT GC-3' (SEQ ID NO: 14), and U35 = 5'-ACA GAG CAA ATG ACC CAA GG-3' (SEQ ID NO:15). Amplification products are visualized below on ethidium bromide stained gels. Amplification of the RNA polymerase II transcript (RNApol II) serves as a positive control. wt, parental ES cells; t, trapped Q017B06 cells; inv, inverted Q017B06 sub-line; re-inv, re-inverted Q017B06 sub-line; endo, endogenous transcript; fus, fusion transcript.
   C: Western blot analysis of the RBBP7 protein expressed in Q017B06 cells. Crude cell lysates from the F1 (wt), Q017B06 (t), inverted Q017B06 (inv) and re-inverted Q017B06 (re-inv) ES cells were resolved by SDS-PAGE and analyzed by Western blotting using the anti-RbAp46 antibody. The anti-lamin A antibody served as a loading control.

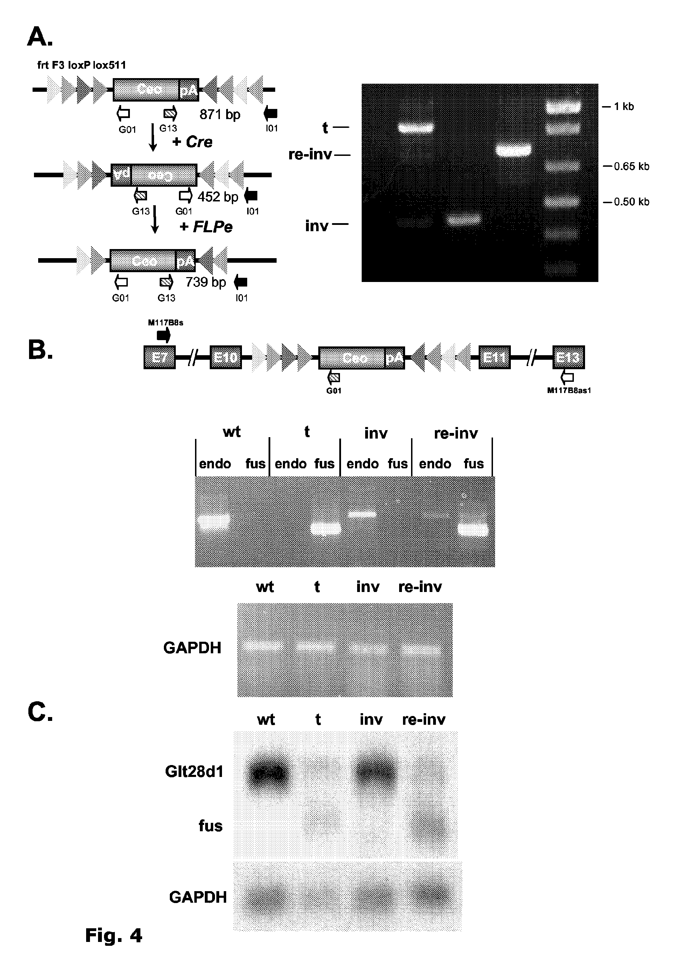

   Figure 4 shows conditional mutation induced by a FlipRosaCeo gene trap insertion in the Glt28d1 gene (ENSEMBL ID: ENSMUST00000040338). The M117B08 gene trap line was treated with recombinases and processed as described for Q017B06 in the Legend to Figure 3, except that Cre was used for the first inversion and FLPe for the second.
   A: Allele specific PCR of the trapped Glt28d1 locus in trapped (t), inverted (inv) and re-inverted cell lines.
   B: RT-PCR of Glt28d1wild type and Glt28d1/gene trap fusion transcripts expressed in M117B08 cells before and after exposure to Cre and FLPe recombinases. The position of the respective primers within the trapped gene are shown on top whereby M117B8s = 5'-GAG AGT GCT GGC CAG CTG GAA C-3' (SEQ ID NO:16), G01 = 5'-CAA GTT GAT GTC CTG ACC CAA G-3' (SEQ ID NO:17), and M117B8as1 = 5'-CCA CCA TAC TCC ACA CAC TCT G-3' (SEQ ID NO:18). Amplification products are visualized on ethidium bromide stained gels below. Amplification of the glyceraldehyde-3-phosphate dehydrogenase (GAPDH) transcript serves as a positive control. wt, parental F1 ES cells; t, trapped M117B08; inv, inverted M117B08 sub-line; re-inv, re-inverted M117B08 sub-line; endo, endogenous transcript; fus, fusion transcript.
   C: Northern blot analysis of Glt28d1 transcripts expressed in M117B08 cells. 2 µg of polyadenylated RNAs from F1 (wt), Q017B06 (t), inverted M117B08 (inv) and re-inverted M117B08 (re-inv) ES cells were fractionated on 1% formaldehyde/agarose gels and hybridized to a ³²P-labeled Git28d1 c-DNA probe. The Git28d1 probe was obtained by asymmetric RT-PCR using a reverse primer in exon 10 to amplify sequences upstream of the insertion site. The loading of each lane was then assessed by using a GAPDH probe. Legend: see Fig. 4B; Git28d1, GIt28d1 transcript.

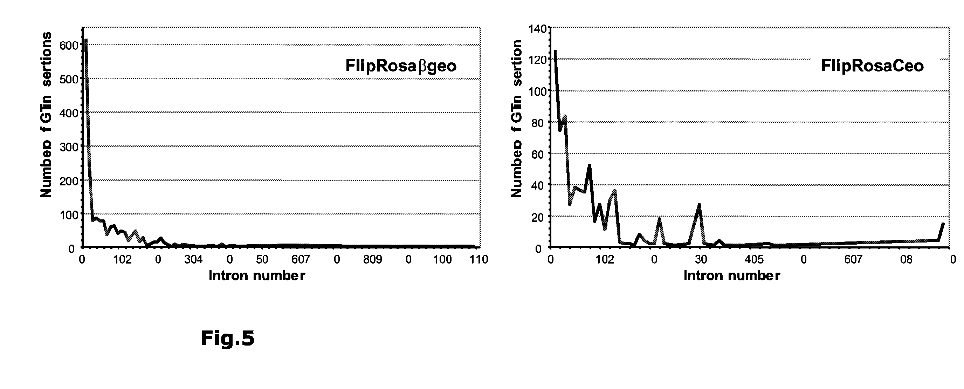

Figure 5 shows the distribution of gene trap (GT) insertions according to the position of the trapped intron within genes. The data are based on NCBI mouse genome build 33 and RefSeq release 8.

### Definitions

"Target Gene" defines a specific gene consisting of exons and at least one intron to be trapped by a gene trap vector.

"Gene disruption and selection cassette (GDSC)" refers to genetic elements comprising from 5' to 3' a splice acceptor sequence, a reporter and/or selection gene and a polyadenylation sequence.

A "further" or "third" recombinase in the context of present application is a recombinase, which does not interfere with said at least two recombination systems of embodiments (1) and (2).

"Gene trapping" refers to a random mutagenesis approach in functional genomics and is based on the random integration of a gene disruption and selection cassette into a genome.

"Gene targeting" is a gene specific mutagensis approach in functional genomics and is based on the insertion of a GDSC in combination with an independently expressed selection cassette into the genome by homologous recombination (this is for targeting non-expressed genes).

"Targeted trapping" refers to a gene specific mutagenesis approach in functional genomics and is based on the insertion of a GDSC into the genome by homologous recombination (this is for targeting expressed genes).

"Gene trap vector" refers to a promoterless gene trapping construct which inserts a GDSC into an intron, so that it induces a fusion transcript with the targeted endogenous gene.

"Reporter gene" refers to a gene encoding for a gene product (e.g. CAT, βgalactosidase, βgeo, GFP, EGFP, alkaline phosphatase) that can be readily detected by standard biochemical assays.

"Selectable marker gene" refers to a gene, which is transduced into a cell (i.e. transfected or infected) where its expression allows for the isolation of gene trap vector-expressing cells in media containing a selecting agent, e.g. neomycin, puromycin, hygromycin, HSV-thymidine kinase.

"PolyA" (A = adenylic acid) refers to a nucleic acid sequence that comprises the AAUAAA consensus sequence, which enables polyadenylation of a processed transcript. In a gene disruption or selection cassette (GDSC), the polyA sequence is located downstream to the reporter and/or selectable marker gene and signals the end of the transcript to the RNA-polymerase.

"Splicing" refers to the process by which non-coding regions (introns) are removed from primary RNA transcripts to produce mature messenger RNA (mRNA) containing only exons.

"5' splice site" (splice donor, SD)" and "3' splice site" (splice acceptor, SA) refer to intron flanking consensus sequences that mark the sites of splicing.

"inversion" refers to a case wherein the GDSC segment is excised from the gene and reinserted in an orientation opposite to its original orientation, so that the gene sequence for the segment is reversed with respect to that of the rest of the chromosome. Said inversions can by accomplished by using recombinase enzymes (e.g. Cre, FLPe).

"ROSA" (Reverse-Orientation-Splice-Acceptor) refers to a gene trap cassette inserted into a retroviral backbone in reverse transcriptional orientation relative to the retrovirus.

"homotypic RRSs" refer to site specific recombinase target sequences that are identical and can recombine with one another in presence of the appropriate recombinase (eg. loxP/loxP, 1ox5171/1ox5171, frt/frt, F3/F3).

"heterotypic RRSs" refer to site specific recombinase target sequences with affinity to the same recombinase (e.g. Cre or FLPe) that are not identical (e.g. loxP/lox5171, frt/F3) and cannot recombine with one another in presence of the appropriate recombinase.

An "organism" in the context of the present invention includes eucaryotes and procaryotes. Eucaryotes witin the meaning of the invention include animals, human beings and plants. Further, the animal is preferably a vertebrate (such as a mammal or fish) or an invertebrate (such as an insect or worm). In one particularly preferred aspect of the invention, the vertebrate is a non-human mammal, such as a rodent, most preferably is a mouse.

"Cells, cell cultures and tissues" in the context of present invention are derived from an organism as defined above.

**Sequence Listing: Detailed Description of Sequence Features**

| SEQ ID NO: | free text | |
|---|---|---|
| 1 | pFIipROSAβgeo | |
| | **Element** | **Position** |
| | F3 | 1391-1438 |
| | frt | 1445-1492 |
| | lox511 | 1543-1576 |
| | loxP | 1623-1656 |
| | bGHpA | 1974-1696 |
| | beta Geo | 5886-1993 |
| | AdSA | 6018-5888 |
| | lox511 | 6068-6101 |
| | loxP | 6148-6181 |
| | F3 | 6232-6279 |
| | frt | 6337-6384 |
| 2 | prFIipROSAβgeo | |
| | **Element** | **Position** |
| | F3 | 1391-1438 |
| | frt | 1445-1492 |
| | lox5171 | 1543-1576 |
| | loxp | 1623-1656 |
| | bGHpA | 1974-1696 |
| | beta Geo | 5886-1993 |
| | AdSA | 6018-5888 |
| | lox5171 | 6068-6101 |
| | loxP | 6148-6181 |
| | F3 | 6232-6279 |
| | frt | 6337-6384 |
| 3 | pFlipROSACeo | |
| | **Element** | **Position** |
| | F3 | 1391-1438 |
| | frt | 1445-1492 |
| | lox511 | 1543-1576 |
| | loxP | 1623-1656 |
| | bGHpA | 1974-1696 |
| | Ceo | 3566-1997 |
| | lox511 | 3581-3614 |
| | loxp | 3661-3694 |
| | F3 | 3745-3792 |
| | frt | 3850-3897 |
| 4 | prFLipROSACeo | |
| | **Element** | **Position** |
| | F3 | 1391-1438 |
| | frt | 1445-1492 |
| | lox5171 | 1543-1576 |
| | loxP | 1623-1656 |
| | bGHpA | 1974-1696 |
| | Ceo | 3566-1997 |
| | Iox5171 | 3581-3614 |
| | IoxP | 3661-3694 |
| | F3 | 3745-3792 |
| | frt | 3850-3897 |
| 5 | loxP | |
| 6 | lox511 | |
| 7 | lox5171 | |
| 8 | lox2272 | |
| 9 | frt | |
| 10 | f3 | |
| 11 | f5 | |
| 12 | U19 | |
| 13 | B32 | |
| 14 | U34 | |
| 15 | U35 | |
| 16 | M117B8s | |
| 17 | G01 | |
| 18 | M117B8as1 | |

### Detailed Description of the Invention

The gene trap cassettes (1) and (2) are hereinafter described in more detail. They preferably comprise the structure
5'-L1-A-L2-B-L3-C-L4-FS-L3-D-L4-E-L1-F-L2-3',
wherein
L1 and L2 are the heterotypic RRSs of the first site-specific recombination system,
L3 and L4 are the heterotypic RRSs of the second site-specific recombination system, both arrayed on either site of the FS in opposite orientations relative to each other and
A to F are independently from each other either a chemical bond or a spacer polynucleotide. Preferably,
(i) B and E are chemical bonds; and/or
(ii) at least either A or F and either C or D is a spacer polynucleotide.
   In other words, spacer polynucleotides are not required between L1 and L2 or between L3 and L4 on both sides of the FS, as spacer polynucleotides on one side are sufficient. Furthermore, there are no spacers required between L2 and L3 or L4 and L1.

It is moreover preferred that in the gene trap cassette the RRSs of said at least two independent directional site-specific recombination systems are recognized by recombinases selected from the site specific recombinases Cre of bacteriophage P1, FLP recombinase of *Saccharomyces cerevisiae,* R recombinase of *Zygosaccharomyces rouxii* pSR1, the A recombinase of *Kluyveromyces drosophilarium* pKD1, the A recombinase of *K. waltii* pKW1, the integrase λ Int, the recombinase of the GIN recombination system of the Mu phage, the bacterial β recombinase, and variants thereof. Preferably, the two recombinases are Cre and FLPe, or their natural or synthetic variants. Most preferably, at least two recombinases are Cre and FLPe.

Site specific recombinase variants refers to derivatives of the wild-type recombinases and/or their coding sequence which are due to truncations, substitions, deletions and/or additions of amino acids or nucleotides, respectively, their respective sequences. Preferably, said variants are due to homologous substitution of amino acids or degenerated codon usage. The said Cre recombinase of bacteriophage P1 (Abremski et al., J Biol Chem 216, 391-6 (1984)) is commercially available.

Furthermore the minimum length of the spacer polynucleotides A to F is 30 nt, preferably 70 nt, most preferably about 86 nt if the two pairs of RRSs are frt/F3 and about 46 nt if the two pairs of RRSs are loxP/lox5171. The spacer nucleotides can be up to several kilobases in length and can be a functional gene or cDNA, such as genes or cDNAs coding for selectable marker and/or reporter proteins.

In a particularly preferred embodiment of the invention one recombinase is Cre recombinase and L1 and L2, or L3 and L4 are selected from LoxP, Lox66, Lox71, Lox511, Lox512, Lox514, Lox5171, Lox2272 and other mutants of LoxP including LoxB, LoxR and LoxL, preferably from LoxP (SEQ ID NO:5), Lox511 (SEQ ID NO: 6), Lox 5171 (SEQ ID NO:7) and Lox2272 (SEQ ID NO:8). More preferably, at least one of L1 and L2, or L3 and L4 is selected from Lox5171 and Lox2272. Most preferably, L1 (or L3) comprises a LoxP sequence as shown in SEQ ID NO:5 and L2 (or L4) comprises a Lox5171 sequence as shown in SEQ ID NO:7, or vice versa, or L1 (or L3) comprises a loxP sequence and L2 (or L4) comprises a lox2272 sequence as shown in SEQ ID NO:8, or vice versa, or L1 (or L3) comprises a Lox5171 and L2 (or L4) comprises a Lox2271 sequence, or vice versa. sequence as shown in SEQ ID NO:7, or vice versa; and/or the other recombinase is FLPe recombinase and L3 and L4, or L1 and L2 are selected from frt, F3 and F5 , preferably L3 (or L1) comprises a frt sequence as shown in SEQ ID NO:9 and L4 (or L2) comprises a F3 sequence as shown in SEQ ID NO:10, or vice versa.

In a preferred embodiment of the invention, the functional DNA segment of the construct (1) further comprises one or more of the following functional elements: splice acceptor, splice donor, internal ribosomal entry site (IRES), polyadenylation sequence, a gene coding for a reporter protein, a toxin, a drug resistance gene and a gene coding for a further site specific recombinase. More preferably, the functional DNA segment comprises at least a splice acceptor and a polyadenylation sequence. Suitable splice acceptors include, but are not limited to, the adenovirus type 2 splice acceptor of exon 2 at positions 6018 to 5888 of SEQ ID NOs:1 and 2; suitable donors include, but are not limited to, the adenovirus exon 1 splice donor; suitable IRES include, but are not limited to, that of the ECM virus; and suitable polyadenylation sequences are the polyadenylation sequence of the bovine growth hormone (bpA or bGHpA) such as the sequence of bpA present in positions 1974-1696 of SEQ ID NOs:1-4.

Suitable reporter genes include, but are not limited to, *E. coli* β-galactosidase, fire fly luciferase, fluorescent proteins (e.g., eGFP) and human placental alkaline phosphatase (PLAP). Suitable resistance genes include, but are not limited to, a preferred aspect, a fusion gene between reporter and resistance gene is used, like the βgalactosidase/neomycinphosphotransferase fusion gene (βGeo) in positions 5886-1993 of SEQ ID NO:1 or the human CD2 cell surface receptor/neomycinphosphotransferase fusion gene (Ceo) in positions 3566-1997 of SEQ ID NO:3.

In a further preferred embodiment of the present invention, the construct (1) further comprises a selection DNA segment suitable for selecting for genes having an incorporated gene trap cassette, said selection DNA segment comprising a reporter or resistance gene and flanking recombinase recognition sites in the same orientation. Suitable resistance genes are those mentioned above, provided, however, that they do not interfere with the resistance gene of the functional DNA segment. Suitable recombinase recognition sites in same orientation include, but are not limited to, loxP and mutants thereof (see SEQ ID NOs:5 to 8), frt and mutants thereof (see SEQ ID NOs:9 to 11), provided, however, that these RRSs do not interfere with the RRSs of the functional segment. Thus, suitable further (third) site specific recombinases are all recombinases mentioned above, which do not interfere with the RRSs of the first and second site-specific recombination system present in the gene trap cassette.

The present invention provides a site specific recombination system which combines gene trap mutagenesis with site-specific recombination to develop an approach suitable for the large scale induction of conditional mutations in ES cells. The strategy is based on a recently described site-specific recombination strategy (FlEx) (Schnutgen, F. et al., Nat. Biotechnol. 21, 562-5 (2003)), which enables directional inversions of gene trap cassettes at the insertion sites. By using gene trap integrations into X-chromosomal genes, we have shown that gene trap vectors equipped with the FlEx system cause mutations that can be repaired and re-induced. Thus, ES cell lines expressing these gene trap vectors can be used for generating mice either with null- or conditional mutations. For example, to obtain straight knock-outs the cell lines can be converted directly into mice by blastocyst injection. However, to obtain conditional mutations, one would first repair the mutation in ES cells, preferentially with FLPe to reserve the more efficient Cre for in vivo recombination, and then proceed to mouse production. Resulting mice would lack germline mutations but would be vulnerable to somatic mutations inducible by Cre. Depending on the type of Cre and the form of its delivery the mutations can be re-activated in prespecified tissues at prespecified times.

Due to the inherent recombinase target sites, the vector insertions create multipurpose alleles enabling a large variety of postinsertional modifications by recombinase mediated cassette exchange (RMCE) (Baer, A. and Bode, J., Curr. Opin. Biotechnol. 12, 473-80 (2001)). Examples include replacing the gene trap cassettes with Cre recombinase genes to expand the Cre-zoo, or with point mutated minigenes to study point mutations. A further option is the insertion of toxin genes for cell lineage specific ablations.

The quality of the conditional mutations induced by the gene trap insertions will largely depend on the gene trap's ability to be neutral from its position on the anti-sense, non-coding strand. While in the two examples described here the anti-sense insertions were innocuous, this will presumably not always be the case. Factors likely to influence the anti-sense neutrality include cryptic splice sites and transcriptional termination signals. In line with this, we have shown previously that aberrant splicing induced by an anti-sense gene trap insertion resulted in a partial gene inactivation and an interesting phenotype (Sterner-Kock, A., Genes Dev. 16, 2264-2273 (2002)). Thus, the most likely outcome of anti-sense insertions that interfere with gene expression are hypomorphic mutations, which have a merit of their own. However, *in silico* analysis failed to identify sequences that might interfere with gene expression from the anti-sense strands of the present vectors, suggesting that the majority of their insertions create *bona fide* conditional alleles.

By using the vectors in high throughput screens, we have assembled the largest library of ES cell lines with conditional mutations of single protein coding genes, including secretory pathway genes. Presently, it contains about 1,000 potentially conditional alleles (Tab. 1 and 2), which is about ten times the number produced within the last ten years by gene targeting.

**Tab. 1: Trapping efficiency with conditional gene trap vectors**

| Gene trap vector | FlipRosaβgeo | FIipRosaCeo | Total |
|---|---|---|---|
| Number of ES cell lines | 3,604 | 921 | 4,525 |
| Number of GTSTs | 3,257 | 881 | 4,138 |
| Number of insertions into annotated genes | 2,944 (90%) | 873 (99%) | 3,817 (92%) |
| Number of unique genes trapped | 924 | 275 | 1,000 |
| Number of "hot spots"** | 505(16%) | 98(11%) | 603 |

| | | | |
|---|---|---|---|
| * Analysis based on NCBI mouse genome build 33 and RefSeq release 8. ** All genes with = 2 insertions were classified as hot spots. | | | |

**Tab.2: Unique genes trapped with**

| A) FlipRosaβgeo vector | | |
|---|---|---|
| **Acc_No** | **Symbol** | **Gene Name** |
| NM_146217 | Aars | alanyl-tRNA synthetase |
| NM_198884 | AB114826 | cDNA sequence AB114826 |
| NM_005845 | ABCC4 | ATP-binding cassette, sub-family C (CFTR/MRP), member 4 |
| NM_015751 | Abce1 | ATP-binding cassette, sub-family E (OABP), member 1 |
| NM_023190 | Acin1 | apoptotic chromatin condensation inducer 1 |
| NM_134037 | Acly | ATP citrate lyase |
| NM_080633 | Aco2 | aconitase 2, mitochondrial |
| NM_019477 | Acsl4 | acyl-CoA synthetase long-chain family member 4 |
| NM_009616 | Adam19 | a disintegrin and metalloproteinase domain 19 (meltrin beta) |
| NM_197985 | Adipor2 | adiponectin receptor 2 |
| NM_134079 | Adk | adenosine kinase |
| NM_015339 | ADNP | activity-dependent neuroprotector |
| NM_009637 | Aebp2 | AE binding protein 2 |
| NM_146036 | Ahsa1 | AHA1, activator of heat shock 90kDa protein ATPase homolog 1 (yeast) |
| NM_198645 | Al413631 | expressed sequence Al413631 |
| NM_178760 | Al790205 | expressed sequence Al790205 |
| NM_019774 | Akap8 | A kinase (PRKA) anchor protein 8 |
| NM_007431 | Akp2 | alkaline phosphatase 2, liver |
| NM_021473 | Akr1a4 | aldo-keto reductase family 1, member A4 (aldehyde reductase) |
| NM_019776 | AL033314 | expressed sequence AL033314 |
| NM_133971 | Ankrd10 | ankyrin repeat domain 10 |
| NM_030886 | Ankrd17 | ankyrin repeat domain 17 |
| NM_009672 | Anp32a | acidic (leucine-rich) nuclear phosphoprotein 32 family, member A |
| NM_130889 | Anp32b | acidic nuclear phosphoprotein 32 family, member B |
| NM_013469 | Anxa11 | annexin A11 |
| NM_009686 | Apbb2 | amyloid beta (A4) precursor protein-binding, family B, member 2 |
| NM_007475 | Arbp | acidic ribosomal phosphoprotein P0 [Mus musculus] |
| NM_145985 | Arcn1 | archain 1 |
| NM_172595 | Arfrp2 | ADP-ribosylation factor related protein 2 |
| NM_133962 | Arhgef18 | rho/rac guanine nucleotide exchange factor (GEF) 18 |
| NM_023598 | Arid5b | AT rich interactive domain 5B (Mrf1 like) |
| NM_011790 | Arih2 | ariadne homolog 2 (Drosophila) |
| NM_011791 | Ash21 | ash2 (absent, small, or homeotic)-like (Drosophila) |
| NM_007494 | Ass1 | argininosuccinate synthetase 1 |
| NM_007497 | Atf1 | activating transcription factor 1 |
| NM_009715 | Atf2 | activating transcription factor 2 |
| NM_030693 | Atf5 | activating transcription factor 5 |
| NM_019426 | Atf7ip | activating transcription factor 7 interacting protein |
| NM_016755 | Atp5j | ATP synthase, H+ transporting, mitochondrial F0 complex, subunit F |
| NM_013795 | Atp5l | ATP synthase, H+ transporting, mitochondrial F0 complex, |
| | | subunit g |
| NM_133826 | Atp6v1h | ATPase, H+ transporting, lysosomal 50/57kDa, V1 subunit H |
| NM_009726 | Atp7a | ATPase, Cu++ transporting, alpha polypeptide |
| NM_009530 | Atrx | alpha thalassemia/mental retardation syndrome X-linked homolog (human) |
| NM_020575 | Axot | axotrophin |
| NM_011793 | Banf1 | barrier to autointegration factor 1 |
| NM_016812 | Banp | Btg3 associated nuclear protein |
| NM_019693 | Bat1a | HLA-B-associated transcript 1A |
| NM_172763 | BB114266 | expressed sequence BB114266 [*Mus musculus*] |
| NM_145397 | BC002059 | cDNA sequence BC002059 |
| NM_145402 | BC003277 | cDNA sequence BC003277 |
| XM_110350 | BC010584 | cDNA sequence BC010584 |
| NM_139065 | BC013481 | cDNA sequence BC013481 |
| NM_145430 | BC017647 | cDNA sequence BC017647 |
| NM_153807 | BC018371 | cDNA sequence BC018371 |
| NM_173748 | BC024322 | cDNA sequence BC024322 |
| NM_145946 | BC025462 | cDNA sequence BC025462 |
| NM_029895 | BC026657 | cDNA sequence BC026657 |
| NM_178059 | BC026657 | cDNA sequence BC026657 |
| NM_145596 | BC031407 | cDNA sequence BC031407 |
| XM_484525 | BC031575 | cDNA sequence BC031575 |
| NM_172758 | BC031853 | cDNA sequence BC031853 |
| XM_140041 | BC032203 | cDNA sequence BC032203 |
| XM_132015 | BC037112 | cDNA sequence BC037112 |
| XM_358340 | BC039282 | cDNA sequence BC039282 |
| NM_007532 | Bcat1 | branched chain aminotransferase 1, cytosolic |
| NM_153787 | Bclaf1 | BCL2-associated transcription factor 1 |
| NM_007544 | Bid | BH3 interacting domain death agonist |
| NM_013481 | Bop1 | block of proliferation 1 |
| NM_009764 | Brca1 | breast cancer 1 |
| NM_020508 | Brd4 | bromodomain containing 4 |
| NM_025788 | Btbd14b | BTB (POZ) domain containing 14B |
| NM_145455 | Btf3 | basic transcription factor 3 |
| NM_009773 | Bub1b | budding uninhibited by benzimidazoles 1 homolog, beta (S. cerevisiae) |
| NM_178684 | C130032J12Rik | RIKEN cDNA C130032J12 gene |
| XM_138091 | C130039O16Rik | RIKEN cDNA C130039O16 gene |
| NM_014837 | C1orf16 | chromosome 1 open reading frame 16 |
| NM_138756 | C330005L02Rik | RIKEN cDNA C330005LO2 gene |
| XM_110478 | C330013J21Rik | RIKEN cDNA C330013J21 gene |
| NM_198676 | C330014B19Rik | RIKEN cDNA C330014B19 gene |
| NM_080562 | C330018L13Rik | RIKEN cDNA C330018L13 gene |
| XM_284552 | C330019L16Rik | RIKEN cDNA C330019L16 gene |
| NM_176897 | C530043A13Rik | RIKEN cDNA C530043A13 gene |
| NM_153547 | C77032 | EST C77032 |
| NM_172578 | C79407 | expressed sequence C79407 |
| NM_177663 | C80587 | expressed sequence C80587 |
| NM_011274 | C80913 | expressed sequence C80913 |
| NM_009786 | Cacybp | calcyclin binding protein |
| NM_007589 | Calm2 | calmodulin 2 |
| NM_007591 | Calr | calreticulin |
| NM_007597 | Canx | calnexin |
| NM_009798 | Capzb | capping protein (actin filament) muscle Z-line, beta |
| NM_009818 | Catna1 | catenin alpha 1 |
| XM_485025 | Catns | PREDICTED: Mus musculus catenin src (Catns), mRNA. |
| NM_172860 | Cbfa2t2h | core-binding factor, runt domain, alpha subunit 2, translocated to, 2 homolog (human) |
| NM_007622 | Cbx1 | chromobox homolog 1 (Drosophila HP1 beta) |
| NM_007626 | Cbx5 | chromobox homolog 5 (Drosophila HP1a) |
| NM_144811 | Cbx7 | chromobox homolog 7 |
| NM_007634 | Ccnf | cyclin F |
| NM_009832 | Ccnk | cyclin K |
| NM_007638 | Cct7 | chaperonin subunit 7 (eta) |
| NM_133655 | Cd81 | CD 81 antigen |
| NM_007657 | Cd9 | CD9 antigen |
| NM_001256 | CDC27 | cell division cycle 27 |
| NM_007659 | Cdc2a | cell division cycle 2 homolog A (S. pombe) |
| NM_001791 | CDC42 | cell division cycle 42 (GTP binding protein, 25kDa) |
| NM_044472 | CDC42 | cell division cycle 42 (GTP binding protein, 25kDa) |
| NM_178626 | Cdc42se2 | CDC42 small effector 2 |
| NM_028023 | Cdca4 | cell division cycle associated 4 |
| NM_009870 | Cdk4 | cyclin-dependent kinase 4 |
| NM_009874 | Cdk7 | cyclin-dependent kinase 7 (homolog of Xenopus M015 cdk-activating kinase) |
| NM_175565 | Cdv3 | carnitine deficiency-associated gene expressed in ventricle 3 |
| NM_175833 | Cdv3 | carnitine deficiency-associated gene expressed in ventricle 3 |
| NM_133869 | Cept1 | choline/ethanolaminephosphotransferase 1 |
| NM_011801 | Cfdp1 | craniofacial development protein 1 |
| NM_178647 | Cggbp1 | CGG triplet repeat binding protein 1 |
| NM_013733 | Chaf1a | chromatin assembly factor 1, subunit A (p150) |
| NM_024166 | Chchd2 | coiled-coil-helix-coiled-coil-helix domain containing 2 |
| NM_001271 | CHD2 | chromodomain helicase DNA binding protein 2 |
| NM_032221 | CHD6 | chromodomain helicase DNA binding protein 6 |
| NM_018818 | Chm | choroidermia |
| NM_007700 | Chuk | conserved helix-loop-helix ubiquitous kinase |
| NM_134141 | Ciapin1 | cytokine induced apoptosis inhibitor 1 |
| NM_007705 | Cirbp | cold inducible RNA binding protein |
| NM_007715 | Clock | circadian locomoter output cycles kaput |
| NM_013493 | Cnbp1 | cellular nucleic acid binding protein 1 |
| NM_028044 | Cnn3 | calponin 3, acidic |
| NM_153585 | Cnot10 | CCR4-NOT transcription complex, subunit 10 |
| NM_028082 | Cnot2 | CCR4-NOT transcription complex, subunit 2 |
| NM_016877 | Cnot4 | CCR4-NOT transcription complex, subunit 4 |
| NM_026949 | Cnot8 | CCR4-NOT transcription complex, subunit 8 |
| NM_181733 | COG5 | component of oligomeric golgi complex 5 |
| NM_009929 | Co118a1 | procollagen, type XVIII, alpha 1 |
| NM_011779 | Coro1c | coronin, actin binding protein 1C |
| NM_009941 | Cox4i1 | cytochrome c oxidase subunit IV isoform 1 |
| NM_183405 | Cox6b2 | cytochrome c oxidase subunit Vib polypeptide 2 |
| NM_053071 | Cox6c | V-src suppressed transcript 3 |
| NM_170588 | Cpne1 | copine I |
| NM_027769 | Cpne3 | copine III |
| NM_016856 | Cpsf2 | cleavage and polyadenylation specific factor 2 |
| NM_007761 | Crcp | calcitonin gene-related peptide-receptor component protein |
| NM_009952 | Creb1 | cAMP responsive element binding protein 1 |
| NM_018776 | Crlf3 | cytokine receptor-like factor 3 |
| NM_027485 | Crsp7 | cofactor required for Sp1 transcriptional activation, subunit 7 |
| NM_001895 | CSNK2A1 | casein kinase 2, alpha 1 polypeptide |
| NM_177559 | CSNK2A1 | casein kinase 2, alpha 1 polypeptide |
| NM_007790 | Cspg6 | chondroitin sulfate proteoglycan 6 |
| NM_007792 | Csrp2 | cysteine and glycine-rich protein 2 |
| NM_145529 | Cstf3 | cleavage stimulation factor, 3' pre-RNA, subunit 3 |
| NM_017368 | Cugbp1 | CUG triplet repeat, RNA binding protein 1 |
| NM_029402 | Cul2 | cullin 2 |
| NM_028288 | Cul4b | cullin 4B |
| NM_146155 | D030015G18Rik | RIKEN cDNA D030015G18 gene |
| NM_172669 | D030051N19Rik | RIKEN cDNA D030051N19 gene |
| NM_025514 | D10Ertd641e | DNA segment, Chr 10, ERATO Doi 641, expressed |
| NM_026023 | D11Ertd603e | DNA segment, Chr 11, ERATO Doi 603, expressed |
| NM_138598 | D11Wsu99e | DNA segment, Chr 11, Wayne State University 99, expressed |
| NM_175299 | D130064I21Rik | RIKEN cDNA D130064I21 gene |
| NM_134100 | D15Mgi27 | DNA Segment, Chr 15, Mouse Genome Informatics 27 |
| NM_198937 | D17Ertd441e | DNA segment, Chr 17, ERATO Doi 441, expressed |
| NM_029456 | D19Ertd703e | DNA segment, Chr 19, ERATO Doi 703, expressed |
| NM_145604 | D230025D16Rik | RIKEN cDNA D230025D16 gene |
| NM_145528 | D2Ertd391e | DNA segment, Chr 2, ERATO Doi 391, expressed |
| NM_212450 | D2Ertd485e | DNA segment, Chr 2, ERATO Doi 485, expressed |
| XM_128090 | D330037H05Rik | RIKEN cDNA D330037H05 gene |
| NM_144901 | D3Jfr1 | DNA segment, Chr 3, Mjeffers 1 |
| NM_027922 | DSErtd585e | DNA segment, Chr 5, ERATO Doi 585, expressed |
| NM_175518 | D730040F13Rik | RIKEN cDNA D730040F13 gene |
| NM_178264 | D830050J10Rik | RIKEN cDNA D830050J10 gene |
| NM_198020 | D8Ertd812e | DNA segment, Chr 8, ERATO Doi 812, expressed |
| NM_010017 | Dag1 | dystroglycan 1 |
| NM_145507 | Dars | aspartyl-tRNA synthetase |
| NM_025705 | Dcbld1 | discoidin, CUB and LCCL domain containing 1 |
| NM_007832 | Dck | deoxycytidine kinase |
| NM_015735 | Ddb1 | damage specific DNA binding protein 1 |
| NM_004818 | DDX23 | DEAD (Asp-Glu-Ala-Asp) box polypeptide 23 |
| NM_197982 | Ddx39 | DEAD (Asp-Glu-Ala-Asp) box polypeptide 39 |
| NM_007840 | Ddx5 | DEAD (Asp-Glu-Ala-Asp) box polypeptide 5 |
| NM_007841 | Ddx6 | DEAD (Asp-Glu-Ala-Asp) box polypeptide 6 |
| NM_007842 | Dhx9 | DEAH (Asp-Glu-Ala-His) box polypeptide 9 |
| XM_372774 | DJ159A19.3 | hypothetical protein DJ159A19.3 |
| NM_011806 | Dmtf1 | cyclin D binding myb-like transcription factor 1 |
| NM_019794 | Dnaja2 | DnaJ (Hsp40) homolog, subfamily A, member 2 |
| NM_011847 | Dnajb6 | DnaJ (Hsp40) homolog, subfamily B, member 6 |
| NM_016775 | Dnajc5 | DnaJ (Hsp40) homolog, subfamily C, member 5 |
| NM_019795 | Dnajc7 | DnaJ (Hsp40) homolog, subfamily C, member 7 |
| NM_030238 | Dnchc1 | dynein, cytoplasmic, heavy chain 1 |
| XM_134573 | Dncli2 | dynein, cytoplasmic, light intermediate polypeptide 2 |
| NM_152816 | Dnm1l | dynamin 1-like |
| NM_007871 | Dnm2 | dynamin 2 |
| NM_010066 | Dnmt1 | DNA methyltransferase (cytosine-5) 1 |
| NM_007879 | Drg1 | developmentally regulated GTP binding protein 1 |
| NM_134448 | Dst | dystonin |
| NM_019771 | Dstn | destrin |
| XM_485355 | E130016E03Rik | RIKEN cDNA E130016E03 gene |
| XM_282906 | E130307A14Rik | RIKEN cDNA E130307A14 gene |
| NM_153548 | E430025E21Rik | RIKEN cDNA E430025E21 gene |
| NM_011816 | E430034L04Rik | RIKEN cDNA E430034L04 gene |
| NM_021876 | Eed | embryonic ectoderm development |
| NM_026007 | Eef1 g | eukaryotic translation elongation factor 1 gamma |
| NM_007907 | Eef2 | eukaryotic translation elongation factor 2 |
| NM_007915 | Ei24 | etoposide induced 2.4 mRNA |
| NM_010120 | Eif1a | eukaryotic translation initiation factor 1A |
| NM_032025 | eIF2A | eukaryotic translation initiation factor (eIF) 2A |
| NM_012199 | EIF2C1 | eukaryotic translation initiation factor 2C, 1 |
| NM_026114 | Eif2s1 | eukaryotic translation initiation factor 2, subunit 1 alpha |
| NM_026030 | Eif2s2 | eukaryotic translation initiation factor 2, subunit 2 (beta) |
| NM_010123 | Eif3s10 | eukaryotic translation initiation factor 3, subunit 10 (theta) |
| NM_133916 | Eif3s9 | eukaryotic translation initiation factor 3, subunit 9 (eta) |
| NM_144958 | Eif4a1 | eukaryotic translation initiation factor 4A1 |
| NM_145625 | Eif4b | eukaryotic translation initiation factor 4B |
| NM_023314 | Eif4e2 | eukaryotic translation initiation factor 4E member 2 |
| NM_023314 | Eif4eI3 | Eukaryotic translation initiation factor 4E like 3 |
| NM_198242 | EIF4G1 | eukaryotic translation initiation factor 4 gamma, 1 |
| NM_013507 | Eif4g2 | eukaryotic translation initiation factor 4, gamma 2 |
| NM_181582 | Eif5a | eukaryotic translation initiation factor 5A |
| NM_001419 | ELAVL1 | ELAV (embryonic lethal, abnormal vision, Drosophila)-like 1 (Hu antigen R) |
| NM_207685 | Elavl2 | ELAV (embryonic lethal, abnormal vision, Drosophila)-like 2 (Hu antigen B) |
| NM_134255 | Elovl5 | ELOVL family member 5, elongation of long chain fatty acids (yeast) |
| NM_130450 | Elovl6 | ELOVL family member 6, elongation of long chain fatty acids (yeast) |
| NM_199466 | Eml4 | echinoderm microtubule associated protein like 4 |
| NM_010135 | Enah | enabled homolog (Drosophila) |
| NM_018212 | ENAH | enabled homolog (Drosophila) |
| NM_007930 | Enc1 | ectodermal-neural cortex 1 |
| NM_023119 | Eno1 | enolase 1, alpha non-neuron |
| NM_207044 | ENSA | endosulfine alpha |
| NM_013512 | Epb4.1l4a | erythrocyte protein band 4.1-like 4a |
| NM_010139 | Epha2 | Eph receptor A2 |
| NM_007936 | Epha4 | Eph receptor A4 |
| XM_129647 | Eprs | glutamyl-prolyl-tRNA synthetase |
| NM_007945 | Eps8 | epidermal growth factor receptor pathway substrate 8 |
| NM_011934 | Esrrb | estrogen related receptor, beta |
| NM_144866 | Etf1 | eukaryotic translation termination factor 1 |
| NM_007964 | Evi5 | ecotropic viral integration site 5 |
| NM_007968 | Ewsr1 | Ewing sarcoma breakpoint region 1 |
| NM_027148 | Exosc8 | exosome component 8 |
| NM_010166 | Eya3 | eyes absent 3 homolog (Drosophila) |
| NM_211357 | Eya3 | eyes absent 3 homolog (Drosophila) |
| NM_172518 | Fbxo42 | F-box protein 42 |
| NM_025995 | Fbxo5 | F-box only protein 5 |
| NM_007999 | Fen1 | flap structure specific endonuclease 1 |
| NM_010206 | Fgfr1 | fibroblast growth factor receptor 1 |
| NM_026218 | Fgfr1op2 | FGFR1 oncogene partner 2 |
| NM_146018 | Flcn | folliculin |
| NM_024953 | FLJ13089 | hypothetical protein FLJ13089 |
| NM_019406 | Fnbp1 | formin binding protein 1 |
| NM_010178 | Fusip1 | FUS interacting protein (serine-arginine rich) 1 |
| NM_008053 | Fxr1h h | fragile X mental retardation gene 1, autosomal homolog |
| NM_198102 | G430041 M01 Rik | RIKEN cDNA G430041 M01 gene |
| NM_010256 | Gart | phosphoribosylglycinamide formyltransferase |
| NM_013525 | Gas5 | growth arrest specific 5 |
| NM_153144 | Ggnbp2 | gametogenetin binding protein 2 |
| NM_010282 | Ggps1 | geranylgeranyl diphosphate synthase 1 |
| NM_010288 | Gja1 | gap junction membrane channel protein alpha 1 |
| NM_009752 | Glb1 | galactosidase, beta 1 |
| XM_136212 | Gli2 | GLI-Kruppel family member GLI2 |
| NM_026247 | Glt28d1 | glycosyltransferase 28 domain containing 1 |
| XM_357972 | Gm1476 | gene model 1476, (NCBI) |
| NM_201366 | Gm1631 | gene model 1631, (NCBI) |
| XM_358591 | Gm1650 | gene model 1650, (NCBI) |
| XM_149164 | Gm559 | gene model 559, (NCBI) |
| NM_027307 | Golph2 | golgi phosphoprotein 2 |
| NM_010324 | Got1 | glutamate oxaloacetate transaminase 1, soluble |
| NM_021610 | Gpa33 | glycoprotein A33 (transmembrane) |
| NM_016739 | Gpiap1 | GPI-anchored membrane protein 1 |
| NM_020331 | Gtf2ird1 | general transcription factor II I repeat domain-containing 1 |
| NM_148934 | Gtrgeo22 | gene trap ROSA b-geo 22 |
| NM_013882 | Gtse1 | G two S phase expressed protein 1 |
| NM_207225 | Hdac4 | histone deacetylase 4 |
| NM_005336 | HDLBP | high density lipoprotein binding protein (vigilin) |
| NM_080446 | Helb | helicase (DNA) B |
| NM_030609 | Hist1h1a | histone 1, H1a |
| NM_015786 | Hist1h1c | histone 1, H1c |
| NM_013820 | Hk2 | hexokinase 2 |
| NM_002131 | HMGA1 | high mobility group AT-hook 1 |
| NM_145903 | HMGA1 | high mobility group AT-hook 1 |
| NM_145942 | Hmgcs1 | 3-hydroxy-3-methylglutaryl-Coenzyme A synthase 1 |
| NM_008258 | Hn1 | hematological and neurological expressed sequence 1 |
| NM_182650 | Hnrpa2b1 | heterogeneous nuclear ribonucleoprotein A2/B1 |
| NM_016884 | Hnrpc | heterogeneous nuclear ribonucleoprotein C |
| NM_007516 | Hnrpd | heterogeneous nuclear ribonucleoprotein D |
| NM 016690 | Hnrpdl | heterogeneous nuclear ribonucleoprotein D-like |
| NM_133834 | Hnrpf | heterogeneous nuclear ribonucleoprotein F |
| NM_025279 | Hnrpk | Heterogeneous nuclear ribonucleoprotein K |
| NM_025279 | Hnrpk | heterogeneous nuclear ribonucleoprotein K |
| NM_028871 | Hnrpr | heterogeneous nuclear ribonucleoprotein R |
| NM_019830 | Hrmt112 | heterogeneous nuclear ribonucleoproteins methyltransferase-like 2 (S. cerevisiae) |
| NM_008300 | Hspa4 | heat shock protein 4 |
| NM_010481 | Hspa9a | heat shock protein, A |
| NM_175111 | Hspbap1 | Hspb associated protein 1 |
| NM_015755 | Hunk | hormonally upregulated Neu-associated kinase |
| NM_031156 | lde | insulin degrading enzyme |
| NM_009951 | lgf2bp1 | insulin-like growth factor 2, binding protein 1 |
| NM_010545 | li | la-associated invariant chain |
| NM_029665 | Ipo11 | importin 11 |
| NM_181517 | Ipo7 | importin 7 |
| NM_172584 | Itpk1 | inositol 1,3,4-triphosphate 5/6 kinase |
| XM_484617 | Itpr3 | inositol 1,4,5-triphosphate receptor 3 |
| NM_023844 | Jam2 | junction adhesion molecule 2 |
| NM_152895 | Jarid1b | jumonji, AT rich interactive domain 1B (Rbp2 like) |
| NM_013668 | Jarid1c | jumonji, AT rich interactive domain 1C (Rbp2 like) |
| NM_021878 | Jarid2 | jumonji, AT rich interactive domain 2 |
| NM_004973 | JARID2 | Jumonji, AT rich interactive domain 2 |
| NM_144787 | Jmjd2c | jumonji domain containing 2C |
| NM_008416 | Junb | Jun-B oncogene |
| NM_053092 | Kars | lysyl-tRNA synthetase |
| NM_019715 | Kcmf1 | potassium channel modulatory factor 1 |
| NM_015210 | KIAA0802 | KIAA0802 |
| NM_016284 | KIAA1007 | KIAA1007 protein |
| NM_010615 | Kif11 | kinesin family member 11 |
| NM_009004 | Kif20a | kinesin family member 20A |
| NM_026167 | KIhl13 | kelch-like 13 (Drosophila) |
| NM_008465 | Kpna1 | karyopherin (importin) alpha 1 |
| NM_010655 | Kpna2 | karyopherin (importin) alpha 2 |
| NM_145993 | L3mbtl2 | 1(3)mbt-like 2 (Drosophila) |
| NM_033565 | Laf41 | lymphoid nuclear protein related to AF4-like |
| NM_010688 | Lasp1 | LIM and SH3 protein 1 |
| NM_133815 | Lbr | lamin B receptor |
| NM_010700 | Ldlr | low density lipoprotein receptor |
| NM_010715 | Lig1 | ligase I, DNA, ATP-dependent |
| NM_025828 | Lman2 | lectin, mannose-binding 2 |
| NM_010721 | Lmnb1 | lamin B1 |
| XM_132499 | Lmtk2 | lemur tyrosine kinase 2 |
| XM_123260 | LOC225307 | similar to Heterogeneous nuclear ribonucleoprotein A1 (Helix-destabilizing protein) (Single-strand binding protein) (hnRNP core protein A1) (HDP-1) (Topoisomerase-inhibitor suppressed) |
| XM_135925 | LOC236864 | similar to UBE2D3 |
| XM_136323 | LOC240853 | similar to ATP synthase, H+ transporting, mitochondrial F0 complex, subunit d |
| XM_145503 | LOC243905 | hypothetical LOC243905 |
| XM_145549 | LOC243955 | similar to hypothetical protein FLJ38281 |
| XM_142564 | LOC245128 | PREDICTED: Mus musculus similar to solute carrier family 7, (cationic amino acid transporter, y+ system), member 3 (LOC245128), mRNA. |
| XM_203729 | LOC277281 | similar to RNP particle component |
| XM_204906 | LOC278757 | similar to hypothetical protein 6720451 E15 |
| XM_283029 | LOC327995 | similar to RNA-binding protein Musashi2-S |
| XM_355157 | LOC381219 | hypothetical LOC381219 |
| XM_355212 | LOC381269 | PREDICTED: Mus musculus similar to hypothetical protein FLJ10116(LOC381269), mRNA. |
| XM_355536 | LOC381575 | similar to RIKEN cDNA 1700029101 |
| XM_355549 | LOC381591 | similar to hypothetical protein FLJ10884 |
| XM_355960 | LOC381936 | similar to Ser/Thr protein kinase PAR-1A |
| XM_356668 | LOC382769 | similar to chromobox homolog 3; heterochromatin protein HP1 gamma; HP1 gamma homolog; heterochromatin-like protein 1; chromobox homolog 3 (Drosophila HP1 gamma) |
| XM_378688 | LOC400607 | hypothetical LOC400607 |
| XM_379174 | LOC401051 | hypothetical LOC401051 |
| XM_483871 | LOC432432 | similar to Heat shock cognate 71 kDa protein |
| XM_488546 | LOC432435 | LOC432435 |
| XM_483955 | LOC432508 | similar to CPSF6 protein |
| XM_483981 | LOC432531 | similar to tensin-like SH2 domain containing 1; tumor endothelial marker 6; thyroid specific PTB domain protein; tensin 3; tensin-like SH2 domain-containing 1; H_NH0549123.2 |
| XM_484135 | LOC432650 | similar to hypothetical protein LOC269211 |
| XM_488646 | LOC432680 | LOC432680 |
| XM_484728 | LOC433182 | similar to Eno1 protein |
| XM_484750 | LOC433205 | similar to ADP-ribosylation factor 1 |
| XM_484773 | LOC433219 | similar to RIKEN cDNA 6330416L07 gene |
| XM_484968 | LOC433399 | similar to RIKEN cDNA C330005L02 |
| XM_358566 | LOC433498 | similar to RIKEN cDNA 9430008C03 gene |
| XM_485110 | LOC433513 | similar to RIKEN cDNA 3300002108 |
| XM_485245 | LOC433598 | similar to histone acetylase complex subunit MRG15-2 |
| XM_485384 | LOC433709 | similar to glyceraldehyde-3-phosphate dehydrogenase (phosphorylating) (EC 1.2.1.12) - mouse |
| XM_485476 | LOC433781 | similar to vacuolar protein sorting 13D |
| XM_485478 | LOC433783 | similar to RIKEN cDNA 1700029101 |
| XM_485482 | LOC433786 | similar to RIKEN cDNA 6330416L07 gene |
| XM_485485 | LOC433789 | similar to RIKEN cDNA 1700029101 |
| XM_485491 | LOC433795 | similar to RIKEN cDNA 2610305D13 |
| XM_485494 | LOC433798 | similar to RIKEN cDNA 1700029101 |
| XM_485498 | LOC433801 | similar to RIKEN cDNA 6330416L07 gene |
| XM_485500 | LOC433804 | similar to RIKEN cDNA 1700029101 |
| XM_485501 | LOC433805 | similar to RIKEN cDNA 6330416L07 gene |
| XM_489078 | LOC433852 | hypothetical gene supported by AK017143 |
| XM_489083 | LOC433871 | LOC433871 |
| XM_485632 | LOC433906 | hypothetical gene supported by AK045300 |
| XM_485662 | LOC433935 | similar to gonadotropin inducible ovarian transcription factor 1 |
| XM_485690 | LOC433955 | similar to histone acetylase complex subunit MRG15-2 |
| XM_489171 | LOC434152 | LOC434152 |
| XM_485924 | LOC434178 | similar to Zinc finger protein 267 (Zinc finger protein HZF2) |
| XM_485962 | LOC434210 | similar to hypothetical protein FLJ25416 |
| XM_489209 | LOC434251 | similar to C-terminal binding protein 2 |
| XM_486096 | LOC434301 | similar to Rho-GTPase-activating protein 7 (Rho-type GTPase-activating protein 7) (Deleted in liver cancer 1 protein homolog) (Dlc-1) (StAR-related lipid transfer protein 12) (StARD12) (START domain-containing protein 12) |
| XM_486133 | LOC434330 | similar to glyceraldehyde-3-phosphate dehydrogenase (phosphorylating) (EC 1.2.1.12) - mouse |
| XM_489245 | LOC434348 | hypothetical gene supported by AK043371 |
| XM_486188 | LOC434373 | similar to Nucleophosmin (NPM) (Nucleolar phosphoprotein B23) (Numatrin) (Nucleolar protein NO38) |
| XM_486329 | LOC434492 | similar to p47 protein isoform a |
| XM_486441 | LOC434596 | similar to CDNA sequence BC002059 |
| XM_486562 | LOC434693 | similar to muscle protein684 |
| XM_486690 | LOC434786 | similar to nuclear receptor co-repressor 1; thyroid hormone- and retinoic acid receptor-associated corepressor 1 |
| XM_486722 | LOC434808 | similar to Non-POU-domain-containing, octamer binding protein |
| XM_489369 | LOC434871 | LOC434871 |
| XM_486835 | LOC434900 | similar to FAM |
| XM_487441 | LOC435488 | similar to ferritin light chain |
| XM_488050 | LOC435987 | similar to Gag-Pol polyprotein |
| XM_289867 | LOC436498 | PREDICTED: Mus musculus similar to RT1 class I, M5 (LOC436498), mRNA. |
| XM_489880 | LOC436548 | similar to RT1 class I, M6, gene 2 |
| XM_495826 | LOC439979 | similar to jumonji domain containing 1A; testis-specific protein A; zinc finger protein |
| XM_499016 | LOC441111 | LOC441111 |
| NM_030695 | Lrba | LPS-responsive beige-like anchor |
| NM_146164 | Lrch4 | leucine-rich repeats and calponin homology (CH) domain containing 4 |
| NM_178701 | Lrrc5 | leucine rich repeat containing 5 |
| NM_175641 | Ltbp4 | latent transforming growth factor beta binding protein 4 |
| NM_028190 | Luc7I | Luc7 homolog (S. cerevisiae)-like |
| NM_138680 | Luc7I2 | LUC7-like 2 (S. cerevisiae) |
| NM_024452 | Luzp1 | leucine zipper protein 1 |
| NM_008866 | Lypla1 | lysophospholipase 1 |
| NM_010749 | M6pr | mannose-6-phosphate receptor, cation dependent |
| NM_007358 | M96 | likely ortholog of mouse metal response element binding transcription factor 2 |
| XM_110503 | Macf1 | microtubule-actin crosslinking factor 1 |
| NM_028108 | Mak3 | Mak3 homolog (S. cerevisiae) |
| NM_027288 | Manba | mannosidase, beta A, lysosomal |
| XM_130628 | Manbal | mannosidase, beta A, lysosomal-like |
| NM_008927 | Map2k1 | mitogen activated protein kinase kinase 1 |
| NM_177345 | Mapkap1 | mitogen-activated protein kinase associated protein 1 |
| NM_010838 | Mapt | microtubule-associated protein tau |
| NM_145569 | Mat2a | methionine adenosyltransferase II, alpha |
| NM_010771 | Matr3 | Matrin 3 |
| NM_018834 | MATR3 | matrin 3 |
| NM_013595 | Mbd3 | methyl-CpG binding domain protein 3 |
| NM_008568 | Mcm7 | minichromosome maintenance deficient 7 (S. cerevisiae) |
| NM_145229 | Mcpr1 | cleft palate-related protein 1 |
| NM_008575 | Mdm4 | transformed mouse 3T3 cell double minute 4 |
| XM_131338 | Mdn1 | midasin homolog (yeast) |
| NM_004992 | MECP2 | methyl CpG binding protein 2 (Rett syndrome) |
| NM_026039 | Med18 | mediator of RNA polymerase II transcription, subunit 18 homolog (yeast) |
| NM_172293 | MGC47262 | hypothetical protein MGC47262 |
| NM_175238 | MGI:1098622 | Rap1 interacting factor 1 homolog (yeast) |
| NM_013716 | MGI:1351465 | Ras-GTPase-activating protein SH3-domain binding protein |
| NM_026375 | MGI:1915033 | embryonic large molecule derived from yolk sac |
| NM_025372 | MGI:1921571 | timeless interacting protein |
| NM_053102 | MGI:1927947 | selenoprotein |
| NM_019736 | MGI:1928939 | acyl-Coenzyme A thioesterase 2, mitochondrial |
| NM_019643 | MGI:1929091 | teratocarcinoma expressed, serine rich |
| NM_019766 | MGI:1929282 | telomerase binding protein, p23 |
| NM_030730 | MGI:1933196 | steroid receptor-interacting SNF2 domain protein |
| NM_031405 | MGI:1933527 | arsenate resistance protein 2 |
| NM_008602 | Miz1 | Msx-interacting-zinc finger |
| NM_018810 | Mkrn1 | makorin, ring finger protein, 1 |
| XM_139743 | MIIt4 | myeloid/lymphoid or mixed lineage-leukemia translocation to 4 homolog (Drosophila) |
| NM_024431 | Morf4I1 | mortality factor 4 like 1 |
| NM_026851 | MrpI52 | mitochondrial ribosomal protein L52 |
| NM_010830 | Msh6 | mutS homolog 6 (E. coli) |
| NM_054043 | Msi2h | Musashi homolog 2 (Drosophila) |
| NM_054082 | Mta3 | metastasis associated 3 |
| NM_008633 | Mtap4 | microtubule-associated protein 4 |
| NM_134092 | Mtbp | Mdm2, transformed 3T3 cell double minute p53 binding protein |
| NM_013827 | Mtf2 | metal response element binding transcription factor 2 |
| NM_016969 | Myadm | myeloid-associated differentiation marker |
| NM_008652 | Mybl2 | myeloblastosis oncogene-like 2 |
| NM_022410 | Myh9 | myosin heavy chain lX |
| NM_023402 | Mylc2b | myosin light chain, regulatory B |
| NM_177619 | Myst2 | MYST histone acetyltransferase 2 |
| NM_013608 | Naca | nascent polypeptide-associated complex alpha polypeptide |
| XM_132755 | Nanog | Nanog homeobox |
| NM_008672 | Nap114 | nucleosome assembly protein 1-like 4 |
| NM_016777 | Nasp | nuclear autoantigenic sperm protein (histone-binding) |
| NM_010878 | Nck1 | non-catalytic region of tyrosine kinase adaptor protein 1 |
| NM_010880 | Ncl | nucleolin |
| NM_008679 | Ncoa3 | nuclear receptor coactivator 3 |
| NM_145518 | Ndufs1 | NADH dehydrogenase (ubiquinone) Fe-S protein 1 |
| NM_029272 | Ndufs7 | NADH dehydrogenase (ubiquinone) Fe-S protein 7 |
| XM_486230 | Nedd4 | neural precursor cell expressed, developmentally down-regulted gene 4 |
| NM_023739 | Nfx1 | nuclear transcription factor, X-box binding 1 |
| NM_010913 | Nfya | nuclear transcription factor-Y alpha |
| NM_002505 | NFYA | nuclear transcription factor Y, alpha |
| NM_010914 | Nfyb | nuclear transcription factor-Y beta |
| NM_008692 | Nfyc | nuclear transcription factor-Y gamma |
| NM_008695 | Nid2 | nidogen 2 |
| NM_133433 | NIPBL | Nipped-B homolog (Drosophila) |
| NM_175460 | Nmnat2 | nicotinamide nucleotide adenylyltransferase 2 |
| NM_008707 | Nmt1 | N-myristoyltransferase 1 |
| NM_013611 | Nodal | nodal |
| NM_018868 | Nol5 | nucleolar protein 5 |
| NM_023144 | Nono | non-POU-domain-containing, octamer binding protein |
| NM_019459 | Nphs1 | nephrosis 1 homolog, nephrin (human) |
| NM_010938 | Nrf1 | nuclear respiratory factor 1 |
| NM_008739 | Nsd1 | Nuclear receptor-binding SET-domain protein 1 |
| NM_008739 | Nsd1 | nuclear receptor-binding SET-domain protein 1 |
| NM_198326 | Nsfl1c | NSFL1 (p97) cofactor (p47) |
| NM_145354 | Nsun2 | NOL1/NOP2/Sun domain family 2 |
| NM_010947 | Ntn3 | netrin 3 |
| NR_001572 | Nudc-ps1 | Mus musculus nuclear distribution gene C homolog (Aspergillus), pseudogene 1 (Nudc-ps1) on chromosome 8. |
| NM_133947 | Numa1 | nuclear mitotic apparatus protein 1 |
| NM_183392 | Nup54 | nucleoporin 54 |
| NM_172394 | Nup88 | nucleoporin 88 |
| XM_284333 | Nup98 | nucleoporin 98 |
| NM_018745 | Oazin | ornithine decarboxylase antizyme inhibitor |
| NM_023429 | Ociad1 | OCIA domain containing 1 |
| NM_002540 | ODF2 | outer dense fiber of sperm tails 2 |
| NM_011015 | Orc11 | origin recognition complex, subunit 1-like (S.cereviaiae) |
| NM_011958 | Orc4I | origin recognition complex, subunit 4-like (S. cerevisiae) |
| NM_019716 | Orc6I | origin recognition complex, subunit 6-like (S. cerevisiae) |
| NM_029565 | ORF18 | open reading frame 18 |
| NM_148908 | OSBPL9 | oxysterol binding protein-like 9 |
| NM_019402 | Pabpn1 | poly(A) binding protein, nuclear 1 |
| NM_013625 | Pafah1b1 | platelet-activating factor acetylhydrolase, isoform 1b, beta1 subunit |
| NM_025939 | Paics | phosphoribosylaminoimidazole carboxylase, phosphoribosylaminoribosylaminoimidazole, succinocarboxamide synthetase |
| NM_016480 | PAIP2 | poly(A) binding protein interacting protein 2 |
| NM_026420 | Paip2 | polyadenylate-binding protein-interacting protein 2 |
| NM_027470 | Pak4 | p21 (CDKN1A)-activated kinase 4 |
| NM_011112 | Papola | poly (A) polymerase alpha |
| NM_020569 | Park7 | Parkinson disease (autosomal recessive, early onset) 7 |
| NM_028761 | Parn | poly(A)-specific ribonuclease (deadenylation nuclease) |
| XM_125814 | Pawr | PRKC, apoptosis, WT1, regulator |
| NM_011042 | Pcbp2 | poly(rC) binding protein 2 |
| NM_023662 | Pcm1 | pericentriolar material 1 |
| NM_011045 | Pcna | proliferating cell nuclear antigen |
| XM_132579 | Pcnp | PEST-containing nuclear protein |
| XM_132501 | Pdap1 | PDGFA associated protein 1 |
| NM_019781 | Pex14 | peroxisomal biogenesis factor 14 |
| XM_111232 | Pfas | phosphoribosylformylglycinamidine synthase (FGAR amidotransferase) |
| NM_019703 | Pfkp | phosphofructokinase, platelet |
| NM_172303 | Phf17 | PHD finger protein 17 |
| NM_172303 | Phf17 | PHD finger protein 17 |
| XM_129836 | Phf3 | PHD finger protein 3 |
| NM_026737 | Phf5a | PHD finger protein 5A |
| NM_172992 | Phtf2 | putative homeodomain transcription factor 2 |
| NM_199026 | Pigl | phosphatidylinositol glycan, class L |
| NM_023371 | Pin1 | protein (peptidyl-prolyl cis/trans isomerase) NIMA-interacting |
| NM_008847 | Pip5k1b | phosphatidylinositol-4-phosphate 5-kinase, type 1 beta |
| NM_145823 | Pitpnc1 | phosphatidylinositol transfer protein, cytoplasmic 1 |
| NM_011099 | Pkm2 | Pyruvate kinase, muscle |
| NM_011099 | Pkm2 | pyruvate kinase, muscle |
| NM_197976 | PKNOX1 | PBX/knotted 1 homeobox 1 |
| NM_026361 | Pkp4 | Mus musculus plakophilin 4 (Pkp4), mRNA. |
| NM_021622 | PLEKHA1 | pleckstrin homology domain containing, family A (phosphoinositide binding specific) member 1 |
| NM_175175 | Plekhf2 | pleckstrin homology domain containing, family F (with FYVE domain) member 2 |
| NM_183034 | Plekhm1 | pleckstrin homology domain containing, family M (with RUN domain) member 1 |
| NM_008891 | Pnn | pinin |
| NM_207171 | POGZ | pogo transposable element with ZNF domain |
| NM_178627 | Poldip3 | polymerase (DNA-directed), delta interacting protein 3 |
| NM_012048 | Polk | polymerase (DNA directed), kappa |
| NM_025298 | Polr3e | polymerase (RNA) III (DNA directed) polypeptide E |
| NM_152894 | Pop1 | processing of precursor 1, ribonuclease P/MRP family, (S. |
| NM_008910 | Ppm1a | cerevisiae) Protein phosphatase 1A, magnesium dependent, alpha isoform |
| NM_013636 | Ppp1cc | protein phosphatase 1, catalytic subunit, gamma isoform |
| NM_017374 | Ppp2cb | protein phosphatase 2a, catalytic subunit, beta isoform |
| NM_002717 | PPP2R2A | protein phosphatase 2 (formerly 2A), regulatory subunit B (PR 52), alpha isoform |
| NM_026391 | Ppp2r2d | protein phosphatase 2, regulatory subunit B, delta isoform |
| NM_002719 | PPP2R5C | protein phosphatase 2, regulatory subunit B (B56), gamma isoform |
| NM_000945 | PPP3R1 | protein phosphatase 3 (formerly 2B), regulatory subunit B, 19kDa, alpha isoform (calcineurin B, type I) |
| NM_024209 | Ppp6c | protein phosphatase 6, catalytic subunit |
| NM_145150 | Prc1 | protein regulator of cytokinesis 1 |
| NM_022115 | PRDM15 | PR domain containing 15 |
| NM_011034 | Prdx1 | peroxiredoxin 1 |
| NM_027230 | Prkcbp1 | protein kinase C binding protein 1 |
| NM_172270 | Prkcbp1 | protein kinase C binding protein 1 |
| NM_008945 | Psmb4 | proteasome (prosome, macropain) subunit, beta type 4 |
| NM_002807 | PSMD1 | proteasome (prosome, macropain) 26S subunit, non-ATPase, 1 |
| NM_021526 | Psmd14 | proteasome (prosome, macropain) 26S subunit, non-ATPase, 14 |
| NM_008956 | Ptbp1 | polypyrimidine tract binding protein 1 |
| NM_002819 | PTBP1 | polypyrimidine tract binding protein 1 |
| NM_011213 | Ptprf | protein tyrosine phosphatase, receptor type, F |
| NM_145925 | Pttg1ip | pituitary tumor-transforming 1 interacting protein |
| NM_030722 | Pum1 | pumilio 1 (Drosophila) |
| NM_030723 | Pum2 | pumilio 2 (Drosophila) |
| NM_008990 | Pvrl2 | poliovirus receptor-related 2 |
| XM_488744 | Pvt1 | plasmacytoma variant translocation 1 |
| NM_008996 | Rab1 | RAB1, member RAS oncogene family |
| NM_016322 | RAB14 | RAB14, member RAS oncogene family |
| NM_181070 | Rab18 | RAB18, member RAS oncogene family |
| NM_004161 | RAB1A | RAB1A, member RAS oncogene family |
| NM_009005 | Rab7 | RAB7, member RAS oncogene family |
| NM_019773 | Rab9 | RAB9, member RAS oncogene family |
| NM_011231 | Rabggtb | RAB geranylgeranyl transferase, b subunit |
| NM_009011 | Rad23b | RAD23b homolog (S. cerevisiae) |
| NM_011236 | Rad52 | RAD52 homolog (S. cerevisiae) |
| NM_029780 | Raf1 | v-raf-1 leukemia viral oncogene 1 |
| NM_011973 | Rage | renal tumor antigen |
| NM_023130 | Raly | hnRNP-associated with lethal yellow |
| NM_011239 | Ranbp1 | RAN binding protein 1 |
| NM_023146 | Ranbp17 | RAN binding protein 17 |
| NM_023579 | Ranbp5 | RAN binding protein 5 |
| NM_011241 | Rangap1 | RAN GTPase activating protein 1 |
| NM_054050 | Rapgef1 | Rap guanine nucleotide exchange factor (GEF) 1 |
| NM_172517 | Rbbp5 | retinoblastoma binding protein 5 |
| NM_009031 | Rbbp7 | retinoblastoma binding protein 7 |
| NM_019733 | Rbpms | RNA binding protein gene with multiple splicing |
| NM_028030 | Rbpms2 | RNA binding protein with multiple splicing 2 |
| NM_009035 | Rbpsuh | recombining binding protein suppressor of hairless (Drosophila) |
| XM_204015 | Rere | arginine glutamic acid dipeptide (RE) repeats |
| NM_009051 | Rex2 | reduced expression 2 |
| NM_053075 | Rheb | RAS-homolog enriched in brain |
| NM_016802 | Rhoa | ras homolog gene family, member A |
| NM_033604 | Rnf111 | ring finger 111 |
| NM_011278 | Rnf4 | ring finger protein 4 |
| NM_133242 | Rnpc2 | RNA-binding region (RNP1, RRM) containing 2 |
| NM_184241 | RNPC2 | RNA-binding region (RNP1, RRM) containing 2 |
| NM_13846 | Ror2 | receptor tyrosine kinase-like orphan receptor 2 [*Mus musculus*] |
| NM_011284 | Rpa2 | replication protein A2 |
| NM_009438 | Rpl13a | ribosomal protein L13a |
| XM_194410 | Rpl18a | Ribosomal protein L18A |
| NM_019674 | Rpl21 | Ribosomal protein L21 |
| NM_009080 | Rpl26 | ribosomal protein L26 |
| NM_025433 | Rpl7l1 | ribosomal protein L7-like 1 |
| NM_181730 | Rpo1-3 | RNA polymerase 1-3 |
| NM_020600 | Rps14 | ribosomal protein S14 |
| NM_029767 | Rps9 | ribosomal protein S9 |
| NM_021383 | Rqcd1 | rcd1 (required for cell differentiation) homolog 1 (S. pombe) |
| NM_009103 | Rrm1 | ribonucleotide reductase M1 |
| NM_019743 | Rybp | RING1 and YY1 binding protein |
| NM_175303 | Sall4 | sal-like 4 (Drosophila) |
| NM_025535 | Sara2 | SAR1a gene homolog 2 (S. cerevisiae) |
| XM_355637 | Sbno1 | sno, strawberry notch homolog 1 (Drosophila) |
| NM_019575 | Scamp4 | secretory carrier membrane protein 4 |
| NM_029023 | Scpep1 | serine carboxypeptidase 1 |
| NM_011341 | Sdf4 | stromal cell derived factor 4 |
| NM_009146 | Sdfr2 | stromal cell derived factor receptor 2 |
| NM_013659 | Sema4b | sema domain, immunoglobulin domain (Ig), transmembrane domain I and short cytoplasmic domain, (semaphorin) 4B |
| NM_027838 | Senp8 | SUMO/sentrin specific protease family member 8 |
| NM_144907 | Sesn2 | sestrin 2 |
| NM_023871 | Set | SET translocation |
| NM_018877 | Setdb1 | SET domain, bifurcated 1 |
| NM_013651 | Sf3a2 | splicing factor 3a, subunit 2 |
| NM_133953 | Sf3b3 | splicing factor 3b, subunit 3 |
| NM_177386 | Sfmbt2 | Scm-like with four mbt domains 2 |
| NM_009186 | Sfrs10 | splicing factor, arginine/serine-rich 10 (transformer 2 homolog, Drosophila) |
| NM_172755 | Sfrs14 | splicing factor, arginine/serine-rich 14 |
| NM_013663 | Sfrs3 | splicing factor, arginine/serine-rich 3 (SRp20) |
| NM_026499 | Sfrs6 | splicing factor, arginine/serine-rich 6 |
| NM_146083 | Sfrs7 | splicing factor, arginine/serine-rich 7 |
| NM_011361 | Sgk | serum/glucocorticoid regulated kinase |
| NM_133816 | Sh3bp4 | SH3-domain binding protein 4 |
| NM_011543 | Skp1a | S-phase kinase-associated protein 1A |
| NM_015747 | SIc20a1 | solute carrier family 20, member 1 |
| NM_011394 | Slc20a2 | solute carrier family 20, member 2 |
| NM_144856 | Slc22a7 | solute carrier family 22 (organic anion transporter), member 7 |
| NM_015829 | SIc25a13 | solute carrier family 25 (mitochondrial carrier, adenine nucleotide translocator), member 13 |
| NM_134086 | SIc38a1 | solute carrier family 38, member 1 |
| NM_027052 | Slc38a4 | solute carrier family 38, member 4 |
| NM_144808 | Slc39a14 | solute carrier family 39 (zinc transporter), member 14 |
| NM_008577 | Slc3a2 | solute carrier family 3 (activators of dibasic and neutral |
| | | amino acid transport), member 2 |
| NM_009320 | Slc6a6 | solute carrier family 6 (neurotransmitter transporter, taurine), member 6 |
| NM_007513 | Slc7a1 | solute carrier family 7 (cationic amino acid transporter, y+ system), member 1 |
| NM_178371 | Slc9a8 | solute carrier family 9 (sodium/hydrogen exchanger), member 8 |
| NM_010754 | Smad2 | MAD homolog 2 (Drosophila) |
| NM_011417 | Smarca4 | SWI/SNF related, matrix associated, actin dependent regulator of chromatin, subfamily a, member 4 |
| XM_132597 | Smarcad1 | SWI/SNF-related, matrix-associated actin-dependent regulator of chromatin, subfamily a, containing DEAD/H box 1' |
| NM_133786 | Smc4I1 | SMC4 structural maintenance of chromosomes 4-like 1 (yeast) |
| NM_027188 | Smyd3 | SET and MYND domain containing 3 |
| NM_009222 | Snap23 | synaptosomal-associated protein 23 |
| XM_133225 | Snrpd2 | small nuclear ribonucleoprotein D2 |
| NM_026095 | Snrpd3 | small nuclear ribonucleoprotein D3 |
| NM_007707 | Socs3 | suppressor of cytokine signaling 3 |
| NM_013672 | Sp1 | trans-acting transcription factor 1 |
| NM_146043 | Spin | spindlin |
| NM_033523 | Spred2 | sprouty protein with EVH-1 domain 2, related sequence |
| NM_011897 | Spry2 | sprouty homolog 2 (Drosophila) |
| NM_011898 | Spry4 | sprouty homolog 4 (Drosophila) |
| NM_016333 | SRRM2 | serine/arginine repetitive matrix 2 |
| NM_175229 | Srrm2 | serine/arginine repetitive matrix 2 |
| NM_009278 | Ssb | Sjogren syndrome antigen B |
| NM_009282 | Stag1 | stromal antigen 1 |
| NM_011490 | Stau1 | staufen (RNA binding protein) homolog 1 (Drosophila) |
| NM_134115 | Stk38 | serine/threonine kinase 38 |
| XM_358343 | Sulf2 | sulfatase 2 |
| NM_009460 | Sumo1 | SMT3 suppressor of mif two 3 homolog 1 (yeast) |
| NM_019929 | Sumo3 | SMT3 suppressor of mif two 3 homolog 3 (yeast) |
| NM_009298 | Surf6 | surfeit gene 6 |
| NM_144871 | Suv420h1 | suppressor of variegation 4-20 homolog 1 (Drosophila) |
| NM_006372 | SYNCRIP | synaptotagmin binding, cytoplasmic RNA interacting protein |
| NM_019666 | Syncrip | synaptotagmin binding, cytoplasmic RNA interacting protein |
| NM_027427 | Taf15 | TAF15 RNA polymerase II, TATA box binding protein (TBP)-associated factor |
| NM_133966 | Taf5l | TAF5-like RNA polymerase II, p300/CBP-associated factor (PCAF)-associated factor |
| NM_027139 | Taf9 | TAF9 RNA polymerase II, TATA box binding protein (TBP)-associated factor |
| XM_043492 | TANC | TPR domain, ankyrin-repeat and coiled-coil-containing |
| NM_009319 | Tarbp2 | TAR (HIV) RNA binding protein 2 |
| NM_145556 | Tardbp | TAR DNA binding protein |
| NM_178337 | Tbce | tubulin-specific chaperone e |
| NM_019786 | Tbk1 | TANK-binding kinase 1 |
| NM_030732 | Tbl1xr1 | transducin (beta)-like 1X-linked receptor 1 |
| NM_134011 | Tbrg4 | transforming growth factor beta regulated gene 4 |
| NM_26456 | Tceb1 | Transcription elongation factor B (SIII), polypeptide 1 |
| NM_019512 | Tcerg1 | transcription elongation regulator 1(CA150) |
| NM_011561 | Tdg | thymine DNA glycosylase |
| NM_021480 | Tdh | L-threonine dehydrogenase |
| NM_009346 | Tead1 | TEA domain family member 1 |
| XM_109868 | Tens1 | tensin-like SH2 domain containing 1 |
| NM_198292 | Tex2 | testis expressed gene 2 |
| NM_011638 | Tfrc | transferrin receptor |
| NM_009372 | Tgif | TG interacting factor |
| NM 009372 | Tgif | TG interacting factor |
| NM_022065 | THADA | thyroid adenoma associated |
| NM_146153 | Thrap3 | thyroid hormone receptor associated protein 3 |
| NM_011585 | Tia1 | cytotoxic granule-associated RNA binding protein 1 |
| XM_358883 | Tiam1 | PREDICTED: Mus musculus T-cell lymphoma invasion and metastasis 1 (Tiam1), mRNA. |
| NM_016897 | Timm23 | translocase of inner mitochondrial membrane 23 homolog (yeast) |
| NM_011597 | Tjp2 | tight junction protein 2 |
| NM_172664 | Tlk1 | tousled-like kinase 1 |
| NM_012290 | TLK1 | tousled-like kinase 1 |
| XM_132970 | Tm7sf3 | transmembrane 7 superfamily member 3 |
| NM_020275 | Tnfrsf10b | tumor necrosis factor receptor superfamily, member 10b |
| NM_178716 | Tnpo1 | transportin 1 |
| NM_145390 | Tnpo2 | Transportin 2 (importin 3, karyopherin beta 2b) |
| NM_146112 | Tnrc15 | trinucleotide repeat containing 15 |
| NM_024214 | Tomm20 | translocase of outer mitochondrial membrane 20 homolog (yeast) |
| NM_138599 | Tomm70a | translocase of outer mitochondrial membrane 70 homolog A (yeast) |
| NM_009408 | Top1 | topoisomerase (DNA) I |
| NM_009412 | Tpd52 | tumor protein D52 |
| NM_022314 | Tpm3 | tropomyosin 3, gamma |
| NM_009429 | Tpt1 | tumor protein, translationally-controlled 1 |
| NM_028109 | Tpx2 | TPX2, microtubule-associated protein homolog (Xenopus laevis) |
| NM_025863 | Trim59 | tripartite motif-containing 59 |
| XM_376178 | TRIP12 | PREDICTED: Homo sapiens thyroid hormone receptor interactor 12(TRIP12), mRNA. |
| NM_011640 | Trp53 | transformation related protein 53 |
| NM_021897 | Trp53inp1 | transformation related protein 53 inducible nuclear protein 1 |
| NM_009445 | Ttk | Ttk protein kinase |
| XM_131709 | Txln | PREDICTED: Mus musculus taxilin (Txln), mRNA. |
| NM_024187 | U2af1 | U2 small nuclear ribonucleoprotein auxiliary factor (U2AF) 1 |
| NM_178794 | U2af1-rs2 | U2 small nuclear ribonucleoprotein auxiliary factor (U2AF) 1, related sequence 2 |
| NM_007279 | U2AF2 | U2 (RNU2) small nuclear RNA auxiliary factor 2 |
| NM_026872 | Ubap2 | ubiquitin-associated protein 2 |
| NM_025985 | Ube2g1 | ubiquitin-conjugating enzyme E2G 1 (UBC7 homolog, C. elegans) |
| NM_021402 | Ube2j2 | ubiquitin-conjugating enzyme E2, J2 homolog (yeast) |
| NM_009456 | Ube213 | ubiquitin-conjugating enzyme E2L 3 |
| NM_014233 | UBTF | upstream binding transcription factor, RNA polymerase I |
| NM_026390 | Ubxd2 | UBX domain containing 2 |
| NM_145441 | Ubxd4 | UBX domain containing 4 |
| XM_140801 | Upf2 | UPF2 regulator of nonsense transcripts homolog (yeast) |
| NM_009477 | Upp1 | uridine phosphorylase 1 |
| XM_497119 | UREB1 | upstream regulatory element binding protein 1 |
| NM_009462 | Usp10 | ubiquitin specific protease 10 |
| NM_024258 | Usp16 | ubiquitin specific protease 16 |
| NM_175482 | Usp28 | ubiquitin specific protease 28 |
| XM_485461 | Usp48 | ubiquitin specific protease 48 |
| NM_009481 | Usp9x | ubiquitin specific protease 9, X chromosome |
| NM_011690 | Vars2 | valyl-tRNA synthetase 2 |
| NM_009503 | Vcp | valosin containing protein |
| NM_011694 | Vdac1 | voltage-dependent anion channel 1 |
| NM_153423 | Wasf2 | WAS protein family, member 2 |
| NM_033561 | Wbscr1 | Williams-Beuren syndrome chromosome region 1 homolog (human) |
| NM_145125 | Wdr9 | WD repeat domain 9 |
| NM_017778 | WHSC1L1 | Wolf-Hirschhorn syndrome candidate 1-like 1 |
| NM_009517 | Wig1 | wild-type p53-induced gene 1 |
| NM_175394 | Wtap | Wilms' tumour 1-associating protein [Mus musculus] |
| NM_025830 | Wwp2 | WW domain containing E3 ubiquitin protein ligase 2 |
| NM_134014 | Xpo1 | exportin 1, CRM1 homolog (yeast) |
| NM_028012 | Xrcc4 | X-ray repair complementing defective repair in Chinese hamster cells 4 |
| NM_009534 | Yap1 | yes-associated protein 1 |
| NM_013771 | Yme1I1 | YME1-like 1 (S. cerevisiae) |
| NM_009536 | Ywhae | tyrosine 3-monooxygenase/tryptophan 5-monooxygenase activation protein, epsilon polypeptide |
| NM_009537 | Yy1 | YY1 transcription factor |
| NM_009551 | Za20d2 | zinc finger, A20 domain containing 2 |
| NM_010731 | Zbtb7 | zinc finger and BTB domain containing 7 |
| NM_172569 | Zc3hdc5 | zinc finger CCCH type domain containing 5 |
| NM_026479 | Zcchc10 | zinc finger, CCHC domain containing 10 |
| XM_489605 | Zcwcc3 | zinc finger, CW-type with coiled-coil domain 3 |
| NM_009540 | Zfa | zinc finger protein, autosomal |
| NM_008717 | Zfml | zinc finger, matrin-like |
| NM_011742 | Zfp1 | zinc finger protein 1 |
| NM_27248 | Zfp219 | Zinc finger protein 219 |
| NM_027248 | Zfp219 | zinc finger protein 219 |
| XM_355521 | Zfp262 | zinc finger protein 262 |
| NM_022409 | Zfp296 | zinc finger protein 296 |
| NM_027947 | Zfp297b | zinc finger protein 297B |
| NM_030743 | Zfp313 | zinc finger protein 313 |
| NM_009556 | Zfp42 | zinc finger protein 42 |
| NM_146253 | Zfp482 | zinc finger protein 482 |
| NM_207255 | Zfp532 | zinc finger protein 532 |
| NM_009559 | Zfp57 | zinc finger protein 57 |
| NM_009560 | Zfp60 | zinc finger protein 60 |
| XM_484778 | Zfp91 | zinc finger protein 91 |
| NM_011757 | Zipro1 | zinc finger proliferation 1 |
| NM_003442 | ZNF143 | zinc finger protein 143 (clone pHZ-1) |
| XM_375065 | ZNF409 | zinc finger protein 409 |
| NM_018181 | ZNF532 | zinc finger protein 532 |
| NM_028028 | Zswim1 | zinc finger, SWIM domain containing 1 |
| NM_198416 | Zzz3 | zinc finger, ZZ domain containing 3 |
| NM_021446 | 0610007P14Rik | RIKEN cDNA 0610007P14 gene |
| NM_025645 | 0610009C03Rik | RIKEN cDNA 0610009C03 gene |
| NM_026681 | 0610010D24Rik | RIKEN cDNA 0610010D24 gene |
| NM_153194 | 1110034°07Rik | RIKEN cDNA 1110034°07 gene |
| XM_358504 | 1110038B12Rik | RIKEN cDNA 1110038B12 gene |
| XM_485388 | 1110054°05Rik | RIKEN cDNA 1110054°05 gene |
| NM_026170 | 1200007D18Rik | RIKEN cDNA 1200007D18 gene |
| NM_028760 | 1200008°12Rik | RIKEN cDNA 1200008°12 gene |
| NM_025814 | 1200009K13Rik | RIKEN cDNA 1200009K13 gene |
| NM_026182 | 1300002C08Rik | RIKEN cDNA 1300002C08 gene |
| NM_173366 | 1500010G04Rik | RIKEN cDNA 1500010G04 gene |
| NM_026411 | 1700021 F05Rik | RIKEN cDNA 1700021 F05 gene |
| NM_024260 | 1700034M03Rik | RIKEN cDNA 1700034M03 gene |
| NM_028487 | 1700034P14Rik | RIKEN cDNA 1700034P14 gene |
| XM_207074 | 1810006K21Rik | RIKEN cDNA 1810006K21 gene |
| XM_148990 | 1810043M20Rik | RIKEN cDNA 1810043M20 gene |
| NM_027360 | 2010107E04Rik | RIKEN cDNA 2010107E04 gene |
| XM_127387 | 2010111I01Rik | RIKEN cDNA 2010111I01 gene |
| NM_028218 | 2210409E12Rik | RIKEN cDNA 2210409E12 gene |
| NM_029813 | 2210418°10Rik | RIKEN cDNA 2210418°10 gene |
| NM_145563 | 2310001H12Rik | RIKEN cDNA 2310001H12 gene |
| NM_175107 | 2310022A10Rik | RIKEN cDNA 2310022A10 gene |
| NM_133714 | 2310037124Rik | RIKEN cDNA 2310037124 gene |
| NM_025531 | 2310042G06Rik | RIKEN cDNA 2310042G06 gene |
| NM_175108 | 2310047C17Rik | RIKEN cDNA 2310047C17 gene |
| NM_026421 | 2310057D15Rik | RIKEN cDNA 2310057D15 gene |
| NM_026844 | 2310061C15Rik | RIKEN cDNA 2310061C15 gene |
| NM_025475 | 2410007P03Rik | RIKEN cDNA 2410007P03 gene |
| NM_023203 | 2410015N17Rik | RIKEN cDNA 2410015N17 gene |
| NM_026643 | 2410017P07Rik | RIKEN cDNA 2410017P07 gene |
| NM_028362 | 2410018L13Rik | RIKEN cDNA 2410018L13 gene |
| NM_024254 | 2410042D21 Rik | RIKEN cDNA 2410042D21 gene |
| NM_028603 | 2410081M15Rik | RIKEN cDNA 2410081M15 gene |
| XM_133019 | 2410127E18Rik | RIKEN cDNA 2410127E18 gene |
| NM_026120 | 2410127L17Rik | RIKEN cDNA 2410127L17 gene |
| NM_029747 | 2410137M14Rik | RIKEN cDNA 2410137M14 gene |
| NM_030241 | 2410195B05Rik | RIKEN cDNA 2410195B05 gene |
| NM_023215 | 2500003M10Rik | RIKEN cDNA 2500003M10 gene |
| XM_127013 | 2600001A11Rik | RIKEN cDNA 2600001A11 gene |
| XM_355888 | 2610021A01Rik | RIKEN cDNA 2610021A01 gene |
| XM_486234 | 2610021l23Rik | RIKEN cDNA 2610021123 gene |
| NM_146084 | 2610024E20Rik | RIKEN cDNA 2610024E20 gene |
| NM_175143 | 2610028H07Rik | RIKEN cDNA 2610028H07 gene |
| NM_026407 | 2610033C09Rik | RIKEN cDNA 2610033C09 gene |
| NM_026476 | 2610101N10Rik | RIKEN cDNA 2610101N10 gene |
| NM_026009 | 2610204L23Rik | RIKEN cDNA 2610204L23 gene |
| NM_028151 | 2610528A15Rik | RIKEN cDNA 2610528A15 gene |
| XM_132261 | 2610528A17Rik | RIKEN cDNA 2610528A17 gene |
| NM_026531 | 2700083B06Rik | RIKEN cDNA 2700083B06 gene |
| NM_026029 | 2700085E05Rik | RIKEN cDNA 2700085E05 gene |
| XM_488640 | 2810011 L15Rik | RIKEN cDNA 2810011 L15 gene |
| NM_026197 | 2810013M15Rik | RIKEN cDNA 2810013M15 gene |
| NM_029766 | 2810047L02Rik | RIKEN cDNA 2810047L02 gene |
| NM_028330 | 2810051 F02Rik | RIKEN cDNA 2810051 F02 gene |
| XM_284425 | 2810422J05Rik | RIKEN cDNA 2810422J05 gene |
| XM_132966 | 2810474°19Rik | RIKEN cDNA 2810474°19 gene |
| NM_028385 | 2900045N06Rik | RIKEN cDNA 2900045N06 gene |
| NM_026064 | 2900073G15Rik | RIKEN cDNA 2900073G15 gene |
| NM_026615 | 2900073H19Rik | RIKEN cDNA 2900073H19 gene |
| NM_175404 | 3010003L21 Rik | RIKEN cDNA 3010003L21 gene |
| XM_134514 | 3010027A04Rik | RIKEN cDNA 3010027A04 gene |
| NM_026521 | 3110006P09Rik | RIKEN cDNA 3110006P09 gene |
| XM_125867 | 3830408P06Rik | RIKEN cDNA 3830408P06 gene |
| XM_196130 | 4432411E13Rik | RIKEN cDNA 4432411E13 gene |
| XM_282969 | 4631424J17Rik | RIKEN cDNA 4631424J17 gene |
| NM_027453 | 4632412E09Rik | RIKEN cDNA 4632412E09 gene |
| XM_130287 | 4930432B04Rik | RIKEN cDNA 4930432B04 gene |
| NM_026289 | 4930465K10Rik | RIKEN cDNA 4930465K10 gene |
| NM_028127 | 4930488L10Rik | RIKEN cDNA 4930488L10 gene |
| NM_026594 | 4930517K11 Rik | RIKEN cDNA 4930517K11 gene |
| NM_029186 | 4930538D17Rik | RIKEN cDNA 4930538D17 gene |
| NM_026296 | 4930548H24Rik | RIKEN cDNA 4930548H24 gene |
| NM_025739 | 4931406120Rik | RIKEN cDNA 4931406120 gene |
| NM_030074 | 4931408L03Rik | RIKEN cDNA 4931408L03 gene |
| NM_178935 | 4932441K18 | CXORF15 |
| NM_178682 | 4933426M11Rik | RIKEN cDNA 4933426M11 gene |
| NM_183200 | 5330438D12Rik | RIKEN cDNA 5330438D12 gene |
| NM_029868 | 5330440M15Rik | RIKEN cDNA 5330440M15 gene |
| NM_172935 | 5730457F11Rik | RIKEN cDNA 5730457F11 gene |
| NM_197940 | 5730509C05Rik | RIKEN cDNA 5730509C05 gene |
| NM_172661 | 5830434P21Rik | RIKEN cDNA 5830434P21 gene |
| NM_172765 | 6030404E16Rik | RIKEN cDNA 6030404E16 gene |
| XM_486150 | 6030411 K04Rik | RIKEN cDNA 6030411 K04 gene |
| XM_133159 | 6230401°10Rik | RIKEN cDNA 6230401°10 gene |
| NM_029532 | 6330548G22Rik | RIKEN cDNA 6330548G22 gene |
| XM_133187 | 6820402°20Rik | RIKEN cDNA 6820402°20 gene |
| XM_144310 | 6820424L24Rik | RIKEN cDNA 6820424L24 gene |
| NM_172501 | 8030451 K01 Rik | RIKEN cDNA 8030451K01 gene |
| NM_175294 | 8430423A01Rik | RIKEN cDNA 8430423A01 gene |
| NM_194351 | 9330175B10Rik | RIKEN cDNA 9330175B10 gene |
| NM_175414 | 9430079M16Rik | RIKEN cDNA 9430079M16 gene |
| NM_181401 | 9630015D15Rik | RIKEN cDNA 9630015D15 gene |
| NM_172380 | 9630046K23Rik | RIKEN cDNA 9630046K23 gene |
| NM_146186 | 2310038K02Rik | RIKEN cDNA 2310038K02 gene |
| NM_175433 | 5430400N05Rik | RIKEN cDNA 5430400N05 gene |
| NM_177136 | 9030227G01Rik | RIKEN cDNA 9030227G01 gene |
| XM_126551 | 9930033H14Rik | RIKEN cDNA 9930033H14 gene |
| NM_183028 | A030012M09Rik | RIKEN cDNA A030012M09 gene |
| NM_175004 | A230072116Rik | RIKEN cDNA A230072116 gene |
| NM_212484 | A230103N10Rik | RIKEN cDNA A230103N10 gene |
| XM_138091 | C130039O16Rik | RIKEN cDNA C130039016 gene |
| NM_022554 | Pdlim5 | PDZ and LIM domain 5 |
| XM_356366 | A830080D01 Rik | RIKEN cDNA A830080D01 gene |
| XM_133935 | AA673488 | expressed sequence AA673488 |
| AB024497 | Rest | Mus musculus NRSF/REST gene for neural-restrictive silencer factor, exon 1a, exon 1b, exon 1 c, 5'UTR. |
| BC048391 | | Mus musculus mRNA similar to thyroid hormone receptor-associated protein, 150 kDa subunit (cDNA clone MGC:56927 IMAGE:6314114), complete cds. |
| AK173249 | | Mus musculus mRNA for mKIAA1745 protein. |
| AK129336 | | Mus musculus mRNA for mKIAA1341 protein. |
| AK129266 | | Mus musculus mRNA for mKIAA1020 protein. |
| AK122371 | | Mus musculus mRNA for mKIAA0799 protein. |
| AK172968 | | Mus musculus mRNA for mKIAA0545 protein. |
| AK129140 | | Mus musculus mRNA for mKIAA0433 protein. |
| AK129117 | | Mus musculus mRNA for mKIAA0333 protein. |
| AK129037 | | Mus musculus mRNA for mKlAA0019 protein. |
| AB050541 | | Mus musculus mPcl2 mRNA for polycomblike 2, partial cds. |
| BC079594 | 1700081 L11 Rik | Mus musculus cDNA clone MGC:90742 IMAGE:6827379, |
| | | complete cds. |
| BC049128 | | Mus musculus cDNA clone MGC:61256 IMAGE:6822178, complete cds. |
| BC052850 | | Mus musculus cDNA clone MGC:60532 IMAGE:30057964, complete cds. |
| BC057163 | | Mus musculus cDNA clone IMAGE:5351131, partial cds. |
| BC033443 | | Mus musculus cDNA clone IMAGE:4036366, partial cds. |
| AL732594 | | Mouse DNA sequence from clone RP24-189G18 on chromosome 4 Contains the gene for the otholog of human PRP4 pre-mRNA processing factor 4 homolog (yeast) PRPF4, three novel genes, the Bspry gene for B-box and SPRY domain containing protein, the Alad gene fo |
| AL732548 | | Mouse DNA sequence from clone RP24-145P21 on chromosome 4 Contains a novel gene, the Slc31a1 gene for solute carrier family 31 member 1, the 5' end of a novel gene and a CpG island, complete sequence. |
| AL137783 | | Human DNA sequence from clone RP5-1181 K21 on chromosome 6 Contains the RPS12 gene encoding the ribosomal protein S12, a High mobility group protein-1 pseudogene, a CpG island, ESTs, STSs and GSSs, complete sequence. |
| BC040987 | | Homo sapiens cDNA clone IMAGE:4813640, partial cds. |
| X73096 | HNRNPA1 | H.sapiens hnRNP A1 gene promoter region. |
| M81871 | RBP-Jkappa | Mus musculus immunoglobulin germline IgK chain recombination binding protein (RBP-J kappa) pseudogene, exons 2-11. |
| BC027311 | 2310007F21 Rik | Mus musculus RIKEN cDNA 2310007F21 gene, mRNA (cDNA clone MGC:28095 IMAGE:3964905), complete cds. |
| BC016099 | 2410002F23Rik | Mus musculus RIKEN cDNA 2410002F23 gene, mRNA (cDNA clone MGC:27669 IMAGE:4910895), complete cds. |
| BC032970 | 2810026P18Rik | Mus musculus RIKEN cDNA 2810026P18 gene, mRNA (cDNA clone MGC:41526 IMAGE:1224948), complete cds. |
| AJ288898 | Als2cr3 | Rattus norvegicus mRNA for GABA-A receptor interacting factor-1 (GRIF-1 gene), splice variants. |
| BC012488 | Arhgef1 | Mus musculus Rho guanine nucleotide exchange factor (GEF) 1, mRNA (cDNA clone MGC:11487 IMAGE:3154558), complete cds. |
| AY255781 | Bat1b | Mus musculus strain C57BL/10 HLA-B associated transcript 1 (Bat1 b) gene, promoter and 5' UTR. |
| BC009202 | C14orf43 | Homo sapiens chromosome 14 open reading frame 43, mRNA (cDNA clone IMAGE:3614143), partial cds. |
| AF033620 | Cd151 | Mus musculus platelet endothelial tetraspan antigen-3 (Peta3) gene, complete cds. |
| BC057645 | Chc1 | Mus musculus chromosome condensation 1, mRNA (cDNA clone MGC:67907 IMAGE:3591859), complete cds. |
| AJ276962 | Clasp1 | Mus musculus partial mRNA for CLIP-associating protein CLASP1. |
| BY098269 | Cox6c | V-src suppressed transcript 3 |
| BC052713 | Cyfip1 | Mus musculus cytoplasmic FMR1 interacting protein 1, mRNA (cDNA clone MGC:64669 IMAGE:6835403), complete cds. |
| AF307845 | D15Ertd366e | Mus musculus epithelial protein lost in neoplasm-b (Eplin) mRNA, complete cds. |
| BC023768 | Epb4.1I2 | Mus musculus erythrocyte protein band 4.1-like 2, mRNA (cDNA clone IMAGE:5343611), partial cds. |
| BC049781 | Fzd7 | Mus musculus, frizzled homolog 7 (Drosophila), clone IMAGE:6334607, mRNA, partial cds. |
| BC021156 | G3bp | Mus musculus Ras-GTPase-activating protein SH3-domain binding protein, mRNA (cDNA clone MGC:13925 |
| | | IMAGE:4020362), complete cds. |
| BC012639 | Gsta4 | Mus musculus glutathione S-transferase, alpha 4, mRNA (cDNA clone MGC:13725 IMAGE:3995378), complete cds. |
| BC065124 | Hic2 | Mus musculus hypermethylated in cancer 2, mRNA (cDNA clone MGC:85994 IMAGE:30537019), complete cds. |
| AJ011802 | HSA011802 | Homo sapiens OZF gene exon 1. |
| X54053 | MMKFGFS | Mouse k-FGF oncogene 5' sequence. |
| BC061811 | Nap1I1 | Rattus norvegicus cDNA clone MGC:72278 IMAGE:5598632, complete cds. |
| BC046478 | | Mus musculus, clone IMAGE:5324476, mRNA. |
| BC061232 | Ogdh | Mus musculus oxoglutarate dehydrogenase (lipoamide), mRNA (cDNA clone IMAGE:6535602), complete cds. |
| AB086633 | Papola | Mus musculus gene for polyA polymerase, exon 1. |
| BC031202 | Plxnb2 | Mus musculus plexin B2, mRNA (cDNA clone MGC:37720 IMAGE:5066347), complete cds. |
| BC055788 | Prkwnk1 | Mus musculus protein kinase, lysine deficient 1, mRNA (cDNA clone IMAGE:6407142), partial cds. |
| D14441 | RATNAP22 | Rattus norvegicus NAP-22 mRNA for acidic membrane protein of rat brain, complete cds. |
| BC011441 | Rbmxrt | Mus musculus RNA binding motif protein, X chromosome retrogene, mRNA (cDNA clone MGC:6954 IMAGE:3153831), complete cds. |
| AJ006837 | Rnu17d | Mus musculus RNA transcript from U17 small nucleolar RNA host gene. |
| AF218255 | Slc29a1 | Mus musculus equilibrative nucleoside transporter 1 gene, complete cds, alternatively spliced. |
| AF510653 | Tcfe3 | Mus musculus transcription factor E3 (Tcfe3) mRNA, partial cds. |
| BC076618 | Tmem23 | Mus musculus RIKEN cDNA 9530058011 gene, mRNA (cDNA clone IMAGE:30635490), partial cds. |
| BC015289 | Vasp | Mus musculus vasodilator-stimulated phosphoprotein, mRNA (cDNA clone MGC:18907 IMAGE:4240907), complete cds. |
| BC019463 | Wdr33 | Mus musculus WD repeat domain 33, mRNA (cDNA clone IMAGE:4035918), complete cds. |
| BC023704 | Wdr42a | Mus musculus DNA segment, Chr 1, University of California at Los Angeles 4, mRNA (cDNA clone MGC:38390 IMAGE:5345701), complete cds. |
| BC066035 | Zranb3 | Mus musculus RIKEN cDNA 4933425L19 gene, mRNA (cDNA clone MGC:91303 IMAGE:6837116), complete cds. |
| BY174699 | | Similar to Flt3 interacting zinc finger protein 1 |
| CD546468 | B230112C05Rik | RIKEN cDNA B230112C05 gene |
| BY196730 | | Gene model 1650, (NCBI) |
| BY752712 | | Transcribed locus |

| B) with FlipROSACeo | | |
|---|---|---|
| **Acc_No** | **Symbol** | **Gene Name** |
| NM_011075 | Abcb1b | ATP-binding cassette, sub-family B (MDR/TAP), member 1B |
| NM_080633 | Aco2 | aconitase 2, mitochondrial |
| NM_009616 | Adam19 | a disintegrin and metalloproteinase domain 19 (meltrin beta) |
| NM_007414 | Adprh | ADP-ribosylarginine hydrolase |
| NM_054070 | Afg3l1 | AFG3(ATPase family gene 3)-like 1 (yeast) |
| NM_009642 | Agtrap | angiotensin II, type I receptor-associated protein |
| NM_198626 | A1480653 | expressed sequence A1480653 |
| NM_178760 | A1790205 | expressed sequence A1790205 |
| NM_177869 | AI847670 | expressed sequence A1847670 |
| NM_009656 | Aldh2 | aldehyde dehydrogenase 2, mitochondrial |
| NM_178784 | Alg6 | asparagine-linked glycosylation 6 homolog (yeast, alpha-1,3,-glucosyltransferase) |
| NM_007469 | Apoc1 | apolipoprotein C-1 |
| NM_019734 | Asah1 | N-acylsphingosine amidohydrolase 1 |
| NM_009721 | Atp1b1 | ATPase, Na+/K+transporting, beta 1 polypeptide |
| NM_009722 | Atp2a2 | ATPase, Ca++ transporting, cardiac muscle, slow twitch 2 |
| NM_007505 | Atp5a1 | ATP synthase, H+ transporting, mitochondrial F1 complex, alpha subunit, isoform 1 |
| NM_016774 | Atp5b | ATP synthase, H+ transporting mitochondrial F1 complex, beta subunit |
| NM_009725 | Atp5f1 | ATP synthase, H+ transporting, mitochondrial F0 complex, subunit b, isoform 1 |
| NM_175015 | Atp5g3 | ATP synthase, H+ transporting, mitochondrial F0 complex, subunit c (subunit 9), isoform 3 |
| NM_027862 | Atp5h | ATP synthase, H+ transporting, mitochondrial F0 complex, subunit d |
| NM_138597 | Atp5o | ATP synthase, H+ transporting, mitochondrial F1 complex, O subunit |
| NM_027439 | Atp6ap2 | ATPase, H+ transporting, lysosomal accessory protein 2 |
| NM_178772 | B230106l24Rik | RIKEN cDNA B230106l24 gene |
| NM_019693 | Bat1a | HLA-B-associated transcript 1A |
| NM_009737 | Bcat2 | branched chain aminotransferase 2, mitochondrial |
| NM_009761 | Bnip3l | BCL2/adenovirus E1B 19kDa-interacting protein 3-like |
| NM_007591 | Calr | calreticulin |
| NM_007597 | Canx | calnexin |
| NM_009838 | Cct6a | chaperonin subunit 6a (zeta) |
| NM_007645 | Cd37 | CD37 antigen |
| NM_007657 | Cd9 | CD9 antigen |
| NM_009864 | Cdh1 | cadherin 1 |
| NM_025876 | Cdk5rap1 | CDK5 regulatory subunit associated protein 1 |
| NM_024536 | CHPF | chondroitin polymerizing factor |
| XM_132045 | Chrna9 | cholinergic receptor, nicotinic, alpha polypeptide 9 |
| XM_125808 | Ckap4 | cytoskeleton-associated protein 4 |
| NM_011929 | Clcn6 | chloride channel 6 |
| NM_019649 | Clptm1 | cleft lip and palate associated transmembrane protein 1 |
| NM_053071 | Cox6c | cytochrome c oxidase, subunit VIc |
| NM_007750 | Cox8a | cytochrome c oxidase, subunit VIIIa |
| NM_133930 | Creld1 | cysteine-rich with EGF-like domains 1 |
| NM_007791 | Csrp1 | cysteine and glycine-rich protein 1 |
| NM_181417 | Csrp2bp | cysteine and glycine-rich protein 2 binding protein |
| NM_009976 | Cst3 | cystatin C |
| NM_009984 | Ctsl | cathepsin L |
| NM_022325 | Ctsz | cathepsin Z |
| NM_178640 | D230016N13Rik | RIKEN cDNA D230016N13 gene |
| NM_028053 | D4Ertd89e | DNA segment, Chr 4, ERATO Doi 89, expressed |
| NM_175518 | D730040F13Rik | RIKEN cDNA D730040F13 gene |
| NM_172681 | D930015E06Rik | RIKEN cDNA D930015E06 gene |
| NM_025705 | Dcbld1 | discoidin, CUB and LCCL domain containing 1 |
| NM_007584 | Ddr1 | discoidin domain receptor family, member 1 |
| NM_007840 | Ddx5 | DEAD (Asp-Glu-Ala-Asp) box polypeptide 5 |
| NM_177310 | E430012M05Rik | RIKEN cDNA E430012M05 gene |
| XM_194337 | Egfl4 | EGF-like-domain, multiple 4 |
| NM_007915 | Ei24 | etoposide induced 2.4 mRNA |
| NM_026030 | Eif2s2 | eukaryotic translation initiation factor 2, subunit 2 (beta) |
| NM_013507 | Eif4g2 | eukaryotic translation initiation factor 4, gamma 2 |
| NM_007932 | Eng | endoglin |
| XM_125594 | Enpp3 | ectonucleotide pyrophosphatase/phosphodiesterase 3 |
| NM_019561 | Ensa | endosulfine alpha |
| NM_010139 | Epha2 | Eph receptor A2 |
| XM_125954 | Erbb3 | v-erb-b2 erythroblastic leukemia viral oncogene homolog 3 (avian) |
| NM_026129 | Erp29 | endoplasmic reticulum protein 29 |
| NM_007968 | Ewsr1 | Ewing sarcoma breakpoint region 1 |
| NM_010180 | Fbln1 | fibulin 1 |
| NM_007996 | Fdx1 | ferredoxin 1 |
| NM_008004 | Fgf17 | fibroblast growth factor 17 |
| NM_010202 | Fgf4 | fibroblast growth factor 4 |
| NM_012056 | Fkbp9 | FK506 binding protein 9 |
| NM_010233 | Fn1 | fibronectin 1 |
| NM_008034 | Folr1 | folate receptor 1 (adult) |
| NM_008047 | Fstl1 | follistatin-like 1 |
| NM_172308 | Fthfsdc1 | formyltetrahydrofolate synthetase domain containing 1 |
| NM 019439 | Gabbr1 | gamma-aminobutyric acid (GABA-B) receptor, 1 |
| NM_183358 | Gadd45gip1 | growth arrest and DNA-damage-inducible, gamma interacting protein 1 |
| NM_172451 | Galnt6 | UDP-N-acetyl-alpha-D-galactosamine:polypeptide N-acetylgalactosaminyltransferase 6 |
| NM_144731 | Galnt7 | UDP-N-acetyl-alpha-D-galactosamine: polypeptide N-acetylgalactosaminyltransferase 7 |
| NM_008105 | Gcnt2 | glucosaminyl (N-acetyl) transferase 2, 1-branching enzyme |
| NM_138591 | Gfm | G elongation factor |
| NM_009752 | Glb1 | galactosidase, beta 1 |
| NM_009149 | Glg1 | golgi apparatus protein 1 |
| NM_008133 | Glud1 | glutamate dehydrogenase 1 |
| NM_027307 | Golph2 | golgi phosphoprotein 2 |
| NM_021610 | Gpa33 | glycoprotein A33 (transmembrane) |
| NM_016739 | Gpiap1 | GPI-anchored membrane protein 1 |
| XM_355385 | Gpr48 | G protein-coupled receptor 48 |
| NM_145558 | Hadhb | hydroxyacyl-Coenzyme A dehydrogenase/3-ketoacyl-Coenzyme A thiolase/enoyl-Coenzyme A hydratase (trifunctional protein), beta subunit |
| NM_010422 | Hexb | hexosaminidase B |
| NM_013820 | Hk2 | hexokinase 2 |
| NM_015818 | Hs6st1 | heparan sulfate 6-O-sulfotransferase 1 |
| NM_022310 | Hspa5 | heat shock 70kD protein 5 (glucose-regulated protein) |
| NM_004134 | HSPA9B | heat shock 70kDa protein 9B (mortalin-2) |
| NM_010477 | Hspd1 | heat shock protein 1 (chaperonin) |
| NM_008303 | Hspe1 | heat shock protein 1 (chaperonin 10) |
| NM_029573 | Idh3a | isocitrate dehydrogenase 3 (NAD+) alpha |
| NM_130884 | Idh3b | isocitrate dehydrogenase 3 (NAD+) beta |
| NM_023065 | Ifi30 | interferon gamma inducible protein 30 |
| NM_030694 | Ifitm2 | interferon induced transmembrane protein 2 |
| NM_134437 | II17rd | interleukin 17 receptor D |
| NM_181517 | Ipo7 | importin 7 |
| NM_013565 | Itga3 | integrin alpha 3 |
| NM_010577 | Itga5 | integrin alpha 5 (fibronectin receptor alpha) |
| NM_008397 | Itga6 | integrin alpha 6 |
| NM_010580 | Itgb5 | integrin beta 5 |
| NM_013566 | Itgb7 | integrin beta 7 |
| NM_008408 | Itm1 | intergral membrane protein 1 |
| NM_000214 | JAG1 | jagged 1 (Alagille syndrome) |
| NM_206924 | Jtb | jumping translocation breakpoint |
| NM_021542 | Kcnk5 | potassium channel, subfamily K, member 5 |
| XM_203796 | Lama5 | laminin, alpha 5 |
| NM_008482 | Lamb1-1 | laminin B1 subunit 1 |
| NM_010683 | Lamc1 | laminin, gamma 1 |
| NM_010686 | Laptm5 | lysosomal-associated protein transmembrane 5 |
| NM_026058 | Lass4 | longevity assurance homolog 4 (S. cerevisiae) |
| NM_177099 | Lefty2 | Left-right determination factor 2 |
| NM_011175 | Lgmn | legumain |
| NM_013584 | Lifr | leukemia inhibitory factor receptor |
| NM_153404 | Liph | lipase, member H |
| NM_025828 | Lman2 | lectin, mannose-binding 2 |
| NM_172827 | Lnpep | leucyl/cystinyl aminopeptidase |
| XM_138959 | LOC239017 | similar to KIAA1290 protein |
| XM_488805 | LOC433082 | hypothetical gene supported by AK086736 |
| XM_485007 | LOC433433 | similar to adenylate kinase 4 |
| XM_485484 | LOC433788 | similar to high mobility group protein B2 |
| XM_489209 | LOC434251 | similar to C-terminal binding protein 2 |
| XM_194114 | Lrig2 | leucine-rich repeats and immunoglobulin-like domains 2 |
| NM_177152 | Lrig3 | leucine-rich repeats and immunoglobulin-like domains 3 |
| NM_008512 | Lrp1 | low density lipoprotein receptor-related protein 1 |
| NM_008513 | Lrp5 | low density lipoprotein receptor-related protein 5 |
| NM_013587 | Lrpap1 | low density lipoprotein receptor-related protein associated protein 1 |
| NM_028233 | Lrpprc | leucine-rich PPR-motif containing |
| NM_020486 | Lu | Lutheran blood group (Auberger b antigen included) |
| NM_010749 | M6pr | mannose-6-phosphate receptor, cation dependent |
| NM_007358 | M96 | likely ortholog of mouse metal response element binding transcription factor 2 |
| NM_027288 | Manba | mannosidase, beta A, lysosomal |
| XM_130628 | Manbal | mannosidase, beta A, lysosomal-like |
| NM_178266 | Mbtps2 | membrane-bound transcription factor protease, site 2 |
| NM_008566 | Mcm5 | minichromosome maintenance deficient 5, cell division cycle 46 (S. cerevisiae) |
| NM_023947 | MGC3234 | hypothetical protein MGC3234 |
| NM_021607 | MGI:1891700 | nicastrin |
| NM_019951 | MGI:1929464 | signal peptidase complex |
| NM_008602 | Miz1 | Msx-interacting-zinc finger |
| NM_029017 | Mrpl47 | mitochondrial ribosomal protein L47 |
| NM_008669 | Naga | N-acetyl galactosaminidase, alpha |
| NM_010886 | Ndufa4 | NADH dehydrogenase (ubiquinone) 1 alpha subcomplex, 4 |
| NM_025316 | Ndufb5 | NADH dehydrogenase (ubiquinone) 1 beta subcomplex, 5 |
| NM_144870 | Ndufs8 | NADH dehydrogenase (ubiquinone) Fe-S protein 8 |
| XM_486230 | Nedd4 | neural precursor cell expressed, developmentally down-regulted gene 4 |
| NM_010917 | Nid1 | nidogen 1 |
| NM_008695 | Nid2 | nidogen 2 |
| NM_019435 | Np15 | nuclear protein 15.6 |
| NM_018815 | Nup210 | nucleoporin 210 |
| NM_145706 | Nup43 | nucleoporin 43 |
| NM_010956 | Ogdh | oxoglutarate dehydrogenase (lipoamide) |
| NM_029565 | ORF18 | open reading frame 18 |
| NM_011030 | P4ha1 | procollagen-proline, 2-oxoglutarate 4-dioxygenase (proline 4-hydroxylase), alpha 1 polypeptide |
| NM_025823 | Pcyox1 | prenylcysteine oxidase 1 |
| NM_008808 | Pdgfa | platelet derived growth factor, alpha |
| NM_008810 | Pdha1 | pyruvate dehydrogenase E1 alpha 1 |
| XM_111232 | Pfas | phosphoribosylformylglycinamidine synthase (FGAR amidotransferase) |
| NM_025801 | Pgd | phosphogluconate dehydrogenase |
| NM_023196 | Pla2g12a | phospholipase A2, group XIIA |
| NM_019755 | Plp2 | proteolipid protein 2 |
| NM_011125 | Pltp | phospholipid transfer protein |
| NM_028199 | Plxdc1 | plexin domain containing 1 |
| NM_008881 | Plxna1 | plexin A1 |
| XM_484491 | Plxnb2 | plexin B2 |
| NM_173180 | Pmpca | peptidase (mitochondrial processing) alpha |
| NM_027869 | Pnpt1 | polyribonucleotide nucleotidyltransferase 1 |
| NM_011149 | Ppib | peptidylprolyl isomerase B |
| NM_145150 | Prc1 | protein regulator of cytokinesis 1 |
| NM_033573 | Prcc | papillary renal cell carcinoma (translocation-associated) |
| NM_016764 | Prdx4 | peroxiredoxin 4 |
| NM_011213 | Ptprf | protein tyrosine phosphatase, receptor type, F |
| NM_008983 | Ptprk | protein tyrosine phosphatase, receptor type, K |
| NM_145925 | Pttg1ip | pituitary tumor-transforming 1 interacting protein |
| NM_019869 | Rbm14 | RNA binding motif protein 14 |
| NM_133933 | Rpn1 | ribophorin |
| NM_009086 | Rpo1-2 | RNA polymerase 1-2 |
| NM_019743 | Rybp | RING1 and YY1 binding protein |
| NM_016741 | Scarb1 | scavenger receptor class B, member 1 |
| NM_007644 | Scarb2 | scavenger receptor class B, member 2 |
| NM_029023 | Scpep1 | serine carboxypeptidase 1 |
| NM_011521 | Sdc4 | syndecan 4 |
| NM_009145 | Sdfr1 | stromal cell derived factor receptor 1 |
| NM_025321 | Sdhc | succinate dehydrogenase complex, subunit C, integral membrane protein |
| NM_013659 | Sema4b | sema domain, immunoglobulin domain (Ig), transmembrane domain (TM) and short cytoplasmic domain, (semaphorin) 4B |
| NM_013663 | Sfrs3 | splicing factor, arginine/serine-rich 3 (SRp20) |
| NM_133221 | Slc24a6 | solute carrier family 24 (sodium/potassium/calcium exchanger), member 6 |
| NM_011400 | Slc2a1 | solute carrier family 2 (facilitated glucose transporter), member 1 |
| NM_011401 | Slc2a3 | solute carrier family 2 (facilitated glucose transporter), member 3 |
| NM_153062 | Slc37a1 | solute carrier family 37 (glycerol-3-phosphate transporter), member 1 |
| NM_028123 | Slc37a3 | solute carrier family 37 (glycerol-3-phosphate transporter), member 3 |
| NM_175121 | Slc38a2 | solute carrier family 38, member 2 |
| NM_144808 | Slc39a14 | solute carrier family 39 (zinc transporter), member 14 |
| NM_028064 | Slc39a4 | solute carrier family 39 (zinc transporter), member 4 |
| NM_148929 | Slc9a8 | solute carrier family 9 (sodium/hydrogen exchanger), member 8 |
| XM_132597 | Smarcad1 | SWI/SNF-related, matrix-associated actin-dependent regulator of chromatin, subfamily a, containing DEAD/H box 1' |
| NM_133888 | Smpdl3b | sphingomyelin phosphodiesterase, acid-like 3B |
| NM_029949 | Snapc3 | small nuclear RNA activating complex, polypeptide 3 |
| NM_011436 | Sorl1 | sortilin-related receptor, LDLR class A repeats-containing |
| NM_013672 | Sp1 | trans-acting transcription factor 1 |
| NM_009242 | Sparc | secreted acidic cysteine rich glycoprotein |
| NM_145502 | Spfh1 | SPFH domain family, member 1 |
| NM_009263 | Spp1 | secreted phosphoprotein 1 |
| NM_025448 | Ssr2 | signal sequence receptor, beta |
| NM_016737 | Stip1 | stress-induced phosphoprotein 1 |
| NM_172294 | Sulf1 | sulfatase 1 |
| NM_006372 | SYNCRIP | synaptotagmin binding, cytoplasmic RNA interacting protein |
| NM_134011 | Tbrg4 | transforming growth factor beta regulated gene 4 |
| NM_011562 | Tdgf1 | teratocarcinoma-derived growth factor |
| NM_011638 | Tfrc | transferrin receptor |
| NM_146153 | Thrap3 | thyroid hormone receptor associated protein 3 |
| NM_009388 | Tkt | transketolase |
| NM_145928 | Tm4sf14 | transmembrane 4 superfamily member 14 |
| NM_080556 | Tm9sf2 | transmembrane 9 superfamily member 2 |
| NM_020275 | Tnfrsf10b | tumor necrosis factor receptor superfamily, member 10b |
| NM_013869 | Tnfrsf19 | tumor necrosis factor receptor superfamily, member 19 |
| NM_172609 | Tomm22 | translocase of outer mitochondrial membrane 22 homolog (yeast) |
| NM_011623 | Top2a | topoisomerase (DNA) II alpha |
| NM_023141 | Tor3a | torsin family 3, member A |
| NM_009429 | Tpt1 | tumor protein, translationally-controlled 1 |
| NM_172745 | Tufm | Tu translation elongation factor, mitochondrial |
| NM_030254 | Tusc3 | tumor suppressor candidate 3 |
| NM_145367 | Txndc5 | thioredoxin domain containing 5 |
| XM_126809 | Txndc7 | thioredoxin domain containing 7 |
| NM_019392 | Tyro3 | TYR03 protein tyrosine kinase 3 |
| NM_019748 | Uble1a | ubiquitin-like 1 (sentrin) activating enzyme E1A |
| NM_198899 | Ugcgl1 | UDP-glucose ceramide glucosyltransferase-like 1 |
| NM_025407 | Uqcrc1 | ubiquinol-cytochrome c reductase core protein 1 |
| NM_009462 | Usp10 | ubiquitin specific protease 10 |
| NM_175482 | Usp28 | ubiquitin specific protease 28 |
| NM_009505 | Vegfa | vascular endothelial growth factor A |
| NM_027121 | Vkorc111 | vitamin K epoxide reductase complex, subunit 1-like 1 |
| NM_019780 | Vps29 | vacuolar protein sorting 29 (S. pombe) |
| NM_028866 | Wdr33 | WD repeat domain 33 |
| NM_011738 | Ywhah | tyrosine 3-monooxygenase/tryptophan 5-monooxygenase activation protein, eta polypeptide |
| NM_009556 | Zfp42 | zinc finger protein 42 |
| NM_018872 | D1Bwg0491e | DNA segment, Chr 1, Brigham & Women's Genetics 0491 expressed [*Mus musculus*] |
| XM_127445 | XM_127445 | PREDICTED: Mus musculus succinate dehydrogenase complex, subunit A, flavoprotein (Fp) (Sdha), mRNA. |
| XM_358805 | XM_358805 | PREDICTED: Mus musculus integrin beta 5 (Itgb5), mRNA. |
| XM_358820 | XM_358821 | PREDICTED: Mus musculus expressed sequence AI480653 (AI480653), |
| XM_485954 | XM_485955 | PREDICTED: Mus musculus similar to solute carrier family 28, (sodium-coupled nucleoside transporter), member 1; concentrative nucleoside transporter 1 (LOC434203), mRNA. |
| NM_020003 | 0610031J06Rik | RIKEN cDNA 0610031J06 gene |
| NM_025334 | 0610040B21Rik | RIKEN cDNA 0610040B21 gene |
| NM_026775 | 1110014C03Rik | RIKEN cDNA 1110014C03 gene |
| NM_144525 | 1110039B18Rik | RIKEN cDNA 1110039B18 gene |
| NM_025814 | 1200009K13Rik | RIKEN cDNA 1200009K13 gene |
| NM_023625 | 1300012G16Rik | RIKEN cDNA 1300012G16 gene |
| NM_026184 | 1300013B24Rik | RIKEN cDNA 1300013B24 gene |
| NM_025464 | 1810021J13Rik | RIKEN cDNA 1810021J13 gene |
| NM_025509 | 2310008M10Rik | RIKEN cDNA 2310008M10 gene |
| NM_197991 | 2310044H10Rik | RIKEN cDNA 2310044H10 gene |
| NM_026211 | 2400003B06Rik | RIKEN cDNA 2400003B06 gene |
| NM_028243 | 2510048K03Rik | RIKEN cDNA 2510048K03 gene |
| NM_025952 | 2610529C04Rik | RIKEN cDNA 2610529C04 gene |
| NM_026528 | 2700060E02Rik | RIKEN cDNA 2700060E02 gene |
| NM_026511 | 281 0002N01 Rik | RIKEN cDNA 2810002N01 gene |
| XM_283848 | 2810407C02Rik | RIKEN cDNA 2810407C02 gene |
| NM_134009 | 3100002P13Rik | RIKEN cDNA 3100002P13 gene |
| NM_026522 | 3110023E09Rik | RIKEN cDNA 3110023E09 gene |
| XM_128959 | 3930401E15Rik | RIKEN cDNA 3930401E15 gene |
| NM_175675 | 4930471 M23Rik | RIKEN cDNA 4930471 M23 gene |
| NM_029720 | 5730592L21 Rik | RIKEN cDNA 5730592L21 gene |
| NM_024465 | 6330583M11Rik | RIKEN cDNA 6330583M11 gene |
| NM_172501 | 8030451 K01 Rik | RIKEN cDNA 8030451 K01 gene |
| NM_172380 | 9630046K23Rik | RIKEN cDNA 9630046K23 gene |
| XM_356366 | A830080D01 Rik | RIKEN cDNA A830080D01 gene |
| NM_173734 | A930025J12Rik | RIKEN cDNA A930025J12 gene |
| NM_198884 | AB114826 | cDNA sequence AB114826 |
| AF005656 | | Gamma proteobacterium MS-1 16S ribosomal RNA gene, complete sequence. |
| BC038652 | 1810014B01Rik | Mus musculus RIKEN cDNA 1810014B01 gene, mRNA (cDNA clone IMAGE:1529193), partial cds. |
| BC047208 | 1500015A07Rik | Mus musculus RIKEN cDNA 1500015A07 gene, mRNA (cDNA clone IMAGE:6310866), partial cds. |
| BC059024 | Glt28d1 | Mus musculus cDNA clone IMAGE:6810589, partial cds. |

Considering that these gene trap lines were isolated in less than a year, conditional gene trapping seems significantly more efficient than conditional gene targeting. However, analysis of the existing gene trap resources indicates that gene trapping is more efficient than gene targeting only up to about 50% of all mouse genes, after which the mutation rate falls to a level comparable to gene targeting (Skarnes, W.C. et al., Nat. Genet. 36, 543-4 (2004)). Moreover, effective gene trapping is restricted to the approximately 70% of the genes expressed in ES cells (Ramalho-Santos, M. et al., Science 298, 597-600 (2002); Ivanova, N.B. et al., Science 298, 601-4 (2002)). We believe that for a comprehensive mutagenesis of the mouse genome, a balance between gene trapping and gene targeting, performed with generic gene trap cassettes inserted into the targeting vectors, is likely to be the most efficient and cost-effective.

The principal elements of a conditional gene trap cassette of embodiments (1) and (2) of the invention that selects for integrations into expressed genes are (i) a conditional gene disruption segment, containing a 3' splice site (splice acceptor; SA) and a polyadenylation sequence (polyA) flanked by the RRSs of the two recombination systems, and (ii) a selection segment containing a reporter or selectable marker gene flanked by an upstream SA- and a downstream polyA- site. The selection segment is flanked by two RRSs in same orientation, which are recognized by a further recombinase and is in opposite orientation to the gene disruption cassette. Selection for gene expression with the gene trap cassette of embodiment (1) and (2) yields recombinants in which the reporter gene is fused to the regulatory elements of an endogenous gene. Transcripts generated by these fusions encode a truncated cellular protein which has lost its normal function.

Since selection for a gene trap event relies on the expression of the selection cassette, which is by itself mutagenic, it needs to be inverted to the antisense, noncoding strand in embodiment (1) or removed to recreate gene function in embodiment (2). This is achieved in (1) by expressing the first recombinase in recombinants selected for gene trap integrations. In a favoured operational process the conditional gene trap is transduced into ES cells. After selecting for integrations into the introns of expressed genes, the first recombinase is transiently expressed in individual clones to invert the gene trap cassette to the antisense, non-coding strand and thus restore gene function. The resulting clones containing the gene disruption cassette on the antisense, non-coding strand are used to create transgenic mouse strains. Such mouse strains are crossed to mouse strains expressing the second recombinase to obtain doubly transgenic offspring where the gene disruption cassette is re-inverted to its original mutagenic (sense) orientation on the coding strand. Alternatively, the removal of the selection cassette of embodiment (2) is achieved by the first recombinase as follows: the gene trap cassette is transduced into ES cells. After selecting for integrations into the introns of expressed genes, the first recombinase is transiently expressed in individual clones to delete the selection cassette and thus restore gene function. The resulting clones containing only the gene disruption cassette on the antisense, non-coding strand are used to create transgenic mouse strains. Such strains are crossed to mouse strains expressing the second recombinase to obtain doubly transgenic offspring, where the gene disruption cassette is inverted to to its mutagenic (sense) orientation on the coding strand.

As a further preferred embodiment the invention provides a conditional gene trap vector that selects for integrations into genes regardless of their expression. In other words, selection for integrations into all genes, expressed and non-expressed, are possible. This is achieved by adding to the original gene disruption cassette a second cassette in which a selection gene is fused to an upstream constitutive promoter and to a downstream 5' splice site (splice donor) (Zambrowicz et al., Nature, 392, 608 (1998)). Expression of this gene trap is dependent on the acquisition of an endogenous polyadenylation sequence, which occurs by splicing of the selection cassette to the downstream exons of the target gene. Since the process is driven by a constitutive promoter, selection for gene trap integrations is independent of the target gene expression. As with the other conditional gene trap, a favoured operational process is its transduction into ES cells and the generation of mutant mouse strains.

The introduction of the gene trap cassette in the processes of embodiments (4) and (5) of the invention into a suitable cells can be effected by conventional methods including electroporation or retroviral infection. "Suitable cells" refers to appropriate starting cells, including cells pretreated for the introduction.

In a preferred embodiment of the processes (4) and (5), the introduction of the gene trap cassette into the cell is done by homologous recombination. The gene trap cassette used in this embodiment is flanked by homology regions apt for homologous recombination, preferably by homology regions corresponding to a first intron of a target gene. This gene trap cassette modification is also a preferred aspect of embodiments (1) and (2). The cassette is introduced into the ES cell by homologous recombination. Thus, the cassette can be used to introduce conditional mutations into specific target genes.

In a preferred embodiment of the process (5) of the invention the induction of a mutation in the trapped gene by inversion of the functional DNA segment according to step (v) is effected by a site specific recombinase capable of unidirectional inversion of the functional DNA of the gene trapping construct. The process (5) is suitable for temporally and/or spatially restricted inactivation of all genes that constitute a living organism and for preparing transgenic non-human mammals, especially transgenic mice. In such process, the gene trap cassette as defined above is introduced into an ES cell.

The above process possesses the following advantages over current technology:
(i) mutations are inducible in prespecified cells and tissues and during prespecified time intervals;
(ii) mutations can be induced either randomly by gene trapping or directed by gene targeting;
(iii) mutations can be induced in all genes, including those for which cloned sequences are not available;
(iv) the functional analysis of the mutant genes in appropriate organisms is relatively fast and cheap.

The present invention is further illustrated by the following Examples which are, however, not to be construed as to limit the invention.

### Examples

### Materials and Methods

Plasmids: pFlipROSAβgeo (SEQ ID NO:1) was assembled in pBabeSrf, a modified pBabepuro retroviral vector lacking the promoter and enhancer elements from the 3'LTR (Gebauer, M. et al., Genome Res 11, 1871-7 (2001)). Pairs of the heterotypic frt/F3 and lox511/loxP recombinase target sequences (RTs) were cloned in the illustrated orientation (Figure 1A) into the unique BamHI and EcoRI sites of pBabeSrf yielding the intermediate plasmid pBLF. RTs were obtained by synthetic oligonucleotide annealing and extension overlap PCR. To enable efficient recombination, 86 bp and 46 bp spacers were inserted between frt/F3 and loxP/lox511 sites, respectively. To obtain pFlipRosaβgeo, a SApgeopA cassette derived from the gene trap vector ROSAβgeo (Friedrich, G. & Soriano, P. Genes Dev. 5, 1513, (1991)) was inserted into the SnaBI site of pBLF between the inversely oriented RT pairs. The final pFlipRosaβgeo vector was verified by sequencing. The pFlipRosaCeo (SEQ ID NO:3) vector was obtained from pFlipRosaβgeo by replacing the SApgeo cassette with the Ceo fusion gene derived from pU3Ceo. The final pFlipRosaCeo plasmid was verified by sequencing. Oligonucleotide and primer sequences used in the various cloning steps are available upon request.

The pCAGGS-FLPe expression plasmid was a gift from A. Francis Stewart (Rodriguez, C. I. et al., Nat Genet 25, 139, (2000)). The pCAGGS-Cre expression plasmid was derived from and pCAGGS-FLPe by replacing the FLPe cDNA with the Cre cDNA of pSG5Cre (Feil, R. et al., Biochem Biophys Res Commun 237, 752 (1997)).

The expression plasmids prFlipRosabgeo and prFlipRosaCeo (SEQ ID Nos:2 and 4, respectively) are based on the plasmids pFlipROSAβgeo and pFlipRosaCeo, respectively, wherein the lox5ll sites have been replaced by lox5171 sites. In the following Examples plasmids with lox511 sites are utilized.

ES-cell cultures, infections and electroporations: The [C57BL/6J x 129S6/SvEvTac] F1 ES cell lines were grown on irradiated or Mitomycin C treated MEF feeder layers in the presence of 1000 U/ml of leukemia inhibitory factor (LIF) (Esgro®, Chemicon Intl., Hofheim, Germany) as previously described (Hansen, J. et al., Proc Natl Acad Sci U S A 100, 9918 (2003)).

Gene trap retrovirus was produced in Phoenix-Eco helper cells by using the transient transfection strategy described previously (Nolan, G. P. & Shatzman, A. R. Curr Opin Biotechnol 9, 447 (1998)). ES cells were infected with the virus containing supernatants at an M.O.I. < 0.5 as previously described (Hansen, J. et al., Proc Natl Acad Sci U S A 100, 9918 (2003)). Gene trap expressing ES-cell lines were selected in 130 µg/ml G418 (Invitrogen), manually picked, expanded, and stored frozen in liquid nitrogen.

Electroporations were carried out using 1 x 10⁷ ES cells, 10 µg of plasmid DNA and a 400 µF capacitator (BioRad, Hercules, USA) as previously described (Floss, T. & Wurst, W., Methods Mol Biol 185, 347 (2002)). After incubating for 2 days in medium supplemented with 0.6 µg/ml puromycin (Sigma-Aldrich, Munich, Germany), the cells were trypsinized and seeded at low density (1000 cells/dish) onto 60 mm Petri dishes. Emerging clones were manually picked after 9 days and expanded. The resulting cell lines were used for X-Gal stainings and molecular analyses.

Nucleic acids and protein analyses: PCRs were performed according to standard protocols using 300-500 ng of genomic DNA or 1 µg of reverse transcribed total RNA in a total volume of 50 µl. The primer sequences used are available upon request.

For Northern blotting, polyA⁺ RNA was purified from total RNA using the Oligotex mRNA-mini-kit (Qiagen, Hilden, Germany) according to the manufacturer's instructions. The mRNA (1-2 µg) was fractionated on 1% formaldehyde-agarose gels, blotted onto Hybond N+ (Amersham, Freiburg, Germany) nylon membranes, and hybridized to ³²P-labeled cDNA probes (Hartmann Analytic, Braunschweig, Germany) in ULTRAhyb hybridization solution (Ambion, Austin, TX, USA) according to manufacturer's instructions. The GIt28d1-cDNA probe was obtained by asymmetric RT-PCR (Buess, M. et al., Nucleic Acids Res 25, 2233 (1997)). using an anti-sense primer complementary to exon 10 of the Glt28d1 gene.

Semiautomated 5'RACE and sequencing was performed as previously described (Hansen, J. et al., Proc Natl Acad Sci U S A 100, 9918 (2003)). The sequences of the generic and vector-specific primers used are available upon request.

Western blots were performed as previously described (Sterner-Kock, A. et al.,Genes Dev 16, 2264-,(2002)), using anti-RbAp46, (Abcam, Cambridge, UK) and lamin A (Santa Cruz, Heidelberg, Germany) primary antibodies.

GTST analysis: GTSTs were analyzed as previously described (Hansen, J., et al., Proc Natl Acad Sci U S A 100, 9918, (2003) using the following databases: GenBank (rel. 144), UniGene (build 141), RefSeq (rel. 8) (all at http://www.ncbi.nlm.nih.gov), ENSEMBL v26.33 (http://www.ensembl.org), MGI (http://www.informatics.jax.org/) and GeneOntology (Dec. 2004 release) (http://www.geneontology.org).

### Example 1: Vector design

Two gene trap vectors were designed for large scale conditional mutagenesis in ES cells. The first vector FlipRosaβgeo contains a classic splice acceptor (SA) - β-galactosidase/neomycintransferase fusion gene (βgeo) - polyadenylation sequence (pA) cassette inserted into the backbone of a promoter- and enhancerless Moloney murine leukemia virus in inverse transcriptional orientation relative to the virus (Figure 1A) (Friedrich, G. & Soriano, P. (1991) Genes Dev. 5, 1513-1523). The second vector FlipRosaCeo is similar to FlipRosaβgeo except that SApgeo has been exchanged with Ceo, which is an in frame fusion between the human CD2 cell surface receptor- and the neomycin resistance genes (Gebauer, M. et al., Genome Res 11, 1871-7 (2001)). Unlike βgeo, Ceo does not require an extra splice acceptor site for trapping as it contains a powerful cryptic 5' splice site close to its 5' end. Moreover, Ceo encodes a type II transmembrane domain, which favors the capture of signal sequence and/or transmembrane encoding genes, i.e. secretory pathway genes (Figure 1A) (Gebauer, M. et al., Genome Res 11, 1871-7 (2001)). Previous studies involving the isolation of 3,620 ES cell lines with the retroviral gene trap vector -U3Ceo- indicated that Ceo captures secretory pathway genes with over 80% efficiency (GGTC resource/www.genetrap.de). This is in contrast to the classic βgeo vectors, of which only 19% insert into such genes (GGTC-resource, www.genetrap.de). Thus, classic and the secretory pathway gene trap vectors are complementary and therefore, we equipped both with a conditional mechanism. The mechanism relies on two site-specific recombination systems (FLPe/frt; Cre/loxP), which enable gene trap cassette inversions from the sense, coding strand of a trapped gene to the anti-sense, non-coding strand and back. As a result, the gene trap vectors allow (i) high throughput selection of gene trap lines using G418, (ii) inactivation of gene trap mutations prior to ES cell line conversion into mice by blastocyst injection, and (iii) reactivation of the mutations at prespecified times and in selected tissues of the resulting mice.

We used an adaptation of a recently published site-specific recombination strategy termed FlEx (flip-excision) (Schnutgen, F. et al., Nat Biotechnol 21, 562-5 (2003)). FlEx uses pairs of inversely oriented heterotypic recombinase target sequences (RTs) such as loxP and lox511 or frt and F3. When inserted upstream and downstream of a gene trap cassette, Cre or FLPe recombinases invert the cassette and place a homotypic RT pair near to each other in a direct orientation. Recombination between this pair of directly repeated RTs excises one of the other heterotypic RTs, thereby locking the recombination product against re-inversion to the original orientation. Thus, by flanking the gene trap cassettes of FlipRosaβgeo and FlipRosaCeo with pairs of heterotypic lox and frt sites (Figure 1A), a successive delivery of FLPe and Cre to a trapped ES cell line will induce two directional inversions, thereby first repairing and then re-inducing the gene trap mutation as exemplified for the SApgeopA gene trap cassette in Figure 1B.

### Example 2: Gene trap cassette inversions in ES cells

To test for recombinase-mediated inversions, several FlipRosaβgeo-trapped ES cell lines were selected for high levels of βgeo expression using X-Gal staining. X-Gal positive (blue) cell lines were then transiently transfected with FLPe or Cre expression plasmids and emerging subclones were stained with X-Gal. As shown in Figure 2, exposure of the gene trap lines to either FLPe (Figure 2A) or Cre (Figure 2B) yielded a mixture of X-Gal positive (blue) and X-Gal negative (white) subclones, indicating that several cell lines have ceased to express βgeo. To test whether this was caused by recombination, we isolated DNA from both the blue and the white sub-lines, and subjected it to an allele-specific PCR. Figure 2 (A and B) shows that, in each case, the amplification products obtained from the blue and white clones corresponded to a normal and to an inverted gene trap allele, respectively. Taken together, the results indicate that both FLPe and Cre can disrupt the gene trap expression by simply flipping it to the anti-sense, non-coding strand.

To test whether the FLPe or Cre inverted cell lines would re-invert following a second recombinase exposure, we re-expressed FLPe and Cre in each of the cell lines and checked their progeny for re-inversions by the allele specific PCR. Figure 2C shows that FLPe readily re-inverted the Cre inverted sub-line FS4B6 C14 (lane 6) but not the FLPe inverted sub-line FS4B6 F14 (lane 9) and conversely, Cre readily re-inverted the FLPe inverted sub-line FS4B6 F14 (lane 8) but not the Cre inverted sub-line FS4B6 C14 (lane 5). Taken together, the results indicate that gene trap re-inversions are inducible only by the recombinase that was not involved in the original inversion, suggesting that the recombination products obtained with either recombinase are stable. Inversions induced by Cre and FLPe in FlipRosaCeo trapped ES cell lines were similarly stable and efficient (see below). In this context, it is noteworthy that under certain circumstances relating to excessive exposure to Cre enzyme either by long periods of exposure in culture or during development or by very high levels of Cre expression some background recombination between heterotypic IoxP/1ox511 sites can occur (Kolb, A. F. Anal Biochem 290, 260-71 (2001); Lauth, M. et al., Genesis 27, 153-8 (2000)). However, in gene trap lines stably transduced with a Cre expression vector, we were unable to detect recombination between loxP and lox511 sites even after several weeks in culture (data not shown), suggesting that background recombination does significantly affect conditional gene trapping.

### Example 3: Reversibility of gene trap mutations

To test whether the mutations induced by the conditional gene trap vectors are reversible, we selected the Q017B06 and M117B08 gene trap lines for further analysis. In Q017B06, the FlipRosaβgeo gene trap vector disrupted the retinoblastoma binding protein 7 (RBBP7) gene at the level of the first intron. In M117B08, the FlipRosaCeo gene trap vector disrupted the glycosyltransferase 28 domain containing 1 gene (Glt28d1) in the 10th intron. Both genes are located on the X-chromosome of a male derived ES cell line, which provided a haploid background for the mutational analysis. As shown in Figures 3 and 4, (panels B and C), the RBBP7 (Figure 3) and Glt28d1 (Figure 4) genes were both expressed in the wild-type cells as expected. However, expression was either blocked (RBBP7, Figure 3) or severely repressed (Glt28d1, Figure 4) by the gene trap insertions. Both trapped cell lines instead expressed fusion transcripts as a result of splicing the upstream exons to the gene trap cassettes (Figures 3, panel B, Figure 4 panels B, C).

A critical issue that could be addressed with these trapped ES cell lines was whether endogenous gene expression would resume after Cre or FLPe induced inversions. Towards this end, we expressed Cre or FLPe in the Q017BO6 and M117B08 cell lines, isolated several sub-lines, and genotyped them by allele-specific PCR (Figure 3,4 panels A). Inverted sub-lines were then analyzed for RBBP7, Git28d1 and gene trap cassette expression using RT-PCR in combination with Northern- and Western blotting. Figures 3 and 4 (panels B and C) show that in both cell lines the endogenous gene expression was restored to wild type levels and the fusion transcripts disappeared, indicating that the anti-sense gene trap insertions do not interfere with gene expression. Finally, to test whether relocating the gene traps back to their original position on the sense, coding strand would re-induce the mutation, we exposed inverted subclones to FLPe or Cre. Figures 3 and 4 show that the re-inverted sub-lines lost the endogenous gene expression, and re-expressed the fusion transcripts, like the original trapped lines. Taken together, the results suggest that the FlipRosapgeo and FlipRosaCeo induced mutations can be repaired and re-induced by the successive activation of the two recombination systems.

### Example 4: Large scale conditional mutagenesis in ES cells

We isolated 4,525 ES cell lines with conditional gene trap insertions and recovered 4,138 gene trap sequence tags by 5'RACE. Of these, 3,257 were derived from FlipRosaβgeo and 881 from FlipRosaCeo integrations. Ninety percent of the FlipRosaβgeo and 99% of the FlipRosaCeo GTSTs belonged to RefSeq annotated genes (Table 1). The number of annotated genes was nearly double that found in our previous analysis (Hansen, J. et al., PNAS 100, 9918 (2003)), reflecting the swift progress in genome annotation. The overall efficiency of trapping was similar to that observed in previous studies, as was the number of preferred insertions sites (i.e., hot spots) (Table 1). Insertions occurred in all chromosomes, including one on the Y chromosome and their number correlated with the number of genes per chromosome (data not shown). Collectively, these observations indicate that the heterotypic lox and frt sites built into the gene trap vectors do not affect the efficiency of trapping. Regardless of the vector, the vast majority of gene trap insertions occurred into first and second introns, confirming the reported preference of retroviral integrations near the 5' ends of genes (Figure 5) (Bushman, F. D., Cell 115, 135 (2003)). As expected, the major difference between the vectors was their ability to capture signal sequence genes. While over 80% of the FlipRosaCeo insertions were in genes encoding secreted or transmembrane proteins, only 21% of FlipRosaβgeo insertions captured secretory pathway genes according to GeneOntology. Thus, like the non-conditional vectors, the two types of conditional gene trap vectors complement each other in gene trapping.

## Claims

1. A gene trap cassette capable of causing conditional mutations in genes, which comprises a functional DNA segment (FS) inserted in a mutagenic or non-mutagenic manner, in sense or antisense direction relative to the gene to be trapped, said FS being flanked by the recombinase recognition sequences (RRSs) of at least two independent directional site-specific recombination systems,
wherein each system
(i) comprises two pairs of heterotypic RRSs, said RRSs being oriented in opposite orientation and the RRSs of the two pairs being lined up in opposite order on both sides of the FS, and
(ii) is capable of inverting FS by means of a recombinase mediated flip-excision mechanism.

2. The gene trap cassette of claim 1, wherein the cassette comprises the structure
5' -L1-A-L2-B-L3-C-L4-FS-L3-D-L4-E-L1-F-L2-3',
wherein
L1 and L2 are the RRSs of the first site-specific recombination system,
L3 and L4 are the RRSs of the second site-specific recombination system, and
A to F are independently from each other either a chemical bond or a spacer polynucleotide.

3. The gene trap cassette of claim 2, wherein
(i) said at least two recombinases specific for the RRSs are selected from the site specific recombinases Cre of bacteriophage P1, FLP recombinase of *Saccharomyces cerevisiae,* R recombinase of *Zygosaccharomyces rouxii* pSR1, the A recombinase of *Kluyveromyces drosophilarium* pKD1, the A recombinase of *K. waltii* pKW1, the integrase λ Int, the recombinase of the GIN recombination system of the Mu phage, the bacterial β recombinase, and variants thereof, preferably are Cre and FLPe and their natural or synthetic variants, most preferably the two recombinases are Cre and FLPe; and/or
(ii) B and E are chemical bonds; and/or
(iii) at least either A or F and either C or D is a spacer polynucleotide; and/or
(iv) the minimum length of the spacer polynucleotides A to F is 30 nt, preferably 70 nt; and/or
(v) one or more of the the spacer polynucleotides A to F are gene coding sequences, preferably for a selectable reporter and/or marker gene.

4. The gene trap cassette of claim 2 or 3, wherein
(i) one recombinase is Cre recombinase and L1 and L2, or L3 and L4 are selected from LoxP, Lox66, Lox71, Lox511, Lox512, Lox514, Lox5171, Lox2272 and other mutants of LoxP, preferably from LoxP (SEQ ID NO:5), Lox511 (SEQ ID NO: 6), Lox5171 (SEQ ID NO:7) and Lox2272 (SEQ ID NO:8), more preferably, L1 (or L3) comprises a LoxP sequence as shown in SEQ ID NO:5 and L2 (or L4) comprises a Lox5171 sequence as shown in SEQ ID NO:7, or vice versa; and/or
(ii) the other recombinase is FLPe recombinase and L3 and L4, or L1 and L2 are selected from frt, F3 and F5, preferably L3 (or L1) comprises a frt sequence as shown in SEQ ID NO:9 and L4 (or L2) comprises a F3 sequence as shown in SEQ ID NO: 10, or vice versa; and/or
(iii) the length of the spacer polynucleotides is about 86 nt for frt/F3 and about 46 nt for loxP/lox5171.

5. The gene trap cassette according to any one of claims 1 to 4, wherein the FS further comprises one or more of the following: splice acceptor, splice donor, internal ribosomal entry site, polyadenylation sequence, a gene coding for a reporter protein, a toxin, a resistance gene and a gene coding for a further site specific recombinase.

6. The gene trap cassette according to any one of claims 1 to 5, which further comprises a selection DNA segment suitable for selecting for genes having an incorporated gene trap cassette, said selection DNA segment comprising a reporter or resistance gene and flanking recombinase recognition sites in same orientation.

7. The gene trap cassette according to any one of claims 1 to 6 which comprises two functional DNA segments,
(a) a first DNA segment (disruption segment) having a FS being oriented in antisense orientation relative to the transcriptional orientation of the gene to be trapped and being flanked by the RRSs of the at least two independent directional site-specific recombination systems, and
(b) a second segment (selection segment) being positioned in sense direction relative to the transcriptional orientation of the gene to be trapped and being flanked by two RRSs of a third site specific recombinase in the same orientation.

8. The gene trap cassette according to claim 7, wherein
(i) in the disruption segment the FS comprises a splice acceptor and a polyadenylation sequence, and the selection segment comprises a reporter or selectable marker gene flanked by an upstream splice acceptor sequence and a downstream polyadenylation sequence; or
(ii) in the disruption segment the FS comprises a splice acceptor and a polyadenylation sequence, and the selection segment comprises a reporter or selectable marker gene fused to an upstream constitutive promoter and a downstream splice donor site,
said gene trap cassette being a conditional gene trap cassette selecting for integrations into all genes..

9. The gene trap cassette according to any one of claims 1 to 8, which can be used in gene targeting and wherein the gene trap cassette is flanked by two homology regions, wherein said homology regions are homologous to an intron sequence of the target gene.

10. A cell, a culture of cells or tissue, or a transgenic organism comprising the gene trap cassette as defined in any one of claims 1 to 9.

11. A process for preparing the cell, the culture of cells or tissue, or the transgenic organism of claim 10, which comprises introducing a gene trap cassette as defined in any one of claims 1 to 9 into a suitable cell.

12. A process for the generation of conditional mutations in one or more genes of an organism comprising
(i) introduction of a gene trap cassette as defined in any one of claims 1 to 9 into a suitable cell,
(ii) selection of cells in which the construct is incorporated in a gene,
(iii) identification and/or isolation of the gene in which the construct is incorporated,
(iv) deletion of the selection cassette from the trapped gene,
(v) induction of a mutation in the trapped gene by inversion of the functional DNA segment.

13. The process according to claim 12, wherein the mutation in step (v) is effected by a site specific recombinase capable of unidirectional inversion of the functional DNA of the gene trapping construct.

14. The process according to claims 12 or 13, wherein the introduction in step (i) is effected by homologous recombination using a cassette as defined in claim 6.

15. The process according to any one of claims 12 to 14, which is suitable for temporarily and/or spatially restricted inactivation of all genes that constitute a living organism.

16. The process according to any one of claims 12 to 15 being a process for preparing transgenic mammals, preferably of non-human mammals such as mice, wherein in step (i) the gene trapping construct is installed in an ES cell.

17. A transgenic mammal obtainable by the method of claim 16.

18. Use of the cell, the culture of cells or tissue, or the transgenic organism of claim 10 for the identification and/or isolation of genes.

19. Use of the transgenic organism of claim 10 or the transgenic mammal of claim 17
(i) to study gene function at various developmental stages;
(ii) as an animal model of human disease; or
(iii) as an *in vivo* drug validation model in drug development.


SEQUENCE LISTING

<110> FrankGen Biotechnologie AG
GSF Forschungszentrum für Umwelt und Gesundheit GmbH
Max-Planck Gesellschaft zur Förderung der Wissenschaften e.V.

<120> Gene Trap Cassettes for Random and Targeted Conditional Gene Inactivation

<130> 050870ep/JH/PCH

<160> 18

<170> PatentIn version 3.1

<210> 1

<211> 8710

<212> DNA

<213> Artificial Sequence

<220>

<223> pFlipROSAβgeo

<400> 1
ctgcagcctg aatatgggcc aaacaggata tctgtggtaa gcagttcctg ccccggctca 60

gggccaagaa cagatggaac agctgaatat gggccaaaca ggatatctgt ggtaagcagt 120

tcctgccccg gctcagggcc aagaacagat ggtccccaga tgcggtccag ccctcagcag 180

tttctagaga accatcagat gtttccaggg tgccccaagg acctgaaatg accctgtgcc 240

ttatttgaac taaccaatca gttcgcttct cgcttctgtt cgcgcgcttc tgctccccga 300

gctcaataaa agagcccaca acccctcact cggggcgcca gtcctccgat tgactgagtc 360

gcccgggtac ccgtgtatcc aataaaccct cttgcagttg catccgactt gtggtctcgc 420

tgttccttgg gagggtctcc tctgagtgat tgactacccg tcagcggggg tctttcattt 480

gggggctcgt ccgggatcgg gagacccctg cccagggacc accgacccac caccgggagg 540

caagctggcc agcaacttat ctgtgtctgt ccgattgtct agtgtctatg actgatttta 600

tgcgcctgcg tcggtactag ttagctaact agctctgtat ctggcggacc cgtggtggaa 660

ctgacgagtt cggaacaccc ggccgcaacc ctgggagacg tcccagggac ttcgggggcc 720

gtttttgtgg cccgacctga gtccaaaaat cccgatcgtt ttggactctt tggtgcaccc 780

cccttagagg agggatatgt ggttctggta ggagacgaga acctaaaaca gttcccgcct 840

ccgtctgaat ttttgctttc ggtttgggac cgaagccgcg ccgcgcgtct tgtctgctgc 900

agcatcgttc tgtgttgtct ctgtctgact gtgtttctgt atttgtctga gaattagggc 960

cagactgtta ccactccctt aagtttgacc ttaggtcact ggaaagatgt cgagcggatc 1020

gctcacaacc agtcggtaga tgtcaagaag agacgttggg ttaccttctg ctctgcagaa 1080

tggccaacct ttaacgtcgg atggccgcga gacggcacct ttaaccgaga cctcatcacc 1140

caggttaaga tcaaggtctt ttcacctggc ccgcatggac acccagacca ggtcccctac 1200

atcgtgacct gggaagcctt ggcttttgac ccccctccct gggtcaagcc ctttgtacac 1260

cctaagcctc cgcctcctct tcctccatcc gccccgtctc tcccccttga acctcctcgt 1320

tcgaccccgc ctcgatcctc cctttatcca gccctcactc cttctctagg cgccggccgg 1380

atccgttgct gaagttccta ttccgaagtt cctattcttc aaatagtata ggaacttcgt 1440

taacgaagtt cctattccga agttcctatt ctctagaaag tataggaact tctcgtgacg 1500

gtctcgaagc cgcggtggag cgaggaagcg gaagagtcta gaataacttc gtatagtata 1560

cattatacga agttatgggt cgatggtgag atctggacta gagggtcgat ggtgatgctt 1620

ggataacttc gtatagcata cattatacga agttatcgga tcccagtgtg gtggtactcg 1680

aggtcgactc tagaggatcg agccccagct ggttctttcc gtctcagaag ccatagagcc 1740

caccgcatcc ccagcatgcc tgctattgtc ttcccaatcc tcccccttgc tgtcctgccc 1800

caccccaccc cccagaatag aatgacacct actcagacaa tgcgatgcaa tttcctcatt 1860

ttattaggaa aggacagtgg gagtggcacc ttccagggtc aaggaaggca cgggggaggg 1920

gcaaacaaca gatggctggc aactagaagg cacagtcgag gctgatcagc gagctctaga 1980

gaattgatcc cctcagaaga actcgtcaag aaggcgatag aaggcgatgc gctgcgaatc 2040

gggagcggcg ataccgtaaa gcacgaggaa gcggtcagcc cattcgccgc caagctcttc 2100

agcaatatca cgggtagcca acgctatgtc ctgatagcgg tccgccacac ccagccggcc 2160

acagtcgatg aatccagaaa agcggccatt ttccaccatg atattcggca agcaggcatc 2220

gccatgggtc acgacgagat cctcgccgtc gggcatgcgc gccttgagcc tggcgaacag 2280

ttcggctggc gcgagcccct gatgctcttc gtccagatca tcctgatcga caagaccggc 2340

ttccatccga gtacgtgctc gctcgatgcg atgtttcgct tggtggtcga atgggcaggt 2400

agccggatca agcgtatgca gccgccgcat tgcatcagcc atgatggata ctttctcggc 2460

aggagcaagg tgagatgaca ggagatcctg ccccggcact tcgcccaata gcagccagtc 2520

ccttcccgct tcagtgacaa cgtcgagcac agctgcgcaa ggaacgcccg tcgtggccag 2580
ccacgatagc cgcgctgcct cgtcctgcag ttcattcagg gcaccggaca ggtcggtctt 2640
gacaaaaaga accgggcgcc cctgcgctga cagccggaac acggcggcat cagagcagcc 2700
gattgtctgt tgtgcccagt catagccgaa tagcctctcc acccaagcgg ccggagaacc 2760
tgcgtgcaat ccatcttgtt caatggccga tcccatattg gctgcagccc gggggatccc 2820
ctgacaccag accaactggt aatggtagcg accggcgctc agctggaatt ccgccgatac 2880
tgacgggctc caggagtcgt cgccaccaat ccccatatgg aaaccgtcga tattcagcca 2940
tgtgccttct tccgcgtgca gcagatggcg atggctggtt tccatcagtt gctgttgact 3000
gtagcggctg atgttgaact ggaagtcgcc gcgccactgg tgtgggccat aattcaattc 3060
gcgcgtcccg cagcgcagac cgttttcgct cgggaagacg tacggggtat acatgtctga 3120
caatggcaga tcccagcggt caaaacaggc ggcagtaagg cggtcgggat agttttcttg 3180
cggccctaat ccgagccagt ttacccgctc tgctacctgc gccagctggc agttcaggcc 3240
aatccgcgcc ggatgcggtg tatcgctcgc cacttcaaca tcaacggtaa tcgccatttg 3300
accactacca tcaatccggt aggttttccg gctgataaat aaggttttcc cctgatgctg 3360
ccacgcgtga gcggtcgtaa tcagcaccgc atcagcaagt gtatctgccg tgcactgcaa 3420
caacgctgct tcggcctggt aatggcccgc cgccttccag cgttcgaccc aggcgttagg 3480
gtcaatgcgg gtcgcttcac ttacgccaat gtcgttatcc agcggtgcac gggtgaactg 3540
atcgcgcagc ggcgtcagca gttgtttttt atcgccaatc cacatctgtg aaagaaagcc 3600
tgactggcgg ttaaattgcc aacgcttatt acccagctcg atgcaaaaat ccatttcgct 3660
ggtggtcaga tgcgggatgg cgtgggacgc ggcggggagc gtcacactga ggttttccgc 3720
cagacgccac tgctgccagg cgctgatgtg cccggcttct gaccatgcgg tcgcgttcgg 3780
ttgcactacg cgtactgtga gccagagttg cccggcgctc tccggctgcg gtagttcagg 3840
cagttcaatc aactgtttac cttgtggagc gacatccaga ggcacttcac cgcttgccag 3900
cggcttacca tccagcgcca ccatccagtg caggagctcg ttatcgctat gacggaacag 3960
gtattcgctg gtcacttcga tggtttgccc ggataaacgg aactggaaaa actgctgctg 4020
gtgttttgct tccgtcagcg ctggatgcgg cgtgcggtcg gcaaagacca gaccgttcat 4080
acagaactgg cgatcgttcg gcgtatcgcc aaaatcaccg ccgtaagccg accacgggtt 4140
gccgttttca tcatatttaa tcagcgactg atccacccag tcccagacga agccgccctg 4200
taaacgggga tactgacgaa acgcctgcca gtatttagcg aaaccgccaa gactgttacc 4260
catcgcgtgg gcgtattcgc aaaggatcag cgggcgcgtc tctccaggta gcgaaagcca 4320
ttttttgatg gaccatttcg gcacagccgg gaagggctgg tcttcatcca cgcgcgcgta 4380
catcgggcaa ataatatcgg tggccgtggt gtcggctccg ccgccttcat actgcaccgg 4440

gcgggaagga tcgacagatt tgatccagcg atacagcgcg tcgtgattag cgccgtggcc 4500

tgattcattc cccagcgacc agatgatcac actcgggtga ttacgatcgc gctgcaccat 4560

tcgcgttacg cgttcgctca tcgccggtag ccagcgcgga tcatcggtca gacgattcat 4620

tggcaccatg ccgtgggttt caatattggc ttcatccacc acatacaggc cgtagcggtc 4680

gcacagcgtg taccacagcg gatggttcgg ataatgcgaa cagcgcacgg cgttaaagtt 4740

gttctgcttc atcagcagga tatcctgcac catcgtctgc tcatccatga cctgaccatg 4800

cagaggatga tgctcgtgac ggttaacgcc tcgaatcagc aacggcttgc cgttcagcag 4860

cagcagacca ttttcaatcc gcacctcgcg gaaaccgaca tcgcaggctt ctgcttcaat 4920

cagcgtgccg tcggcggtgt gcagttcaac caccgcacga tagagattcg ggatttcggc 4980

gctccacagt ttcgggtttt cgacgttcag acgtagtgtg acgcgatcgg cataaccacc 5040

acgctcatcg ataatttcac cgccgaaagg cgcggtgccg ctggcgacct gcgtttcacc 5100

ctgccataaa gaaactgtta cccgtaggta gtcacgcaac tcgccgcaca tctgaacttc 5160

agcctccagt acagcgcggc tgaaatcatc attaaagcga gtggcaacat ggaaatcgct 5220

gatttgtgta gtcggtttat gcagcaacga gacgtcacgg aaaatgccgc tcatccgcca 5280

catatcctga tcttccagat aactgccgtc actccaacgc agcaccatca ccgcgaggcg 5340

gttttctccg gcgcgtaaaa atgcgctcag gtcaaattca gacggcaaac gactgtcctg 5400

gccgtaaccg acccagcgcc cgttgcacca cagatgaaac gccgagttaa cgccatcaaa 5460

aataattcgc gtctggcctt cctgtagcca gctttcatca acattaaatg tgagcgagta 5520

acaacccgtc ggattctccg tgggaacaaa cggcggattg accgtaatgg gataggttac 5580

gttggtgtag atgggcgcat cgtaaccgtg catctgccag tttgagggga cgacgacagt 5640

atcggcctca ggaagatcgc actccagcca gctttccggc accgcttctg gtgccggaaa 5700

ccaggcaaag cgccattcgc cattcaggct gcgcaactgt tgggaagggc gatcggtgcg 5760

ggcctcttcg ctattacgcc agctggcgaa agggggatgt gctgcaaggc gattaagttg 5820

ggtaacgcca gggttttccc agtcacgacg ttgtaaaacg acgggatccg ccatgtcaca 5880

gatcatcaag cttatcgata ccgtcgatcc ccactggaaa gaccgcgaag agtttgtcct 5940

caaccgcgag ctgtggaaaa aaaagggaca ggataagtat gacatcatca aggaaaccct 6000

ggactactgc gccctacaga tctgcagccc gggggatcca ctagttctag cctcgagtag 6060

gaattcgata acttcgtata atgtatacta tacgaagtta tgggtcgatg gtgatgcttg 6120

gcaattcggg tcgatggtga agcttggata acttcgtata atgtatgcta tacgaagtta 6180

tcaattcgcc ctatagtgag tcgtattctc ccgaaaaccg cttctagcaa cgaagttcct 6240

atactatttg aagaatagga acttcggaat aggaacttca gcagatcttc tgtatcgtct 6300

atcgttatgt ctaatggtat ctgatgcaga tctgctgaag ttcctatact ttctagagaa 6360

taggaacttc ggaataggaa cttcgaattc tcgagggccc gggctcgacc agctgtgcgc 6420

atagtggctt gaatcgataa aataaaagat tttatttagt ctccagaaaa aggggggaat 6480

gaaagacccc acctgtaggt ttggcaagct agcacaaccc ctcactcggg gcgccagtcc 6540

tccgattgac tgagtcgccc gggtacccgt gtatccaata aaccctcttg cagttgcatc 6600

cgacttgtgg tctcgctgtt ccttgggagg gtctcctctg agtgattgac tacccgtcag 6660

cgggggtctt tcacatgcag catgtatcaa aattaatttg gttttttttc ttaagtattt 6720

acattaaatg gccatagttg cattaatgaa tcggccaacg cgcggggaga ggcggtttgc 6780

gtattgggcg ctcttccgct tcctcgctca ctgactcgct gcgctcggtc gttcggctgc 6840

ggcgagcggt atcagctcac tcaaaggcgg taatacggtt atccacagaa tcaggggata 6900

acgcaggaaa gaacatgtga gcaaaaggcc agcaaaaggc caggaaccgt aaaaaggccg 6960

cgttgctggc gtttttccat aggctccgcc cccctgacga gcatcacaaa aatcgacgct 7020

caagtcagag gtggcgaaac ccgacaggac tataaagata ccaggcgttt ccccctggaa 7080

gctccctcgt gcgctctcct gttccgaccc tgccgcttac cggatacctg tccgcctttc 7140

tcccttcggg aagcgtggcg ctttctcata gctcacgctg taggtatctc agttcggtgt 7200

aggtcgttcg ctccaagctg ggctgtgtgc acgaaccccc cgttcagccc gaccgctgcg 7260

ccttatccgg taactatcgt cttgagtcca acccggtaag acacgactta tcgccactgg 7320

cagcagccac tggtaacagg attagcagag cgaggtatgt aggcggtgct acagagttct 7380

tgaagtggtg gcctaactac ggctacacta gaaggacagt atttggtatc tgcgctctgc 7440

tgaagccagt taccttcgga aaaagagttg gtagctcttg atccggcaaa caaaccaccg 7500

ctggtagcgg tggttttttt gtttgcaagc agcagattac gcgcagaaaa aaaggatctc 7560

aagaagatcc tttgatcttt tctacggggt ctgacgctca gtggaacgaa aactcacgtt 7620

aagggatttt ggtcatgaga ttatcaaaaa ggatcttcac ctagatcctt ttgcggccgc 7680

aaatcaatct aaagtatata tgagtaaact tggtctgaca gttaccaatg cttaatcagt 7740

gaggcaccta tctcagcgat ctgtctattt cgttcatcca tagttgcctg actccccgtc 7800

gtgtagataa ctacgatacg ggagggctta ccatctggcc ccagtgctgc aatgataccg 7860

cgagacccac gctcaccggc tccagattta tcagcaataa accagccagc cggaagggcc 7920

gagcgcagaa gtggtcctgc aactttatcc gcctccatcc agtctattaa ttgttgccgg 7980

gaagctagag taagtagttc gccagttaat agtttgcgca acgttgttgc cattgctgca 8040

ggcatcgtgg tgtcacgctc gtcgtttggt atggcttcat tcagctccgg ttcccaacga 8100

tcaaggcgag ttacatgatc ccccatgttg tgcaaaaaag cggttagctc cttcggtcct 8160

ccgatcgttg tcagaagtaa gttggccgca gtgttatcac tcatggttat ggcagcactg 8220

cataattctc ttactgtcat gccatccgta agatgctttt ctgtgactgg tgagtactca 8280

accaagtcat tctgagaata gtgtatgcgg cgaccgagtt gctcttgccc ggcgtcaata 8340

cgggataata ccgcgccaca tagcagaact ttaaaagtgc tcatcattgg aaaacgttct 8400

tcggggcgaa aactctcaag gatcttaccg ctgttgagat ccagttcgat gtaacccact 8460

cgtgcaccca actgatcttc agcatctttt actttcacca gcgtttctgg gtgagcaaaa 8520

acaggaaggc aaaatgccgc aaaaaaggga ataagggcga cacggaaatg ttgaatactc 8580

atactcttcc tttttcaata ttattgaagc atttatcagg gttattgtct catgagcgga 8640

tacatatttg aatgtattta gaaaaataaa caaatagggg ttccgcgcac atttccccga 8700

aaagtgccac 8710

<210> 2

<211> 8710

<212> DNA

<213> Artificial Sequence

<220>

<223> prFlipROSAβgeo

<400> 2
ctgcagcctg aatatgggcc aaacaggata tctgtggtaa gcagttcctg ccccggctca 60

gggccaagaa cagatggaac agctgaatat gggccaaaca ggatatctgt ggtaagcagt 120

tcctgccccg gctcagggcc aagaacagat ggtccccaga tgcggtccag ccctcagcag 180

tttctagaga accatcagat gtttccaggg tgccccaagg acctgaaatg accctgtgcc 240

ttatttgaac taaccaatca gttcgcttct cgcttctgtt cgcgcgcttc tgctccccga 300

gctcaataaa agagcccaca acccctcact cggggcgcca gtcctccgat tgactgagtc 360

gcccgggtac ccgtgtatcc aataaaccct cttgcagttg catccgactt gtggtctcgc 420

tgttccttgg gagggtctcc tctgagtgat tgactacccg tcagcggggg tctttcattt 480

gggggctcgt ccgggatcgg gagacccctg cccagggacc accgacccac caccgggagg 540

caagctggcc agcaacttat ctgtgtctgt ccgattgtct agtgtctatg actgatttta 600

tgcgcctgcg tcggtactag ttagctaact agctctgtat ctggcggacc cgtggtggaa 660

ctgacgagtt cggaacaccc ggccgcaacc ctgggagacg tcccagggac ttcgggggcc 720

gtttttgtgg cccgacctga gtccaaaaat cccgatcgtt ttggactctt tggtgcaccc 780

cccttagagg agggatatgt ggttctggta ggagacgaga acctaaaaca gttcccgcct 840

ccgtctgaat ttttgctttc ggtttgggac cgaagccgcg ccgcgcgtct tgtctgctgc 900

agcatcgttc tgtgttgtct ctgtctgact gtgtttctgt atttgtctga gaattagggc 960

cagactgtta ccactccctt aagtttgacc ttaggtcact ggaaagatgt cgagcggatc 1020

gctcacaacc agtcggtaga tgtcaagaag agacgttggg ttaccttctg ctctgcagaa 1080

tggccaacct ttaacgtcgg atggccgcga gacggcacct ttaaccgaga cctcatcacc 1140

caggttaaga tcaaggtctt ttcacctggc ccgcatggac acccagacca ggtcccctac 1200

atcgtgacct gggaagcctt ggcttttgac ccccctccct gggtcaagcc ctttgtacac 1260

cctaagcctc cgcctcctct tcctccatcc gccccgtctc tcccccttga acctcctcgt 1320

tcgaccccgc ctcgatcctc cctttatcca gccctcactc cttctctagg cgccggccgg 1380

atccgttgct gaagttccta ttccgaagtt cctattcttc aaatagtata ggaacttcgt 1440

taacgaagtt cctattccga agttcctatt ctctagaaag tataggaact tctcgtgacg 1500

gtctcgaagc cgcggtggag cgaggaagcg gaagagtcta gaataacttc gtatagtaca 1560

cattatacga agttatgggt cgatggtgag atctggacta gagggtcgat ggtgatgctt 1620

ggataacttc gtatagcata cattatacga agttatcgga tcccagtgtg gtggtactcg 1680

aggtcgactc tagaggatcg agccccagct ggttctttcc gtctcagaag ccatagagcc 1740

caccgcatcc ccagcatgcc tgctattgtc ttcccaatcc tcccccttgc tgtcctgccc 1800

caccccaccc cccagaatag aatgacacct actcagacaa tgcgatgcaa tttcctcatt 1860

ttattaggaa aggacagtgg gagtggcacc ttccagggtc aaggaaggca cgggggaggg 1920

gcaaacaaca gatggctggc aactagaagg cacagtcgag gctgatcagc gagctctaga 1980

gaattgatcc cctcagaaga actcgtcaag aaggcgatag aaggcgatgc gctgcgaatc 2040

gggagcggcg ataccgtaaa gcacgaggaa gcggtcagcc cattcgccgc caagctcttc 2100

agcaatatca cgggtagcca acgctatgtc ctgatagcgg tccgccacac ccagccggcc 2160

acagtcgatg aatccagaaa agcggccatt ttccaccatg atattcggca agcaggcatc 2220

gccatgggtc acgacgagat cctcgccgtc gggcatgcgc gccttgagcc tggcgaacag 2280

ttcggctggc gcgagcccct gatgctcttc gtccagatca tcctgatcga caagaccggc 2340

ttccatccga gtacgtgctc gctcgatgcg atgtttcgct tggtggtcga atgggcaggt 2400

agccggatca agcgtatgca gccgccgcat tgcatcagcc atgatggata ctttctcggc 2460

aggagcaagg tgagatgaca ggagatcctg ccccggcact tcgcccaata gcagccagtc 2520

ccttcccgct tcagtgacaa cgtcgagcac agctgcgcaa ggaacgcccg tcgtggccag 2580

ccacgatagc cgcgctgcct cgtcctgcag ttcattcagg gcaccggaca ggtcggtctt 2640

gacaaaaaga accgggcgcc cctgcgctga cagccggaac acggcggcat cagagcagcc 2700

gattgtctgt tgtgcccagt catagccgaa tagcctctcc acccaagcgg ccggagaacc 2760
tgcgtgcaat ccatcttgtt caatggccga tcccatattg gctgcagccc gggggatccc 2820
ctgacaccag accaactggt aatggtagcg accggcgctc agctggaatt ccgccgatac 2880
tgacgggctc caggagtcgt cgccaccaat ccccatatgg aaaccgtcga tattcagcca 2940
tgtgccttct tccgcgtgca gcagatggcg atggctggtt tccatcagtt gctgttgact 3000
gtagcggctg atgttgaact ggaagtcgcc gcgccactgg tgtgggccat aattcaattc 3060
gcgcgtcccg cagcgcagac cgttttcgct cgggaagacg tacggggtat acatgtctga 3120
caatggcaga tcccagcggt caaaacaggc ggcagtaagg cggtcgggat agttttcttg 3180
cggccctaat ccgagccagt ttacccgctc tgctacctgc gccagctggc agttcaggcc 3240
aatccgcgcc ggatgcggtg tatcgctcgc cacttcaaca tcaacggtaa tcgccatttg 3300
accactacca tcaatccggt aggttttccg gctgataaat aaggttttcc cctgatgctg 3360
ccacgcgtga gcggtcgtaa tcagcaccgc atcagcaagt gtatctgccg tgcactgcaa 3420
caacgctgct tcggcctggt aatggcccgc cgccttccag cgttcgaccc aggcgttagg 3480
gtcaatgcgg gtcgcttcac ttacgccaat gtcgttatcc agcggtgcac gggtgaactg 3540
atcgcgcagc ggcgtcagca gttgtttttt atcgccaatc cacatctgtg aaagaaagcc 3600
tgactggcgg ttaaattgcc aacgcttatt acccagctcg atgcaaaaat ccatttcgct 3660
ggtggtcaga tgcgggatgg cgtgggacgc ggcggggagc gtcacactga ggttttccgc 3720
cagacgccac tgctgccagg cgctgatgtg cccggcttct gaccatgcgg tcgcgttcgg 3780
ttgcactacg cgtactgtga gccagagttg cccggcgctc tccggctgcg gtagttcagg 3840
cagttcaatc aactgtttac cttgtggagc gacatccaga ggcacttcac cgcttgccag 3900
cggcttacca tccagcgcca ccatccagtg caggagctcg ttatcgctat gacggaacag 3960
gtattcgctg gtcacttcga tggtttgccc ggataaacgg aactggaaaa actgctgctg 4020
gtgttttgct tccgtcagcg ctggatgcgg cgtgcggtcg gcaaagacca gaccgttcat 4080
acagaactgg cgatcgttcg gcgtatcgcc aaaatcaccg ccgtaagccg accacgggtt 4140
gccgttttca tcatatttaa tcagcgactg atccacccag tcccagacga agccgccctg 4200
taaacgggga tactgacgaa acgcctgcca gtatttagcg aaaccgccaa gactgttacc 4260
catcgcgtgg gcgtattcgc aaaggatcag cgggcgcgtc tctccaggta gcgaaagcca 4320
ttttttgatg gaccatttcg gcacagccgg gaagggctgg tcttcatcca cgcgcgcgta 4380
catcgggcaa ataatatcgg tggccgtggt gtcggctccg ccgccttcat actgcaccgg 4440
gcgggaagga tcgacagatt tgatccagcg atacagcgcg tcgtgattag cgccgtggcc 4500
tgattcattc cccagcgacc agatgatcac actcgggtga ttacgatcgc gctgcaccat 4560
tcgcgttacg cgttcgctca tcgccggtag ccagcgcgga tcatcggtca gacgattcat 4620

tggcaccatg ccgtgggttt caatattggc ttcatccacc acatacaggc cgtagcggtc 4680

gcacagcgtg taccacagcg gatggttcgg ataatgcgaa cagcgcacgg cgttaaagtt 4740

gttctgcttc atcagcagga tatcctgcac catcgtctgc tcatccatga cctgaccatg 4800

cagaggatga tgctcgtgac ggttaacgcc tcgaatcagc aacggcttgc cgttcagcag 4860

cagcagacca ttttcaatcc gcacctcgcg gaaaccgaca tcgcaggctt ctgcttcaat 4920

cagcgtgccg tcggcggtgt gcagttcaac caccgcacga tagagattcg ggatttcggc 4980

gctccacagt ttcgggtttt cgacgttcag acgtagtgtg acgcgatcgg cataaccacc 5040

acgctcatcg ataatttcac cgccgaaagg cgcggtgccg ctggcgacct gcgtttcacc 5100

ctgccataaa gaaactgtta cccgtaggta gtcacgcaac tcgccgcaca tctgaacttc 5160

agcctccagt acagcgcggc tgaaatcatc attaaagcga gtggcaacat ggaaatcgct 5220

gatttgtgta gtcggtttat gcagcaacga gacgtcacgg aaaatgccgc tcatccgcca 5280

catatcctga tcttccagat aactgccgtc actccaacgc agcaccatca ccgcgaggcg 5340

gttttctccg gcgcgtaaaa atgcgctcag gtcaaattca gacggcaaac gactgtcctg 5400

gccgtaaccg acccagcgcc cgttgcacca cagatgaaac gccgagttaa cgccatcaaa 5460

aataattcgc gtctggcctt cctgtagcca gctttcatca acattaaatg tgagcgagta 5520

acaacccgtc ggattctccg tgggaacaaa cggcggattg accgtaatgg gataggttac 5580

gttggtgtag atgggcgcat cgtaaccgtg catctgccag tttgagggga cgacgacagt 5640

atcggcctca ggaagatcgc actccagcca gctttccggc accgcttctg gtgccggaaa 5700

ccaggcaaag cgccattcgc cattcaggct gcgcaactgt tgggaagggc gatcggtgcg 5760

ggcctcttcg ctattacgcc agctggcgaa agggggatgt gctgcaaggc gattaagttg 5820

ggtaacgcca gggttttccc agtcacgacg ttgtaaaacg acggatccgg ccatgtcaca 5880

gatcatcaag cttatcgata ccgtcgatcc ccactggaaa gaccgcgaag agtttgtcct 5940

caaccgcgag ctgtggaaaa aaaagggaca ggataagtat gacatcatca aggaaaccct 6000

ggactactgc gccctacaga tctgcagccc gggggatcca ctagttctag cctcgagtag 6060

gaattcgata acttcgtata atgtgtacta tacgaagtta tgggtcgatg gtgatgcttg 6120

gcaattcggg tcgatggtga agcttggata acttcgtata atgtatgcta tacgaagtta 6180

tcaattcgcc ctatagtgag tcgtattctc ccgaaaaccg cttctagcaa cgaagttcct 6240

atactatttg aagaatagga acttcggaat aggaacttca gcagatcttc tgtatcgtct 6300

atcgttatgt ctaatggtat ctgatgcaga tctgctgaag ttcctatact ttctagagaa 6360

taggaacttc ggaataggaa cttcgaattc tcgagggccc gggctcgacc agctgtgcgc 6420

atagtggctt gaatcgataa aataaaagat tttatttagt ctccagaaaa aggggggaat 6480

gaaagacccc acctgtaggt ttggcaagct agcacaaccc ctcactcggg gcgccagtcc 6540

tccgattgac tgagtcgccc gggtacccgt gtatccaata aaccctcttg cagttgcatc 6600

cgacttgtgg tctcgctgtt ccttgggagg gtctcctctg agtgattgac tacccgtcag 6660

cgggggtctt tcacatgcag catgtatcaa aattaatttg gttttttttc ttaagtattt 6720

acattaaatg gccatagttg cattaatgaa tcggccaacg cgcggggaga ggcggtttgc 6780

gtattgggcg ctcttccgct tcctcgctca ctgactcgct gcgctcggtc gttcggctgc 6840

ggcgagcggt atcagctcac tcaaaggcgg taatacggtt atccacagaa tcaggggata 6900

acgcaggaaa gaacatgtga gcaaaaggcc agcaaaaggc caggaaccgt aaaaaggccg 6960

cgttgctggc gtttttccat aggctccgcc cccctgacga gcatcacaaa aatcgacgct 7020

caagtcagag gtggcgaaac ccgacaggac tataaagata ccaggcgttt ccccctggaa 7080

gctccctcgt gcgctctcct gttccgaccc tgccgcttac cggatacctg tccgcctttc 7140

tcccttcggg aagcgtggcg ctttctcata gctcacgctg taggtatctc agttcggtgt 7200

aggtcgttcg ctccaagctg ggctgtgtgc acgaaccccc cgttcagccc gaccgctgcg 7260

ccttatccgg taactatcgt cttgagtcca acccggtaag acacgactta tcgccactgg 7320

cagcagccac tggtaacagg attagcagag cgaggtatgt aggcggtgct acagagttct 7380

tgaagtggtg gcctaactac ggctacacta gaaggacagt atttggtatc tgcgctctgc 7440

tgaagccagt taccttcgga aaaagagttg gtagctcttg atccggcaaa caaaccaccg 7500

ctggtagcgg tggttttttt gtttgcaagc agcagattac gcgcagaaaa aaaggatctc 7560

aagaagatcc tttgatcttt tctacggggt ctgacgctca gtggaacgaa aactcacgtt 7620

aagggatttt ggtcatgaga ttatcaaaaa ggatcttcac ctagatcctt ttgcggccgc 7680

aaatcaatct aaagtatata tgagtaaact tggtctgaca gttaccaatg cttaatcagt 7740

gaggcaccta tctcagcgat ctgtctattt cgttcatcca tagttgcctg actccccgtc 7800

gtgtagataa ctacgatacg ggagggctta ccatctggcc ccagtgctgc aatgataccg 7860

cgagacccac gctcaccggc tccagattta tcagcaataa accagccagc cggaagggcc 7920

gagcgcagaa gtggtcctgc aactttatcc gcctccatcc agtctattaa ttgttgccgg 7980

gaagctagag taagtagttc gccagttaat agtttgcgca acgttgttgc cattgctgca 8040

ggcatcgtgg tgtcacgctc gtcgtttggt atggcttcat tcagctccgg ttcccaacga 8100

tcaaggcgag ttacatgatc ccccatgttg tgcaaaaaag cggttagctc cttcggtcct 8160

ccgatcgttg tcagaagtaa gttggccgca gtgttatcac tcatggttat ggcagcactg 8220

cataattctc ttactgtcat gccatccgta agatgctttt ctgtgactgg tgagtactca 8280

accaagtcat tctgagaata gtgtatgcgg cgaccgagtt gctcttgccc ggcgtcaata 8340

cgggataata ccgcgccaca tagcagaact ttaaaagtgc tcatcattgg aaaacgttct 8400

tcggggcgaa aactctcaag gatcttaccg ctgttgagat ccagttcgat gtaacccact 8460

cgtgcaccca actgatcttc agcatctttt actttcacca gcgtttctgg gtgagcaaaa 8520

acaggaaggc aaaatgccgc aaaaaaggga ataagggcga cacggaaatg ttgaatactc 8580

atactcttcc tttttcaata ttattgaagc atttatcagg gttattgtct catgagcgga 8640

tacatatttg aatgtattta gaaaaataaa caaatagggg ttccgcgcac atttccccga 8700

aaagtgccac 8710

<210> 3

<211> 6223

<212> DNA

<213> Artificial Sequence

<220>

<223> pFlipROSACeo

<400> 3
ctgcagcctg aatatgggcc aaacaggata tctgtggtaa gcagttcctg ccccggctca 60

gggccaagaa cagatggaac agctgaatat gggccaaaca ggatatctgt ggtaagcagt 120

tcctgccccg gctcagggcc aagaacagat ggtccccaga tgcggtccag ccctcagcag 180

tttctagaga accatcagat gtttccaggg tgccccaagg acctgaaatg accctgtgcc 240

ttatttgaac taaccaatca gttcgcttct cgcttctgtt cgcgcgcttc tgctccccga 300

gctcaataaa agagcccaca acccctcact cggggcgcca gtcctccgat tgactgagtc 360

gcccgggtac ccgtgtatcc aataaaccct cttgcagttg catccgactt gtggtctcgc 420

tgttccttgg gagggtctcc tctgagtgat tgactacccg tcagcggggg tctttcattt 480

gggggctcgt ccgggatcgg gagacccctg cccagggacc accgacccac caccgggagg 540

caagctggcc agcaacttat ctgtgtctgt ccgattgtct agtgtctatg actgatttta 600

tgcgcctgcg tcggtactag ttagctaact agctctgtat ctggcggacc cgtggtggaa 660

ctgacgagtt cggaacaccc ggccgcaacc ctgggagacg tcccagggac ttcgggggcc 720

gtttttgtgg cccgacctga gtccaaaaat cccgatcgtt ttggactctt tggtgcaccc 780

cccttagagg agggatatgt ggttctggta ggagacgaga acctaaaaca gttcccgcct 840

ccgtctgaat ttttgctttc ggtttgggac cgaagccgcg ccgcgcgtct tgtctgctgc 900

agcatcgttc tgtgttgtct ctgtctgact gtgtttctgt atttgtctga gaattagggc 960

cagactgtta ccactccctt aagtttgacc ttaggtcact ggaaagatgt cgagcggatc 1020

gctcacaacc agtcggtaga tgtcaagaag agacgttggg ttaccttctg ctctgcagaa 1080

tggccaacct ttaacgtcgg atggccgcga gacggcacct ttaaccgaga cctcatcacc 1140

caggttaaga tcaaggtctt ttcacctggc ccgcatggac acccagacca ggtcccctac 1200

atcgtgacct gggaagcctt ggcttttgac ccccctccct gggtcaagcc ctttgtacac 1260

cctaagcctc cgcctcctct tcctccatcc gccccgtctc tcccccttga acctcctcgt 1320

tcgaccccgc ctcgatcctc cctttatcca gccctcactc cttctctagg cgccggccgg 1380

atccgttgct gaagttccta ttccgaagtt cctattcttc aaatagtata ggaacttcgt 1440

taacgaagtt cctattccga agttcctatt ctctagaaag tataggaact tctcgtgacg 1500

gtctcgaagc cgcggtggag cgaggaagcg gaagagtcta gaataacttc gtatagtata 1560

cattatacga agttatgggt cgatggtgag atctggacta gagggtcgat ggtgatgctt 1620

ggataacttc gtatagcata cattatacga agttatcgga tcccagtgtg gtggtacatt 1680

gaggtcgact ctagaggatc gagccccagc tggttctttc cgcctcagaa gccatagagc 1740

ccaccgcatc cccagcatgc ctgctattgt cttcccaatc ctcccccttg ctgtcctgcc 1800

ccaccccacc ccccagaata gaatgacacc tactcagaca atgcgatgca atttcctcat 1860

tttattagga aaggacagtg ggagtggcac cttccagggt caaggaaggc acgggggagg 1920

ggcaaacaac agatggctgg caactagaag gcacagtcga ggctgatcag cgagctctag 1980

agaattgatc tacgtattag aagaactcgt caagaaggcg atagaaggcg atgcgctgcg 2040

aatcgggagc ggcgataccg taaagcacga ggaagcggtc agcccattcg ccgccaagct 2100

cttcagcaat atcacgggta gccaacgcta tgtcctgata gcggtccgcc acacccagcc 2160

ggccacagtc gatgaatcca gaaaagcggc cattttccac catgatattc ggcaagcagg 2220

catcgccatg ggtcacgacg agatcctcgc cgtcgggcat gcgcgccttg agcctggcga 2280

acagttcggc tggcgcgagc ccctgatgct cttcgtccag atcatcctga tcgacaagac 2340

cggcttccat ccgagtacgt gctcgctcga tgcgatgttt cgcttggtgg tcgaatgggc 2400

aggtagccgg atcaagcgta tgcagccgcc gcattgcatc agccatgatg gatactttct 2460

cggcaggagc aaggtgagat gacaggagat cctgccccgg cacttcgccc aatagcagcc 2520

agtcccttcc cgcttcagtg acaacgtcga gcacagctgc gcaaggaacg cccgtcgtgg 2580

ccagccacga tagccgcgct gcctcgtcct gcagttcatt cagggcaccg gacaggtcgg 2640

tcttgacaaa aagaaccggg cgcccctgcg ctgacagccg gaacacggcg gcatcagagc 2700

agccgattgt ctgttgtgcc cagtcatagc cgaatagcct ctccacccaa gcggccggag 2760

aacctgcgtg caatccatct tgttcaatgg gatccgtagc tactctgtgg gctcttgtct 2820

ccagctcctc atcatttctc cgactcctct gttttttcct tttggtgata tagaaaacga 2880

gcagtgccac aaagaccatc aagaggctgc ctcctccaca tatgccaatg atgagataga 2940

tgtccagacc tttctctgga cagctgacag gctcgacact ggattccttg ctgactttgt 3000

tccctgctgt gcacttgaat tttgcactca ggctggtggt ccacttgtgt gtgatgaccc 3060

tctgagaaag ttttagatgt ttcccatctt gatacaggtt taattcgggg tcagttccat 3120

tcattacctc acaggtcagg gttgtgttga tacaagtcca ggagatcttt ggttttgaga 3180

ccctctcttg aatcttcaaa tcaaatattt tttccaacac attttttcct tttgtatcat 3240

atattgatac cttgtagata tcctgatcat cggtcttcag atgcttaatt ttcagagttc 3300

catttttaaa tagcttatat gtatcttttt ccttgaaagt ctctttctct tttctgaatt 3360

gtgcaatctt tttcttgtct gaagtttttt cccattttat atcgtcaata tcatcactca 3420

tttgaaaact aggaatgtcc aagttgatgt cctgacccaa ggcaccccag gtttccaagg 3480

cattcgtaat ctctttggag actgcacctt tggaagaaac attgaaaatc agaaggaagc 3540

tggctacaaa tttacatgga atctagtacg taggaattcg ataacttcgt ataatgtata 3600

ctatacgaag ttatgggtcg atggtgatgc ttggcaattc gggtcgatgg tgaagcttgg 3660

ataacttcgt ataatgtatg ctatacgaag ttatcaattc gccctatagt gagtcgtatt 3720

ctcccgaaaa ccgcttctag caacgaagtt cctatactat ttgaagaata ggaacttcgg 3780

aataggaact tcagcagatc ttctgtatcg tctatcgtta tgtctaatgg tatctgatgc 3840

agatctgctg aagttcctat actttctaga gaataggaac ttcggaatag gaacttcgaa 3900

ttctcgaggg cccgggctcg accagctgtg cgcatagtgg cttgaatcga taaaataaaa 3960

gattttattt agtctccaga aaaagggggg aatgaaagac cccacctgta ggtttggcaa 4020

gctagcacaa cccctcactc ggggcgccag tcctccgatt gactgagtcg cccgggtacc 4080

cgtgtatcca ataaaccctc ttgcagttgc atccgacttg tggtctcgct gttccttggg 4140

agggtctcct ctgagtgatt gactacccgt cagcgggggt ctttcacatg cagcatgtat 4200

caaaattaat ttggtttttt ttcttaagta tttacattaa atggccatag ttgcattaat 4260

gaatcggcca acgcgcgggg agaggcggtt tgcgtattgg gcgctcttcc gcttcctcgc 4320

tcactgactc gctgcgctcg gtcgttcggc tgcggcgagc ggtatcagct cactcaaagg 4380

cggtaatacg gttatccaca gaatcagggg ataacgcagg aaagaacatg tgagcaaaag 4440

gccagcaaaa ggccaggaac cgtaaaaagg ccgcgttgct ggcgtttttc cataggctcc 4500

gcccccctga cgagcatcac aaaaatcgac gctcaagtca gaggtggcga aacccgacag 4560

gactataaag ataccaggcg tttccccctg gaagctccct cgtgcgctct cctgttccga 4620

ccctgccgct taccggatac ctgtccgcct ttctcccttc gggaagcgtg gcgctttctc 4680

atagctcacg ctgtaggtat ctcagttcgg tgtaggtcgt tcgctccaag ctgggctgtg 4740

tgcacgaacc ccccgttcag cccgaccgct gcgccttatc cggtaactat cgtcttgagt 4800

ccaacccggt aagacacgac ttatcgccac tggcagcagc cactggtaac aggattagca 4860

gagcgaggta tgtaggcggt gctacagagt tcttgaagtg gtggcctaac tacggctaca 4920

ctagaaggac agtatttggt atctgcgctc tgctgaagcc agttaccttc ggaaaaagag 4980

ttggtagctc ttgatccggc aaacaaacca ccgctggtag cggtggtttt tttgtttgca 5040

agcagcagat tacgcgcaga aaaaaaggat ctcaagaaga tcctttgatc ttttctacgg 5100

ggtctgacgc tcagtggaac gaaaactcac gttaagggat tttggtcatg agattatcaa 5160

aaaggatctt cacctagatc cttttgcggc cgcaaatcaa tctaaagtat atatgagtaa 5220

acttggtctg acagttacca atgcttaatc agtgaggcac ctatctcagc gatctgtcta 5280

tttcgttcat ccatagttgc ctgactcccc gtcgtgtaga taactacgat acgggagggc 5340

ttaccatctg gccccagtgc tgcaatgata ccgcgagacc cacgctcacc ggctccagat 5400

ttatcagcaa taaaccagcc agccggaagg gccgagcgca gaagtggtcc tgcaacttta 5460

tccgcctcca tccagtctat taattgttgc cgggaagcta gagtaagtag ttcgccagtt 5520

aatagtttgc gcaacgttgt tgccattgct gcaggcatcg tggtgtcacg ctcgtcgttt 5580

ggtatggctt cattcagctc cggttcccaa cgatcaaggc gagttacatg atcccccatg 5640

ttgtgcaaaa aagcggttag ctccttcggt cctccgatcg ttgtcagaag taagttggcc 5700

gcagtgttat cactcatggt tatggcagca ctgcataatt ctcttactgt catgccatcc 5760

gtaagatgct tttctgtgac tggtgagtac tcaaccaagt cattctgaga atagtgtatg 5820

cggcgaccga gttgctcttg cccggcgtca atacgggata ataccgcgcc acatagcaga 5880

actttaaaag tgctcatcat tggaaaacgt tcttcggggc gaaaactctc aaggatctta 5940

ccgctgttga gatccagttc gatgtaaccc actcgtgcac ccaactgatc ttcagcatct 6000

tttactttca ccagcgtttc tgggtgagca aaaacaggaa ggcaaaatgc cgcaaaaaag 6060

ggaataaggg cgacacggaa atgttgaata ctcatactct tcctttttca atattattga 6120

agcatttatc agggttattg tctcatgagc ggatacatat ttgaatgtat ttagaaaaat 6180

aaacaaatag gggttccgcg cacatttccc cgaaaagtgc cac 6223

<210> 4

<211> 6223

<212> DNA

<213> Artificial Sequence

<220>

<223> prFlipROSACeo

<400> 4
ctgcagcctg aatatgggcc aaacaggata tctgtggtaa gcagttcctg ccccggctca 60

gggccaagaa cagatggaac agctgaatat gggccaaaca ggatatctgt ggtaagcagt 120

tcctgccccg gctcagggcc aagaacagat ggtccccaga tgcggtccag ccctcagcag 180

tttctagaga accatcagat gtttccaggg tgccccaagg acctgaaatg accctgtgcc 240

ttatttgaac taaccaatca gttcgcttct cgcttctgtt cgcgcgcttc tgctccccga 300

gctcaataaa agagcccaca acccctcact cggggcgcca gtcctccgat tgactgagtc 360

gcccgggtac ccgtgtatcc aataaaccct cttgcagttg catccgactt gtggtctcgc 420

tgttccttgg gagggtctcc tctgagtgat tgactacccg tcagcggggg tctttcattt 480

gggggctcgt ccgggatcgg gagacccctg cccagggacc accgacccac caccgggagg 540

caagctggcc agcaacttat ctgtgtctgt ccgattgtct agtgtctatg actgatttta 600

tgcgcctgcg tcggtactag ttagctaact agctctgtat ctggcggacc cgtggtggaa 660

ctgacgagtt cggaacaccc ggccgcaacc ctgggagacg tcccagggac ttcgggggcc 720

gtttttgtgg cccgacctga gtccaaaaat cccgatcgtt ttggactctt tggtgcaccc 780

cccttagagg agggatatgt ggttctggta ggagacgaga acctaaaaca gttcccgcct 840

ccgtctgaat ttttgctttc ggtttgggac cgaagccgcg ccgcgcgtct tgtctgctgc 900

agcatcgttc tgtgttgtct ctgtctgact gtgtttctgt atttgtctga gaattagggc 960

cagactgtta ccactccctt aagtttgacc ttaggtcact ggaaagatgt cgagcggatc 1020

gctcacaacc agtcggtaga tgtcaagaag agacgttggg ttaccttctg ctctgcagaa 1080

tggccaacct ttaacgtcgg atggccgcga gacggcacct ttaaccgaga cctcatcacc 1140

caggttaaga tcaaggtctt ttcacctggc ccgcatggac acccagacca ggtcccctac 1200

atcgtgacct gggaagcctt ggcttttgac ccccctccct gggtcaagcc ctttgtacac 1260

cctaagcctc cgcctcctct tcctccatcc gccccgtctc tcccccttga acctcctcgt 1320

tcgaccccgc ctcgatcctc cctttatcca gccctcactc cttctctagg cgccggccgg 1380

atccgttgct gaagttccta ttccgaagtt cctattcttc aaatagtata ggaacttcgt 1440

taacgaagtt cctattccga agttcctatt ctctagaaag tataggaact tctcgtgacg 1500

gtctcgaagc cgcggtggag cgaggaagcg gaagagtcta gaataacttc gtatagtaca 1560

cattatacga agttatgggt cgatggtgag atctggacta gagggtcgat ggtgatgctt 1620

ggataacttc gtatagcata cattatacga agttatcgga tcccagtgtg gtggtacatt 1680

gaggtcgact ctagaggatc gagccccagc tggttctttc cgcctcagaa gccatagagc 1740

ccaccgcatc cccagcatgc ctgctattgt cttcccaatc ctcccccttg ctgtcctgcc 1800

ccaccccacc ccccagaata gaatgacacc tactcagaca atgcgatgca atttcctcat 1860

tttattagga aaggacagtg ggagtggcac cttccagggt caaggaaggc acgggggagg 1920

ggcaaacaac agatggctgg caactagaag gcacagtcga ggctgatcag cgagctctag 1980

agaattgatc tacgtattag aagaactcgt caagaaggcg atagaaggcg atgcgctgcg 2040

aatcgggagc ggcgataccg taaagcacga ggaagcggtc agcccattcg ccgccaagct 2100

cttcagcaat atcacgggta gccaacgcta tgtcctgata gcggtccgcc acacccagcc 2160

ggccacagtc gatgaatcca gaaaagcggc cattttccac catgatattc ggcaagcagg 2220

catcgccatg ggtcacgacg agatcctcgc cgtcgggcat gcgcgccttg agcctggcga 2280

acagttcggc tggcgcgagc ccctgatgct cttcgtccag atcatcctga tcgacaagac 2340

cggcttccat ccgagtacgt gctcgctcga tgcgatgttt cgcttggtgg tcgaatgggc 2400

aggtagccgg atcaagcgta tgcagccgcc gcattgcatc agccatgatg gatactttct 2460

cggcaggagc aaggtgagat gacaggagat cctgccccgg cacttcgccc aatagcagcc 2520

agtcccttcc cgcttcagtg acaacgtcga gcacagctgc gcaaggaacg cccgtcgtgg 2580

ccagccacga tagccgcgct gcctcgtcct gcagttcatt cagggcaccg gacaggtcgg 2640

tcttgacaaa aagaaccggg cgcccctgcg ctgacagccg gaacacggcg gcatcagagc 2700

agccgattgt ctgttgtgcc cagtcatagc cgaatagcct ctccacccaa gcggccggag 2760

aacctgcgtg caatccatct tgttcaatgg gatccgtagc tactctgtgg gctcttgtct 2820

ccagctcctc atcatttctc cgactcctct gttttttcct tttggtgata tagaaaacga 2880

gcagtgccac aaagaccatc aagaggctgc ctcctccaca tatgccaatg atgagataga 2940

tgtccagacc tttctctgga cagctgacag gctcgacact ggattccttg ctgactttgt 3000

tccctgctgt gcacttgaat tttgcactca ggctggtggt ccacttgtgt gtgatgaccc 3060

tctgagaaag ttttagatgt ttcccatctt gatacaggtt taattcgggg tcagttccat 3120

tcattacctc acaggtcagg gttgtgttga tacaagtcca ggagatcttt ggttttgaga 3180

ccctctcttg aatcttcaaa tcaaatattt tttccaacac attttttcct tttgtatcat 3240

atattgatac cttgtagata tcctgatcat cggtcttcag atgcttaatt ttcagagttc 3300

catttttaaa tagcttatat gtatcttttt ccttgaaagt ctctttctct tttctgaatt 3360

gtgcaatctt tttcttgtct gaagtttttt cccattttat atcgtcaata tcatcactca 3420

tttgaaaact aggaatgtcc aagttgatgt cctgacccaa ggcaccccag gtttccaagg 3480

cattcgtaat ctctttggag actgcacctt tggaagaaac attgaaaatc agaaggaagc 3540

tggctacaaa tttacatgga atctagtacg taggaattcg ataacttcgt ataatgtgta 3600

ctatacgaag ttatgggtcg atggtgatgc ttggcaattc gggtcgatgg tgaagcttgg 3660

ataacttcgt ataatgtatg ctatacgaag ttatcaattc gccctatagt gagtcgtatt 3720

ctcccgaaaa ccgcttctag caacgaagtt cctatactat ttgaagaata ggaacttcgg 3780

aataggaact tcagcagatc ttctgtatcg tctatcgtta tgtctaatgg tatctgatgc 3840

agatctgctg aagttcctat actttctaga gaataggaac ttcggaatag gaacttcgaa 3900

ttctcgaggg cccgggctcg accagctgtg cgcatagtgg cttgaatcga taaaataaaa 3960

gattttattt agtctccaga aaaagggggg aatgaaagac cccacctgta ggtttggcaa 4020

gctagcacaa cccctcactc ggggcgccag tcctccgatt gactgagtcg cccgggtacc 4080

cgtgtatcca ataaaccctc ttgcagttgc atccgacttg tggtctcgct gttccttggg 4140

agggtctcct ctgagtgatt gactacccgt cagcgggggt ctttcacatg cagcatgtat 4200

caaaattaat ttggtttttt ttcttaagta tttacattaa atggccatag ttgcattaat 4260

gaatcggcca acgcgcgggg agaggcggtt tgcgtattgg gcgctcttcc gcttcctcgc 4320

tcactgactc gctgcgctcg gtcgttcggc tgcggcgagc ggtatcagct cactcaaagg 4380

cggtaatacg gttatccaca gaatcagggg ataacgcagg aaagaacatg tgagcaaaag 4440

gccagcaaaa ggccaggaac cgtaaaaagg ccgcgttgct ggcgtttttc cataggctcc 4500

gcccccctga cgagcatcac aaaaatcgac gctcaagtca gaggtggcga aacccgacag 4560

gactataaag ataccaggcg tttccccctg gaagctccct cgtgcgctct cctgttccga 4620

ccctgccgct taccggatac ctgtccgcct ttctcccttc gggaagcgtg gcgctttctc 4680

atagctcacg ctgtaggtat ctcagttcgg tgtaggtcgt tcgctccaag ctgggctgtg 4740

tgcacgaacc ccccgttcag cccgaccgct gcgccttatc cggtaactat cgtcttgagt 4800

ccaacccggt aagacacgac ttatcgccac tggcagcagc cactggtaac aggattagca 4860

gagcgaggta tgtaggcggt gctacagagt tcttgaagtg gtggcctaac tacggctaca 4920

ctagaaggac agtatttggt atctgcgctc tgctgaagcc agttaccttc ggaaaaagag 4980

ttggtagctc ttgatccggc aaacaaacca ccgctggtag cggtggtttt tttgtttgca 5040

agcagcagat tacgcgcaga aaaaaaggat ctcaagaaga tcctttgatc ttttctacgg 5100

ggtctgacgc tcagtggaac gaaaactcac gttaagggat tttggtcatg agattatcaa 5160

aaaggatctt cacctagatc cttttgcggc cgcaaatcaa tctaaagtat atatgagtaa 5220

acttggtctg acagttacca atgcttaatc agtgaggcac ctatctcagc gatctgtcta 5280

tttcgttcat ccatagttgc ctgactcccc gtcgtgtaga taactacgat acgggagggc 5340

ttaccatctg gccccagtgc tgcaatgata ccgcgagacc cacgctcacc ggctccagat 5400

ttatcagcaa taaaccagcc agccggaagg gccgagcgca gaagtggtcc tgcaacttta 5460

tccgcctcca tccagtctat taattgttgc cgggaagcta gagtaagtag ttcgccagtt 5520

aatagtttgc gcaacgttgt tgccattgct gcaggcatcg tggtgtcacg ctcgtcgttt 5580

ggtatggctt cattcagctc cggttcccaa cgatcaaggc gagttacatg atcccccatg 5640

ttgtgcaaaa aagcggttag ctccttcggt cctccgatcg ttgtcagaag taagttggcc 5700

gcagtgttat cactcatggt tatggcagca ctgcataatt ctcttactgt catgccatcc 5760

gtaagatgct tttctgtgac tggtgagtac tcaaccaagt cattctgaga atagtgtatg 5820

cggcgaccga gttgctcttg cccggcgtca atacgggata ataccgcgcc acatagcaga 5880

actttaaaag tgctcatcat tggaaaacgt tcttcggggc gaaaactctc aaggatctta 5940

ccgctgttga gatccagttc gatgtaaccc actcgtgcac ccaactgatc ttcagcatct 6000

tttactttca ccagcgtttc tgggtgagca aaaacaggaa ggcaaaatgc cgcaaaaaag 6060

ggaataaggg cgacacggaa atgttgaata ctcatactct tcctttttca atattattga 6120

agcatttatc agggttattg tctcatgagc ggatacatat ttgaatgtat ttagaaaaat 6180

aaacaaatag gggttccgcg cacatttccc cgaaaagtgc cac 6223

<210> 5

<211> 34

<212> DNA

<213> Artificial Sequence

<220>

<223> loxP

<400> 5
ataacttcgt ataatgtatg ctatacgaag ttat 34

<210> 6

<211> 34

<212> DNA

<213> Artificial Sequence

<220>

<223> lox511

<400> 6
ataacttcgt ataatgtata ctatacgaag ttat 34

<210> 7

<211> 34

<212> DNA

<213>  Artificial Sequence

<220>

<223>  lox5171

<400>  7
ataacttcgt ataatgtgta ctatacgaag ttat                                    34

<210>  8

<211>  34

<212>  DNA

<213>  Artificial Sequence

<220>

<223>  lox2272

<400>  8
ataacttcgt ataaagtatc ctatacgaag ttat                                    34

<210>  9

<211>  48

<212>  DNA

<213>  Artificial Sequence

<220>

<223>  frt

<400>  9
gaagttccta ttccgaagtt cctattctct agaaagtata ggaacttc                     48

<210>  10

<211>  48

<212>  DNA

<213>  Artificial Sequence

<220>

<223>  f3

<400>  10

gaagttccta ttccgaagtt cctattcttc aaatagtata ggaacttc 48

<210> 11

<211> 48

<212> DNA

<213> Artificial Sequence

<220>

<223> f5

<400> 11
gaagttccta ttccgaagtt cctattcttc aaaaggtata ggaacttc 48

<210> 12

<211> 23

<212> DNA

<213> Artificial Sequence

<220>

<223> U19

<400> 12
gctcttgact agcgagagag aag 23

<210> 13

<211> 23

<212> DNA

<213> Artificial Sequence

<220>

<223> B32

<400> 13
caaggcgatt aagttgggta acg 23

<210> 14

<211> 20

<212> DNA

<213>  Artificial Sequence

<220>

<223>  U34

<400>  14
ccagaaggaa aggattatgc                                                    20

<210>  15

<211>  20

<212>  DNA

<213>  Artificial Sequence

<220>

<223>  U35

<400>  15
acagagcaaa tgacccaagg                                                    20

<210>  16

<211>  22

<212>  DNA

<213>  Artificial Sequence

<220>

<223>  M117B8s

<400>  16
gagagtgctg gccagctgga ac                                                 22

<210>  17

<211>  22

<212>  DNA

<213>  Artificial Sequence

<220>

<223>  G01

<400>  17

caagttgatg tcctgaccca ag                                                22

<210>  18

<211>  22

<212>  DNA

<213>  Artificial Sequence

<220>

<223>  M117B8as1

<400>  18
ccaccatact ccacacactc tg                                                22